(19) Europäisches Patentamt / European Patent Office / Office européen des brevets

(11) EP 4 501 926 A1

(12) EUROPEAN PATENT APPLICATION
published in accordance with Art. 153(4) EPC

(43) Date of publication:
**05.02.2025 Bulletin 2025/06**

(21) Application number: **23778388.1**

(22) Date of filing: **30.03.2023**

(51) International Patent Classification (IPC):
*C07D 409/12* (2006.01) *A61K 31/496* (2006.01)
*A61P 25/18* (2006.01) *A61P 25/14* (2006.01)
*A61P 25/00* (2006.01) *A61P 25/20* (2006.01)
*A61P 25/16* (2006.01) *A61P 25/24* (2006.01)

(52) Cooperative Patent Classification (CPC):
**A61K 31/496; A61K 31/5377; A61P 25/00; A61P 25/04; A61P 25/14; A61P 25/16; A61P 25/18; A61P 25/20; A61P 25/24; A61P 25/28; C07D 409/12; C07D 409/14**

(86) International application number:
**PCT/CN2023/085136**

(87) International publication number:
**WO 2023/186023 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.03.2022 CN 202210333341**

(71) Applicant: **Vigonvita Life Sciences Co., Ltd.**
**Suzhou, Jiangsu 215123 (CN)**

(72) Inventors:
- **WU, Chunhui**
  **Suzhou, Jiangsu 215123 (CN)**
- **HE, Yang**
  **Suzhou, Jiangsu 215123 (CN)**
- **LI, Jian**
  **Suzhou, Jiangsu 215123 (CN)**
- **TIAN, Guanghui**
  **Suzhou, Jiangsu 215123 (CN)**

(74) Representative: **Kador & Partner Part mbB**
**Corneliusstraße 15**
**80469 München (DE)**

# (54) N-SUBSTITUTED QUINOLINONE COMPOUND, PREPARATION METHOD THEREFOR AND USE THEREOF

(57) The present invention belongs to the field of pharmaceutical chemistry. Specifically disclosed are a N-substituted quinolinone compound, a preparation method therefor and pharmaceutical use thereof. The compound of the present invention has a structure represented by formula (I), has excellent oil solubility, has a multi-target effect on the $5\text{-}HT_{2A}$ receptor, the dopamine $D_{2/3}$ receptor, the $5\text{-}HT_{1A}$ receptor and the 5-HT transporter, helps adjust the balance of intracerebral neurotransmitters, has relatively good efficacy on various diseases of the central nervous system, has few toxic and side effects, is very safe and tolerable, has good general druggability, and has good clinical application prospects.

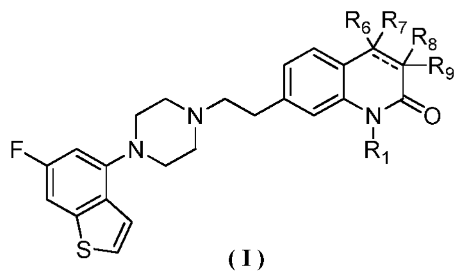

(I)

EP 4 501 926 A1

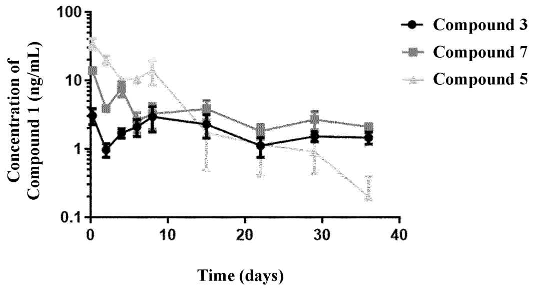
Concentration of Compound 1 (ng/mL)
100
10
1
0.1
0
10
20
30
40
Time (days)
Compound 3
Compound 7
Compound 5

FIG. 1

## Description

The present application claims priority to Chinese Patent Application No. 202210333341.5 entitled "N-SUBSTITUTED QUINOLINONE COMPOUND, PREPARATION METHOD THEREFOR AND USE THEREOF" filed on March 30, 2022, which is incorporated herein by reference in its entirety.

## TECHNICAL FIELD

The present invention belongs to the field of pharmaceutical chemistry, and particularly relates to a N-substituted quinolinone compound, a preparation method therefor, and pharmaceutical use thereof.

## BACKGROUND

With the rapid development of society, the pace of life and the pressure of people are increasing day by day, and mental diseases have become diseases which seriously affect the health of people and bring serious consequences to patients and their families. The average life expectancy of patients with mental diseases is shortened significantly due to susceptibility to suicide, lack of medical care, relative high risk of complications, and other factors. Numerous studies have shown that mental diseases are associated with dysfunction of various central neurotransmitters and receptors, such as monoamine transmitters in the brain, especially the dopamine (DA) system and the 5-hydroxytryptamine (5-HT) system, which are closely associated with normal mental activities of the human body. When the DA and 5-HT systems are dysfunctional, various neuropsychiatric diseases such as schizophrenia, depressive disorder, neuropathic pain, mania, anxiety, and Parkinson’s disease are easy to occur.

Neuropsychiatric diseases often exist as comorbidities with each other. For example, patients with schizophrenia often have depression and anxiety comorbidities, and schizophrenia patients with depression or anxiety symptoms have worse outcomes. The 5-HT transporter is an important target of antidepressant and anxiolytic drugs, and existing SSRIs (selective 5-HT reuptake inhibitors) drugs can all selectively inhibit the 5-HT transporter and increase the concentration of 5-HT in the synaptic cleft, thereby exerting the drug effect. Clinically, antipsychotics are often used in combination with SSRIs to treat the depression and anxiety symptoms of schizophrenia. Therefore, antipsychotics having an inhibitory effect on the 5-HT transporter are theoretically more effective in ameliorating the depression and anxiety comorbidities of schizophrenia. Therefore, drugs with a multi-target effect on targets including the 5-HT transporter are favorable for better regulation of the balance of various receptors in the brain, modulation of the DA/5-HT system, and thus better treatment of diseases in the field of the central nervous system.

Patent WO2015131856 reports a heterocyclic compound with a wide range of therapeutic effects on central nervous system diseases (e.g., schizophrenia, etc.), which has a general formula as follows:

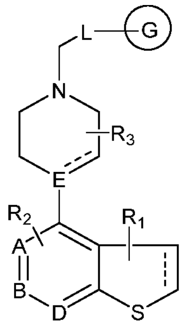

The above compounds, particularly the compound of Example 85 (7-(2-(4-(6-fluorophenothiophen-4-yl)piperazin-1-yl)ethyl)-3,4-dihydroquinofin-2(1H)-one, which is named as "compound 1-a" in the present invention), are antipsychotic drugs that have a wider treatment range, cause fewer side effects, and are excellent in safety and tolerability, as compared to conventional typical antipsychotics and atypical antipsychotics.

However, these compounds are poorly soluble in oily media (such as sesame oil and benzyl benzoate), resulting in limited application to oil injections.

In view of the above, the present invention is proposed.

## SUMMARY OF THE INVENTION

Problems to be Solved by the Present Invention

The present invention aims to provide a novel N-substituted quinolinone compound with improved and even excellent solubility in oily media (such as sesame oil and benzyl benzoate), a preparation method therefor, and pharmaceutical use thereof. Meanwhile, the prototype or *in-vivo* metabolite of the compound has an antagonistic effect on the 5-$HT_{2A}$ receptor, dopamine $D_2$ receptor, and 5-HT transporter, has an agonistic effect on the dopamine $D_3$ receptor and 5-$HT_{1A}$ receptor, and has relatively good safety and tolerability.

Solutions to the Problems

In a first aspect, the present invention provides a N-substituted quinolinone compound represented by general formula (1), or a pharmaceutically acceptable salt, a solvate, a stereoisomer, a geometric isomer, an isotopically labeled compound or a prodrug thereof:

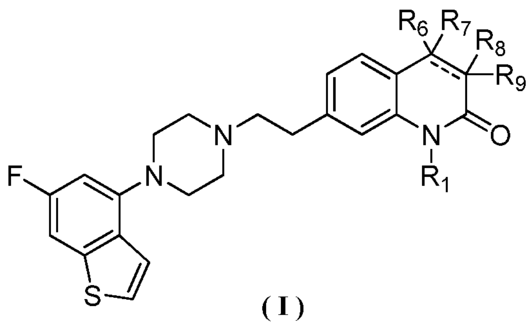

(I)

wherein:

- $R_6$, $R_7$, $R_8$, and $R_9$ are each independently selected from hydrogen, deuterium, halogen, methyl, ethyl, hydroxy, amino, or acetylamino;
- ------ represents a single bond or a double bond, and when it is a double bond, one of $R_6$ and $R_7$ is absent, and one of $R_8$ and $R_9$ is absent; preferably, ------ represents a single bond;
- $R_1$ is -$Y_1$-O-C(=O)-$R_2$, -$Y_1$-O-C(=O)-O-$R_3$, -$Y_1$-O-$R_4$, or -C(=O)-$R_5$;
- each $Y_1$ is independently C1-C6 alkylene; preferably, each $Y_1$ is independently C1-C4 alkylene; more preferably, each $Y_1$ is independently methylene, ethylene, or propylene;
- $R_2$ is C1-C30 alkyl, halogenated C1-C30 alkyl, C2-C30 alkenyl, amino C1-C30 alkyl, amino substituted with C1-C30 alkyl, amino substituted with C2-C30 alkenyl, amino substituted with halogenated C1-C30 alkyl, amino substituted with phenyl C1-C30 alkyl, amino substituted with hydroxy C1-C30 alkyl, amino substituted with C3-C10 cycloalkyl, amino substituted with C3-C10 cycloalkyl C1-C6 alkyl, C3-C10 cycloalkyl, 5- to 10-membered heterocyclyl, C6-C12 aryl, C6-C12 aryl C1-C30 alkyl, C1-C30 alkoxycarbonyl C1-C30 alkyl, C1-C30 alkoxy C1-C30 alkyl, C1-C30 alkoxy C1-C30 alkoxy C1-C30 alkyl, or C1-C30 alkoxy C1-C30 alkoxy C1-C30 alkoxy C1-C30 alkyl; preferably, $R_2$ is C1-C30 alkyl, halogenated C1-C30 alkyl, C2-C30 alkenyl, amino C1-C16 alkyl, amino substituted with C1-C16 alkyl, amino substituted with C2-C16 alkenyl, amino substituted with halogenated C1-C16 alkyl, amino substituted with phenyl C1-C16 alkyl, amino substituted with hydroxy C1-C16 alkyl, amino substituted with C3-C10 cycloalkyl, amino substituted with C3-C10 cycloalkyl C1-C6 alkyl, C3-C10 cycloalkyl, 5- to 10-membered heterocyclyl, C6-C12 aryl, C6-C12 aryl C1-C16 alkyl, C1-C16 alkoxycarbonyl C1-C16 alkyl, C1-C16 alkoxy C1-C16 alkyl, C1-C16 alkoxy C1-C16 alkoxy C1-C16 alkyl, or C1-C16 alkoxy C1-C16 alkoxy C1-C16 alkoxy C1-C16 alkyl; more preferably, $R_2$ is C1-C30 alkyl, halogenated C1-C30 alkyl, C2-C30 alkenyl, amino C1-C12 alkyl, amino substituted with C1-C12 alkyl, amino substituted with C2-C12 alkenyl, amino substituted with halogenated C1-C12 alkyl, amino substituted with phenyl C1-C12 alkyl, amino substituted with hydroxy C1-C12 alkyl, amino substituted with C3-C7 cycloalkyl, amino substituted with C3-C7 alkyl C1-C4 alkyl, C3-C7 cycloalkyl, 5- to 10-membered heterocyclyl, C6-C12 aryl, C6-C12 aryl C1-C12 alkyl, C1-C12 alkoxycarbonyl C1-C12 alkyl, C1-C12 alkoxy C1-C12 alkyl, C1-C12 alkoxy C1-C12 alkoxy C1-C12 alkyl, or C1-C12 alkoxy C1-C12 alkoxy C1-C12 alkoxy C1-C12 alkyl; more preferably, $R_2$ is C1-C30 alkyl (e.g., C1-C30 linear alkyl, isopropyl, tert-butyl, 1-methylpropyl, 3-methylbutyl, 1-methylbutyl, 1,1-dimethylpentyl, 1-methylpentyl, 1-methylhexyl, 1-pentylhexyl, etc.), halogenated C1-C30 alkyl (e.g., halogenated C1-C16 alkyl, such as halogenated C1-C12 alkyl or halogenated C1-C6 alkyl, further, e.g., 3,3,3-trifluoropropyl), C2-C30 alkenyl (e.g., C2-C20 alkenyl), amino C1-C6 alkyl (e.g., $NH_2$-methyl), amino substituted with C1-C12 alkyl (e.g., decyl-NH-,

heptanyl-NH-), amino substituted with C2-C6 alkenyl (e.g., allyl-NH-), amino substituted with halogenated C1-C6 alkyl (e.g., 3,3,3-trifluoropropyl-NH-), amino substituted with phenyl C1-C6 alkyl (e.g., benzyl-NH-), amino substituted with hydroxy C1-C6 alkyl (e.g., hydroxyethyl-NH-), amino substituted with C3-C7 cycloalkyl (e.g., cyclohexyl-NH-), amino substituted with C3-C7 cycloalkyl C1-C6 alkyl (e.g., C3-C7 cycloalkylmethyl-NH-), C3-C7 cycloalkyl, 5- to 10-membered heterocyclyl (e.g., pyridinyl, piperidinyl, 4-tetrahydropyranyl, morpholinyl, furanyl, thienyl, pyrimidinyl, quinolyl, etc.), phenyl, naphthyl, phenyl C1-C6 alkyl (e.g., benzyl), C1-C6 alkoxycarbonyl C1-C6 alkyl (e.g., tert-butoxycarbonylmethyl), C1-C6 alkoxy C1-C6 alkyl (e.g., methoxymethyl), C1-C6 alkoxy C1-C6 alkoxy C1-C6 alkyl (e.g., methoxyethoxymethyl or butoxyethoxymethyl), or C1-C6 alkoxy C1-C6 alkoxy C1-C6 alkoxy C1-C6 alkyl (e.g., methoxyethoxyethoxymethyl);

for example, $R_2$ is C1-C16 alkyl, halogenated C1-C16 alkyl, C2-C16 alkenyl, amino C1-C16 alkyl, amino substituted with C1-C16 alkyl, amino substituted with C2-C16 alkenyl, amino substituted with halogenated C1-C16 alkyl, amino substituted with phenyl C1-C16 alkyl, amino substituted with hydroxy C1-C16 alkyl, amino substituted with C3-C10 cycloalkyl, amino substituted with C3-C10 cycloalkyl C1-C6 alkyl, C3-C10 cycloalkyl, 5- to 10-membered heterocyclyl, C6-C12 aryl, C6-C12 aryl C1-C16 alkyl, C1-C16 alkoxycarbonyl C1-C16 alkyl, C1-C16 alkoxy C1-C16 alkyl, C1-C16 alkoxy C1-C16 alkoxy C1-C16 alkyl, or C1-C16 alkoxy C1-C16 alkoxy C1-C16 alkoxy C1-C16 alkyl; for example, $R_2$ is C1-C16 alkyl, halogenated C1-C16 alkyl, 5- to 10-membered heterocyclyl, C6-C12 aryl, C6-C12 aryl C1-C16 alkyl, or C1-C16 alkoxy C1-C16 alkyl; for another example, $R_2$ is C1-C16 alkyl (e.g., methyl, ethyl, isopropyl, tert-butyl, etc.), halogenated C1-C16 alkyl, phenyl, naphthyl, phenyl C1-C16 alkyl, pyridinyl, piperidinyl, morpholinyl, furanyl, thienyl, pyrimidinyl, or quinolyl; most especially, $R_2$ is C1-C16 alkyl or phenyl;

$R_3$ is C1-C30 alkyl, halogenated C1-C30 alkyl, phenyl, phenyl C1-C30 alkyl, or C3-C10 cycloalkyl; preferably, $R_3$ is C1-C16 alkyl, halogenated C1-C16 alkyl, phenyl, phenyl C1-C16 alkyl, or C3-C10 cycloalkyl; preferably, $R_3$ is C1-C16 alkyl, halogenated C1-C12 alkyl, phenyl, phenyl C1-C12 alkyl, or C3-C7 cycloalkyl (e.g., cyclohexyl); more preferably, $R_3$ is C1-C16 alkyl, halogenated C1-C6 alkyl (e.g., 2,2,2-trifluoroethyl), phenyl, phenyl C1-C6 alkyl (e.g., benzyl), or C3-7 cycloalkyl (e.g., cyclohexyl);

for example, $R_3$ is C1-C16 alkyl, halogenated C1-C16 alkyl, phenyl, phenyl C1-C16 alkyl, or C3-C10 cycloalkyl; for example, $R_3$ is C1-C16 alkyl or halogenated C1-C16 alkyl; for another example, $R_3$ is C1-C12 alkyl;

$R_4$ is hydrogen, C1-C30 alkyl, halogenated C1-C30 alkyl, phenyl C1-C30 alkyl, or phenyl C1-C30 alkoxy C1-C30 alkyl; preferably, $R_4$ is hydrogen, C1-C16 alkyl, halogenated C1-C16 alkyl, phenyl C1-C16 alkyl, or phenyl C1-C16 alkoxy C1-C16 alkyl; preferably, $R_4$ is hydrogen, C1-C12 alkyl, halogenated C1-C12 alkyl, phenyl C1-C12 alkyl, or phenyl C1-C12 alkoxy C1-C12 alkyl; more preferably, $R_4$ is hydrogen, C1-C6 alkyl (e.g., isopropyl), halogenated C1-C6 alkyl (e.g., 2,2,2-trifluoroethyl), phenyl C1-C6 alkyl (e.g., benzyl), or phenyl C1-C6 alkoxy C1-C6 alkyl (e.g., benzyloxymethyl);

preferably, $R_4$ is hydrogen, C1-C16 alkyl, halogenated C1-C16 alkyl, phenyl C1-C16 alkyl, or phenyl C1-C16 alkoxy C1-C16 alkyl;

$R_5$ is C1-C30 alkyl, C2-C30 alkenyl, morpholinyl, phenyl, phenyl C1-C30 alkyl, hydroxy C1-C30 alkyl, C3-C10 cycloalkyl, C1-C30 alkoxy, phenyloxy, phenyl C1-C30 alkoxy, C1-C30 alkoxy C1-C30 alkoxy, amino C1-C30 alkyl, amino substituted with C1-C30 alkyl, amino substituted with hydroxy C1-C30 alkyl, amino substituted with phenyl C1-C30 alkyl, C3-C10 cycloalkoxy, C1-C30 alkanoyloxy C1-C6 alkoxy, C1-C30 alkoxy C1-C30 alkyl, C1-C30 alkoxy C1-C30 alkoxy C1-C30 alkyl, or C1-C30 alkoxy C1-C30 alkoxy C1-C30 alkoxy C1-C30 alkyl; preferably, $R_5$ is C1-C16 alkyl, C2-C16 alkenyl, morpholinyl, phenyl, phenyl C1-C16 alkyl, hydroxy C1-C16 alkyl, C3-C10 cycloalkyl, C1-C16 alkoxy, phenyloxy, phenyl C1-C16 alkoxy, C1-C16 alkoxy C1-C16 alkoxy, amino C1-C16 alkyl, amino substituted with C1-C16 alkyl, amino substituted with hydroxy C1-C16 alkyl, amino substituted with phenyl C1-C16 alkyl, C3-C10 cycloalkoxy, C1-C16 alkanoyloxy C1-C6 alkoxy, C1-C16 alkoxy C1-C16 alkyl, C1-C16 alkoxy C1-C16 alkoxy C1-C16 alkyl, or C1-C16 alkoxy C1-C16 alkoxy C1-C16 alkoxy C1-C16 alkyl; preferably, $R_5$ is C1-C12 alkyl, C2-C12 alkenyl, morpholinyl, phenyl, phenyl C1-C12 alkyl, hydroxy C1-C12 alkyl, C3-C10 cycloalkyl, C1-C12 alkoxy, phenyloxy, phenyl C1-C12 alkoxy, C1-C12 alkyloxy C1-C12 alkoxy, amino C1-C12 alkyl, amino substituted with C1-C12 alkyl, amino substituted with hydroxy C1-C12 alkyl, amino substituted with phenyl C1-C12 alkyl, C3-C10 cycloalkoxy, C1-C12 alkanoyloxy C1-C6 alkoxy, C1-C12 alkoxy C1-C12 alkyl, C1-C12 alkoxy C1-C12 alkoxy C1-C12 alkyl, or C1-C12 alkoxy C1-C12 alkoxy C1-C12 alkoxy C1-C12 alkyl; more preferably, $R_5$ is C1-C6 alkyl (e.g., methyl, ethyl, propyl, etc.), C2-C16 alkenyl (e.g., decenyl), morpholinyl, phenyl, phenyl C1-C6 alkyl, hydroxy C1-C6 alkyl, C3-C10 cycloalkyl (e.g., cyclopentyl or cyclohexyl), C1-C6 alkoxy (e.g., methoxy, ethoxy, propoxy, isopropoxy, etc.), phenoxy, phenyl C1-C6 alkoxy, C1-C6 alkoxy C1-C6 alkoxy (e.g., methoxyethoxy), amino C1-C6 alkyl ($NH_2$-methyl), amino substituted with C1-C6 alkyl (e.g., propylamino), amino substituted with hydroxy C1-C6 alkyl (e.g., hydroxyethyl-NH-), amino substituted with phenyl C1-C6 alkyl (e.g., benzyl-NH-), C1-C16 alkanoyloxy C1-C6 alkoxy (e.g., decanoyloxymethyloxy), C1-C6 alkoxy C1-C6 alkyl (e.g., methoxymethyl), C1-C6 alkoxy C1-C6 alkoxy C1-C6 alkyl (e.g., methoxyethoxymethyl or methoxyethoxymethyl), or C1-C6 alkoxy C1-C6 alkoxy C1-C6 alkoxy C1-C6 alkyl (e.g., methoxyethoxyethoxymethyl);

for example, $R_5$ is C1-C16 alkyl, C2-C16 alkenyl, morpholinyl, phenyl, phenyl C1-C16 alkyl, hydroxy C1-C16 alkyl, C3-C10 cycloalkyl, C1-C16 alkoxy, phenyl C1-C16 alkoxy, C1-C16 alkoxy C1-C16 alkoxy, amino C1-C16 alkyl, amino substituted with C1-C16 alkyl, amino substituted with hydroxy C1-C16 alkyl, amino substituted with phenyl C1-C16 alkyl, C3-C10 cycloalkoxy, C1-C16 alkoxy C1-C16 alkyl, C1-C16 alkoxy C1-C16 alkoxy C1-C16 alkyl, or C1-C16 alkoxy C1-C16 alkoxy C1-C16 alkoxy C1-C16 alkyl; for example, $R_5$ is C1-C12 alkyl, phenyl C1-C12 alkyl, C1-C12 alkoxy, phenyl C1-C12 alkoxy, C1-C12 alkoxy C1-C12 alkoxy, or C1-C12 alkoxy C1-C12 alkyl; for another example, $R_5$ is C1-C6 alkyl (e.g., methyl, ethyl, propyl, etc.), phenyl C1-C6 alkyl, C1-C6 alkoxy (e.g., methoxy, ethoxy, propoxy, etc.), phenyl C1-C6 alkoxy, C1-C6 alkoxy C1-C6 alkoxy, or C1-C6 alkoxy C1-C6 alkyl.

In some embodiments, the N-substituted quinolinone compound, or the pharmaceutically acceptable salt, the solvate, the stereoisomer, the geometric isomer, the isotopically labeled compound or the prodrug thereof is represented by general formula (1-1) or (1-2) as follows:

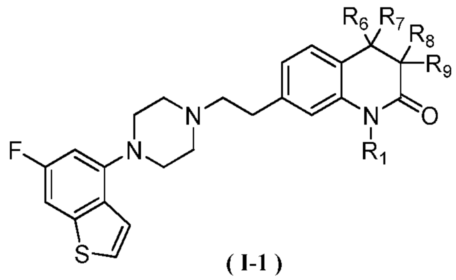
( I-1 )

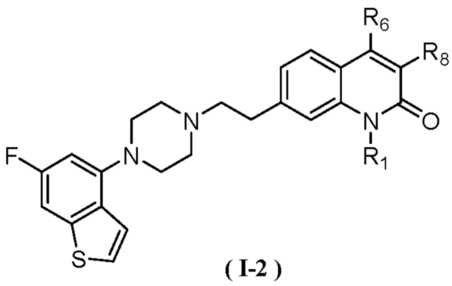
( I-2 )

wherein, $R_1$, $R_6$, $R_7$, $R_8$, and $R_9$ have definitions as described above for formula (I).

In some preferred embodiments, $R_1$ is $-Y_1$-O-C(=O)-$R_2$, $-Y_1$-O-C(=O)-O-$R_3$, $-Y_1$-O-$R_4$, or -C(=O)-Rs, preferably $-Y_1$-O-C(=O)-$R_2$;

$Y_1$ are each independently C1-C4 alkylene, preferably $-CH_2-$ or $-CH(CH_3)-$;

$R_2$ is C1-C30 alkyl, halogenated C1-C30 alkyl, C2-C30 alkenyl, amino C1-C16 alkyl, amino substituted with C1-C16 alkyl, amino substituted with C2-C16 alkenyl, amino substituted with halogenated C1-C16 alkyl, amino substituted with phenyl C1-C16 alkyl, amino substituted with hydroxy C1-C16 alkyl, amino substituted with C3-C10 cycloalkyl, amino substituted with C3-C10 cycloalkyl C1-C6 alkyl, C3-C10 cycloalkyl, 5- to 10-membered heterocyclyl, C6-C12 aryl, C6-C12 aryl C1-C16 alkyl, C1-C16 alkoxycarbonyl C1-C16 alkyl, C1-C16 alkoxy C1-C16 alkyl, C1-C16 alkoxy C1-C16 alkoxy C1-C16 alkyl, or C1-C16 alkoxy C1-C16 alkoxy C1-C16 alkoxy C1-C16 alkyl; preferably, $R_2$ is C1-C30 alkyl, halogenated C1-C30 alkyl, C2-C30 alkenyl, amino C1-C12 alkyl, amino substituted with C1-C12 alkyl, amino substituted with C2-C12 alkenyl, amino substituted with halogenated C1-C12 alkyl, amino substituted with phenyl C1-C12 alkyl, amino substituted with hydroxy C1-C12 alkyl, amino substituted with C3-C7 cycloalkyl, amino substituted with C3-C7 cycloalkyl C1-C4 alkyl, C3-C7 cycloalkyl, 5- to 10-membered heterocyclyl, C6-C12 aryl, C6-C12 aryl C1-C12 alkyl, C1-C12 alkoxycarbonyl C1-C12 alkyl, C1-C12 alkoxy C1-C12 alkyl, C1-C12 alkoxy C1-C12 alkoxy C1-C12 alkyl, or C1-C12 alkoxy C1-C12 alkoxy C1-C12 alkoxy C1-C12 alkyl; more preferably, $R_2$ is C1-C30 alkyl (e.g., C1-C30 linear alkyl, isopropyl, tert-butyl, 1-methylpropyl, 3-methylbutyl, 1-methylbutyl, 1,1-dimethylpentyl, 1-methylpentyl, 1-methylhexyl, 1-pentylhexyl, etc.), halogenated C1-C30 alkyl (e.g., halogenated C1-C16 alkyl, halogenated C1-C12 alkyl, or halogenated C1-C6 alkyl, further, e.g., 3,3,3-trifluoropropyl), C2-C30 alkenyl (e.g., C2-C20 alkenyl), amino C1-C6 alkyl (e.g., $NH_2$-methyl), amino substituted with C1-C12 alkyl (e.g., decyl-NH- or heptanyl-NH-), amino substituted with C2-C6 alkenyl (e.g., 2-allyl-NH-), amino substituted with halogenated C1-C6 alkyl (e.g., 3,3,3-trifluoropropyl-NH-), amino substituted with phenyl C1-C6 alkyl (e.g., benzyl-NH-), amino substituted with hydroxy C1-C6 alkyl (e.g., hydroxyethyl-NH-), amino substituted with C3-C7 cycloalkyl (e.g., C3-C7 cycloalkyl-NH-), amino substituted with C3-C7 cycloalkyl C1-C6 alkyl (e.g., C3-C7 cycloalkylmethyl-NH-), C3-C7 cycloalkyl, 5- to 10-membered heterocyclyl (e.g., pyridinyl, piperidinyl, morpholinyl, furanyl, thienyl, pyrimidinyl, quinolyl, etc.), phenyl, naphthyl, phenyl C1-C6 alkyl (e.g., benzyl), C1-C6 alkoxycarbonyl C1-C6 alkyl (e.g., *tert*-butoxycarbonylmethyl), C1-C6 alkoxy C1-C6 alkyl (e.g., methoxymethyl), C1-C6 alkoxy C1-C6 alkoxy C1-C6 alkyl (e.g., methoxyethoxymethyl or butoxyethoxymethyl), or C1-C6 alkoxy C1-C6 alkoxy C1-C6 alkoxy C1-C6 alkyl (e.g., methoxyethoxyethoxymethyl);

$R_3$ is C1-C16 alkyl, halogenated C1-C16 alkyl, phenyl, phenyl C1-C16 alkyl, or C3-C10 cycloalkyl; preferably, $R_3$ is C1-C12 alkyl, halogenated C1-C12 alkyl, phenyl, phenyl C1-C12 alkyl, or C3-C7 cycloalkyl (e.g., cyclohexyl); more preferably, $R_3$ is C1-C6 alkyl, halogenated C1-C6 alkyl (e.g., 2,2,2-trifluoroethyl), phenyl, phenyl C1-C6 alkyl (e.g., benzyl), or C3-7 cycloalkyl (e.g., cyclohexyl);

$R_4$ is hydrogen, C1-C16 alkyl, halogenated C1-C16 alkyl, phenyl C1-C16 alkyl, or phenyl C1-C16 alkoxy C1-C16 alkyl; preferably, $R_4$ is hydrogen, C1-C12 alkyl, halogenated C1-C12 alkyl, phenyl C1-C12 alkyl, or phenyl C1-C12 alkoxy C1-C12 alkyl; more preferably, $R_4$ is hydrogen, C1-C6 alkyl (e.g., isopropyl), halogenated C1-C6 alkyl (e.g., 2,2,2-trifluoroethyl), phenyl C1-C6 alkyl (e.g., benzyl), or phenyl C1-C6 alkoxy C1-C6 alkyl (e.g., benzyloxymethyl);
$R_5$ is C1-C16 alkyl, C2-C16 alkenyl, morpholinyl, phenyl, phenyl C1-C16 alkyl, hydroxy C1-C16 alkyl, C3-C10 cycloalkyl, C1-C16 alkoxy, phenyloxy, phenyl C1-C16 alkoxy, C1-C16 alkoxy C1-C16 alkoxy, amino C1-C16 alkyl, amino substituted with C1-C16 alkyl, amino substituted with hydroxy C1-C16 alkyl, amino substituted with phenyl C1-C16 alkyl, C3-C10 cycloalkoxy, C1-C16 alkanoyloxy C1-C6 alkoxy, C1-C16 alkoxy C1-C16 alkyl, C1-C16 alkoxy C1-C16 alkoxy C1-C16 alkyl, or C1-C16 alkoxy C1-C16 alkoxy C1-C16 alkoxy C1-C16 alkyl; preferably, $R_5$ is C1-C12 alkyl, C2-C12 alkenyl, morpholinyl, phenyl, phenyl C1-C12 alkyl, hydroxy C1-C12 alkyl, C3-C10 cycloalkyl, C1-C12 alkoxy, phenyloxy, phenyl C1-C12 alkoxy, C1-C12 alkoxy C1-C12 alkoxy, amino C1-C12 alkyl, amino substituted with C1-C12 alkyl, amino substituted with hydroxy C1-C12 alkyl, amino substituted with phenyl C1-C12 alkyl, C3-C10 cycloalkyl, C1-C12 alkanoyloxy C1-C6 alkoxy, C1-C12 alkoxy C1-C12 alkyl, C1-C12 alkoxy C1-C12 alkoxy C1-C12 alkyl, or C1-C12 alkoxy C1-C12 alkoxy C1-C12 alkoxy C1-C12 alkyl; more preferably, $R_5$ is C1-C6 alkyl (e.g., methyl, ethyl, propyl, etc.), C2-C16 alkenyl (e.g., decenyl), morpholinyl, phenyl, phenyl C1-C6 alkyl, hydroxy C1-C6 alkyl, C3-C10 cycloalkyl (e.g., cyclopentyl or cyclohexyl), C1-C6 alkoxy (e.g., methoxy, ethoxy, propoxy, isopropoxy, etc.), phenoxy, phenyl C1-C6 alkoxy, C1-C6 alkoxy C1-C6 alkoxy (e.g., methoxyethoxy), amino C1-C6 alkyl ($NH_2$-methyl), amino substituted with C1-C6 alkyl (e.g., propylamino), amino substituted with hydroxy C1-C6 alkyl (e.g., hydroxyethyl-NH-), amino substituted with phenyl C1-C6 alkyl (e.g., benzyl-NH-), C1-C16 alkanoyloxy C1-C6 alkoxy (decanoyloxymethyoxy), C1-C6 alkoxy C1-C6 alkyl (e.g., methoxymethyl), C1-C6 alkoxy C1-C6 alkoxy C1-C6 alkyl (e.g., methoxyethoxymethyl or methoxyethoxymethyl), or C1-C6 alkoxy C1-C6 alkoxy C1-C6 alkoxy C1-C6 alkyl (e.g., methoxyethoxyethoxymethyl);
the remaining groups are as defined above.

In some preferred embodiments, $R_1$ is $-Y_1$-O-C(=O)-$R_2$ or $-Y_1$-O-C(=O)-O-$R_3$, preferably $-Y_1$-O-C(=O)-$R_2$; $Y_1$ are each independently C1-C4 alkylene, preferably $-CH_2$- or $-CH(CH_3)$-;

$R_2$ is C1-C12 alkyl, halogenated C1-C12 alkyl, C2-C12 alkenyl, amino C1-C12 alkyl, amino substituted with C1-C12 alkyl, amino substituted with C2-C12 alkenyl, amino substituted with halogenated C1-C12 alkyl, amino substituted with phenyl C1-C12 alkyl, amino substituted with hydroxy C1-C12 alkyl, amino substituted with C3-C6 cycloalkyl, amino substituted with C3-C6 cycloalkyl C1-C4 alkyl, C3-C6 cycloalkyl, pyridinyl, piperidinyl, morpholinyl, furanyl, thienyl, pyrimidinyl, quinolyl, phenyl, phenyl C1-C12 alkyl, C1-C12 alkoxycarbonyl C1-C12 alkyl, C1-C12 alkoxy C1-C12 alkoxy C1-C12 alkyl, or C1-C12 alkoxy C1-C12 alkoxy C1-C12 alkoxy C1-C12 alkyl, preferably C1-C12 alkyl, halogenated C1-C12 alkyl, C3-C6 cycloalkyl, phenyl, phenyl C1-C12 alkyl, C1-C12 alkoxycarbonyl C1-C12 alkyl, C1-C12 alkoxy C1-C12 alkoxy C1-C12 alkyl, or C1-C12 alkoxy C1-C12 alkoxy C1-C12 alkoxy C1-C12 alkyl;
$R_3$ is C1-C12 alkyl, halogenated C1-C12 alkyl, phenyl, phenyl C1-C12 alkyl, or C3-C6 cycloalkyl, preferably C1-C12 alkyl, halogenated C1-C12 alkyl, phenyl, or C3-C6 cycloalkyl.

In some preferred embodiments, $R_1$ is $-Y_1$-O-C(=O)-$R_2$;

$Y_1$ is C1-C4 alkylene, preferably $-CH_2$- or $-CH(CH_3)$-;
$R_2$ is C3-C16 alkyl, halogenated C3-C16 alkyl, C3-C16 alkenyl, amino C3-C16 alkyl, amino substituted with C3-C16 alkyl, amino substituted with C3-C16 alkenyl, amino substituted with halogenated C3-C16 alkyl, amino substituted with phenyl C3-C16 alkyl, amino substituted with hydroxy C3-C16 alkyl, amino substituted with C3-C6 cycloalkyl, amino substituted with C3-C6 cycloalkyl C3-C16 alkyl, C3-C6 cycloalkyl, pyridinyl, piperidinyl, morpholinyl, furanyl, thienyl, pyrimidinyl, quinolyl, phenyl, phenyl C3-C16 alkyl, C1-C12 alkoxycarbonyl C3-C16 alkyl, C1-C12 alkoxy C1-C12 alkoxy C3-C16 alkyl, or C1-C12 alkoxy C1-C12 alkoxy C1-C12 alkoxy C3-C16 alkyl, preferably C3-C16 alkyl, halogenated C3-C16 alkyl, C3-C6 cycloalkyl, phenyl, phenyl C3-C16 alkyl, C1-C12 alkoxycarbonyl C3-C16 alkyl, C1-C12 alkoxy C1-C12 alkoxy C3-C16 alkyl, or C1-C12 alkoxy C1-C12 alkoxy C1-C12 alkoxy C3-C16 alkyl.

In some preferred embodiments, $R_1$ is $-Y_1$-O-$R_4$;
$R_4$ is hydrogen, C1-C12 alkyl, halogenated C1-C12 alkyl, phenyl C1-C12 alkyl, phenyl C1-C12 alkoxy C1-C12 alkyl, preferably hydrogen, C1-C12 alkyl, halogenated C1-C12 alkyl, or phenyl C1-C12 alkyl.
In some preferred embodiments, $R_1$ is -C(=O)-$R_5$;
$R_5$ is C1-C12 alkyl, C2-C12 alkenyl, morpholinyl, phenyl, phenyl C1-C12 alkyl, hydroxy C1-C12 alkyl, C3-C6 cycloalkyl, C1-C12 alkoxy, phenyl C1-C12 alkoxy, amino C1-C12 alkyl, amino substituted with C1-C12 alkyl, amino substituted with hydroxy C1-C12 alkyl, amino substituted with phenyl C1-C12 alkyl, C3-C6 cycloalkoxy, C1-C12 alkoxy C1-C12 alkoxy C1-C12 alkyl, or C1-C12 alkoxy C1-C12 alkoxy C1-C12 alkoxy C1-C12 alkyl, preferably C1-C12 alkyl, phenyl, phenyl C1-C12 alkyl, C3-C6 cycloalkyl, C1-C12 alkoxy, phenyl C1-C12 alkoxy, C3-C6 cycloalkoxy, C1-C12 alkoxy C1-C12 alkoxy C1-C12 alkyl, or C1-C12 alkoxy C1-C12 alkoxy C1-C12 alkoxy C1-C12 alkyl.
In some preferred embodiments, in general formula (1-1) or (1-2) described above,

$R_6$, $R_7$, $R_8$, and $R_9$ are each independently hydrogen;
$R_1$ is $-Y_1-O-C(=O)-R_2$, $-Y_1-O-C(=O)-O-R_3$, $-Y_1-O-R_4$, or $-C(=O)-R_5$;
$Y_1$ are each independently C1-C4 alkylene, preferably $-CH_2-$ or $-CH(CH_3)-$;
$R_2$ is C1-C16 alkyl or phenyl;
$R_3$ is C1-C12 alkyl;
$R_4$ is hydrogen;
$R_5$ is C1-C6 alkyl or C1-C6 alkoxy.

In some preferred embodiments,

$R_6$, $R_7$, $R_8$, and $R_9$ are each independently hydrogen;
$R_1$ is $-Y_1-O-C(=O)-R_2$;
$Y_1$ is C1-C4 alkylene, preferably $-CH_2-$ or $-CH(CH_3)-$;
$R_2$ C1-C30 alkyl.

In a second aspect, the present invention provides N-substituted quinolinone compounds, or pharmaceutically acceptable salts, solvates, stereoisomers, geometric isomers, isotopically labeled compounds or prodrugs thereof as follows:

(1) 7-(2-(4-(6-fluorobenzo[*b*]thiophen-4-yl)piperazin-1-yl)ethyl)-1-(hydroxymethyl)-3,4-dihydroquinolin-2(1*H*) -one;

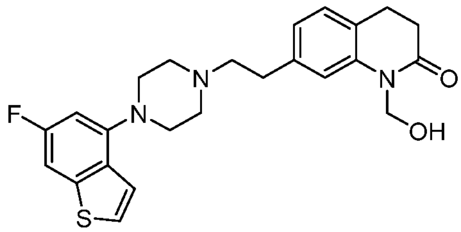

(2) (7-(2-(4-(6-fluorobenzo[*b*]thiophen-4-yl)piperazin-1-yl)ethyl)-2-oxo-3,4-dihydroquinolin-1(2*H*)-yl)methyl isopropyl carbonate;

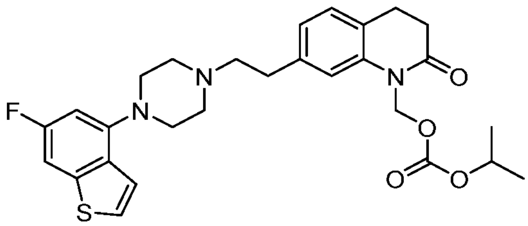

(3) (7-(2-(4-(6-fluorobenzo[*b*]thiophen-4-yl)piperazin-1-yl)ethyl)-2-oxo-3,4-dihydroquinolin-1(2*H*)-yl)methyl dodecanoate;

(4)

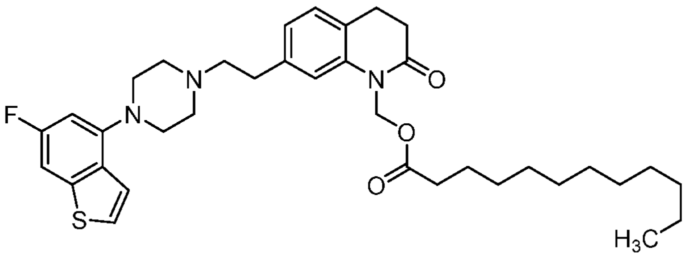

isopropyl 7-(2-(4-(6-fluorobenzo[*b*]thiophen-4-yl)piperazin-1-yl)ethyl)-2-oxo-3,4-dihydroquinoline-1(2*H*)-carboxylate

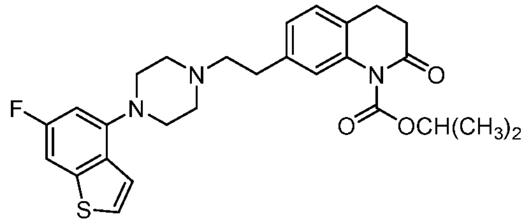

(5) (7-(2-(4-(6-fluorobenzo[*b*]thiophen-4-yl)piperazin-1-yl)ethyl)-2-oxo-3,4-dihydroquinolin-1(2*H*)-yl)methyl acetate;

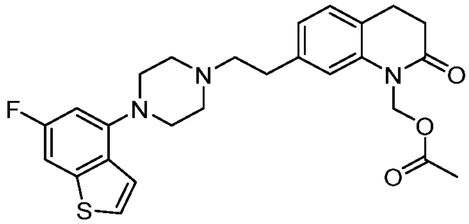

(6) (7-(2-(4-(6-fluorobenzo[*b*]thiophen-4-yl)piperazin-1-yl)ethyl)-2-oxo-3,4-dihydroquinolin-1(2*H*)-yl)methyl pentanoate;

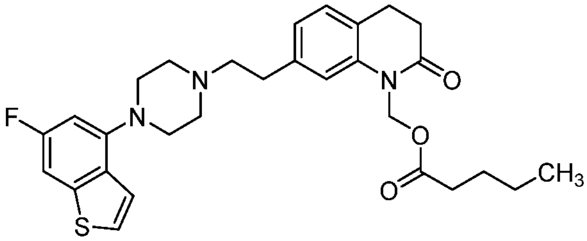

(7) (7-(2-(4-(6-fluorobenzo[*b*]thiophen-4-yl)piperazin-1-yl)ethyl)-2-oxo-3,4-dihydroquinolin-1(2*H*)-yl)methyl decanoate;

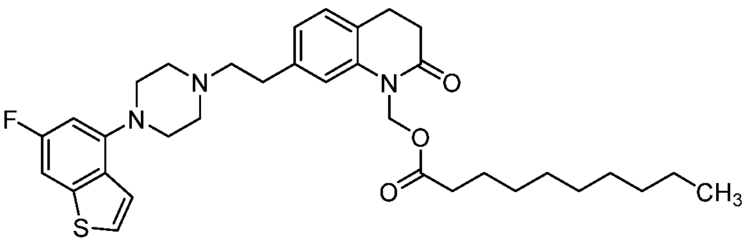

(8) (7-(2-(4-(6-fluorobenzo[*b*]thiophen-4-yl)piperazin-1-yl)ethyl)-2-oxo-3,4-dihydroquinolin-1(2*H*)-yl)methyl palmitate;

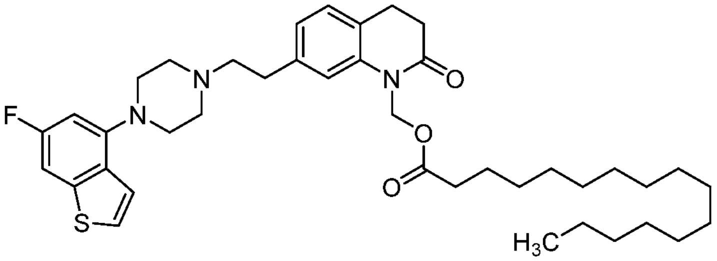

(9) (7-(2-(4-(6-fluorobenzo[*b*]thiophen-4-yl)piperazin-1-yl)ethyl)-2-oxoquinolin-1(2*H*)-yl)methyl palmitate;

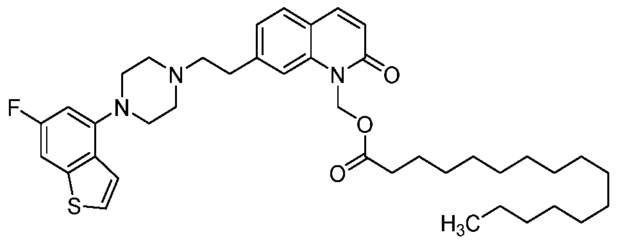

(10) (7-(2-(4-(6-fluorobenzo[*b*]thiophen-4-yl)piperazin-1-yl)ethyl)-2-oxoquinolin-1(2*H*)-yl)methyl decanoate;

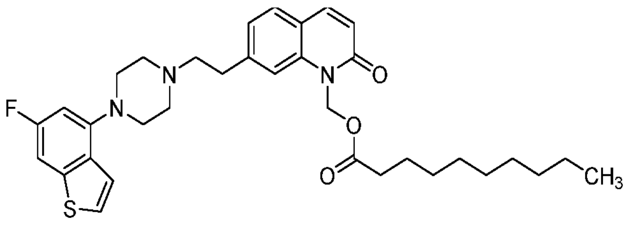

(11) 1-acetyl-7-(2-(4-(6-fluorobenzo[*b*]thiophen-4-yl)piperazin-1-yl)ethyl)-3,4-dihydroquinolin-2(1*H*)-one;

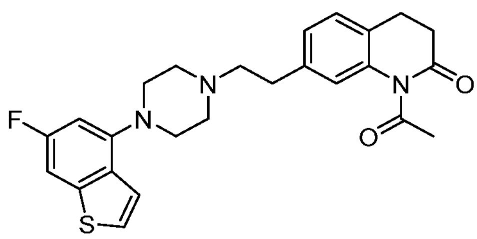

(12) (7-(2-(4-(6-fluorobenzo[*b*]thiophen-4-yl)piperazin-1-yl)ethyl)-2-oxo-3,4-dihydroquinolin-1(2*H*)-yl)methyl benzoate;

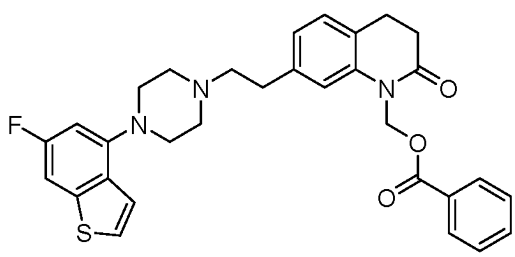

(13) 1-(7-(2-(4-(6-fluorobenzo[*b*]thiophen-4-yl)piperazin-1-yl)ethyl)-2-oxo-3,4-dihydroquinolin-1(2*H*)-yl)ethyl dodecanoate;

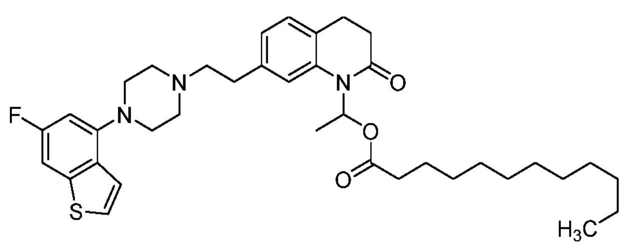

(14) (7-(2-(4-(6-fluorobenzo[*b*]thiophen-4-yl)piperazin-1-yl)ethyl)-2-oxo-3,4-dihydroquinolin-1(2*H*)-yl)methyl undecyl carbonate;

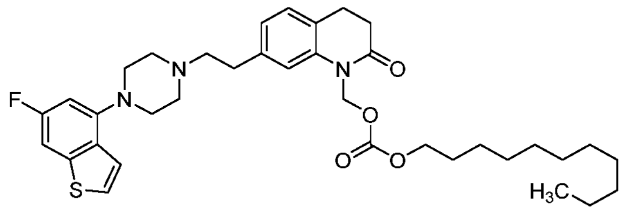

(15) 1-(7-(2-(4-(6-fluorobenzo[*b*]thiophen-4-yl)piperazin-1-yl)ethyl)-2-oxo-3,4-dihydroquinolin-1(2*H*)-yl)ethyl palmitate;

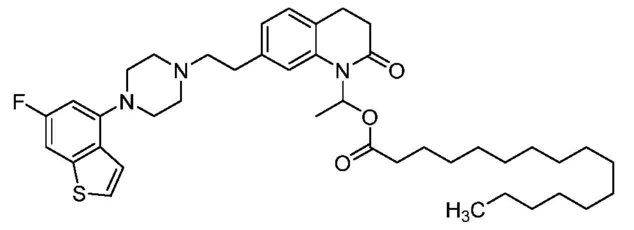

(16) 1-(7-(2-(4-(6-fluorobenzo[*b*]thiophen-4-yl)piperazin-1-yl)ethyl)-2-oxo-3,4-dihydroquinolin-1(2*H*)-yl)ethyl isobutyrate;

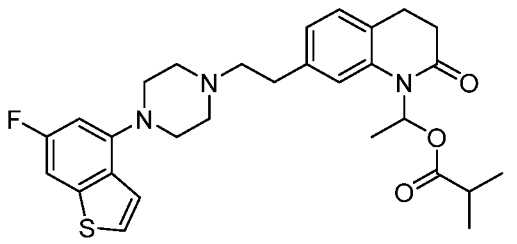

(17) (7-(2-(4-(6-fluorobenzo[*b*]thiophen-4-yl)piperazin-1-yl)ethyl)-2-oxo-3,4-dihydroquinolin-1(2*H*)-yl)methyl pivalate;

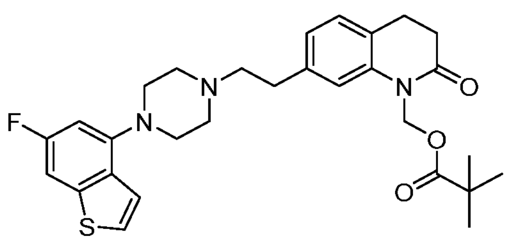

(18) (7-(2-(4-(6-fluorobenzo[*b*]thiophen-4-yl)piperazin-1-yl)ethyl)-2-oxo-3,4-dihydroquinolin-1(2*H*)-yl)methyl hexanoate;

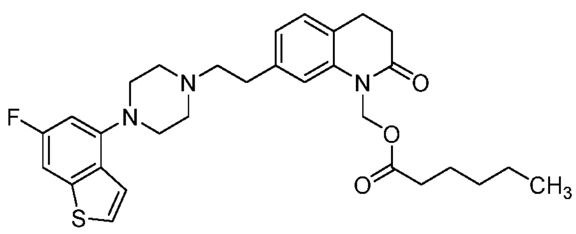

(19) (7-(2-(4-(6-fluorobenzo[*b*]thiophen-4-yl)piperazin-1-yl)ethyl)-2-oxo-3,4-dihydroquinolin-1(2*H*)-yl)methyl methyl carbonate;

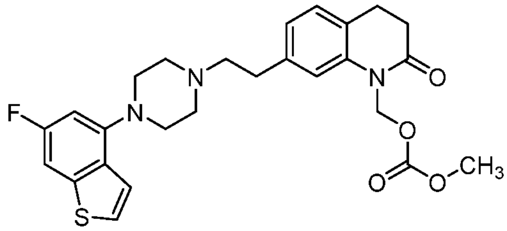

(20) (7-(2-(4-(6-fluorobenzo[*b*]thiophen-4-yl)piperazin-1-yl)ethyl)-2-oxoquinolin-1(2*H*)-yl)methyl methyl carbonate;

(21)

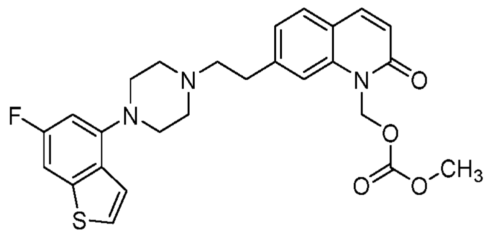

ethyl ((7-(2-(4-(6-fluorobenzo[*b*]thiophen-4-yl)piperazin-1-yl)ethyl)-2-oxo-3,4-dihydroquinolin-1(2*H*)-yl)methyl) carbonate;

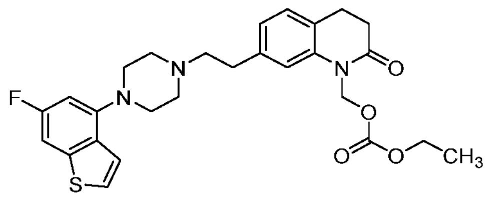

(22) (7-(2-(4-(6-fluorobenzo[*b*]thiophen-4-yl)piperazin-1-yl)ethyl)-2-oxo-3,4-dihydroquinolin-1(2*H*)-yl)methyl propyl carbonate;

(23)

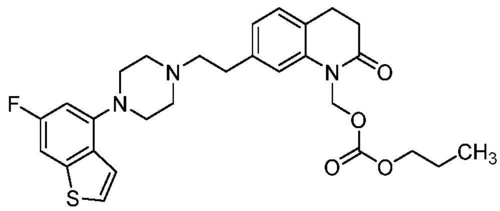

butyl ((7-(2-(4-(6-fluorobenzo[*b*]thiophen-4-yl)piperazin-1-yl)ethyl)-2-oxo-3,4-dihydroquinolin-1(2*H*)-yl)methyl) carbonate;

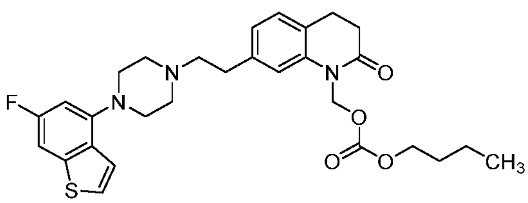

(24) (7-(2-(4-(6-fluorobenzo[*b*]thiophen-4-yl)piperazin-1-yl)ethyl)-2-oxo-3,4-dihydroquinolin-1(2*H*)-yl)methyl hexyl carbonate;

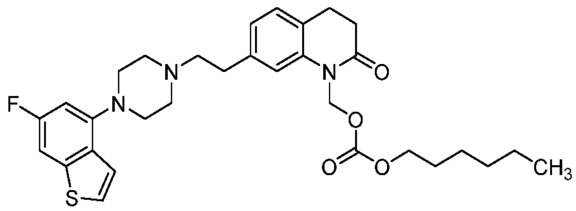

(25) (7-(2-(4-(6-fluorobenzo[*b*]thiophen-4-yl)piperazin-1-yl)ethyl)-2-oxo-3,4-dihydroquinolin-1(2*H*)-yl)methyl heptyl carbonate;

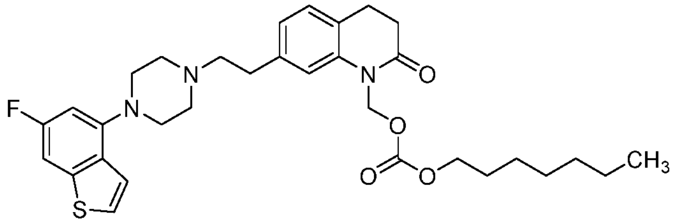

(26) (7-(2-(4-(6-fluorobenzo[*b*]thiophen-4-yl)piperazin-1-yl)ethyl)-2-oxo-3,4-dihydroquinolin-1(2*H*)-yl)methyl octyl carbonate;

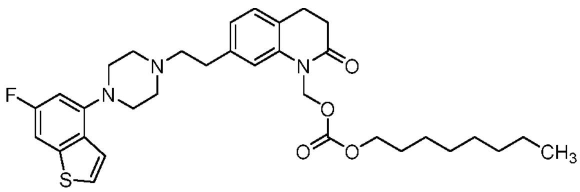

(27) (7-(2-(4-(6-fluorobenzo[*b*]thiophen-4-yl)piperazin-1-yl)ethyl)-2-oxo-3,4-dihydroquinolin-1(2*H*)-yl)methyl nonyl carbonate

(28)

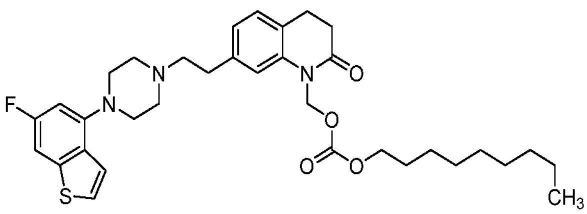

decyl ((7-(2-(4-(6-fluorobenzo[*b*]thiophen-4-yl)piperazin-1-yl)ethyl)-2-oxo-3,4-dihydroquinolin-1(2*H*)-yl)methyl) carbonate;

(29)

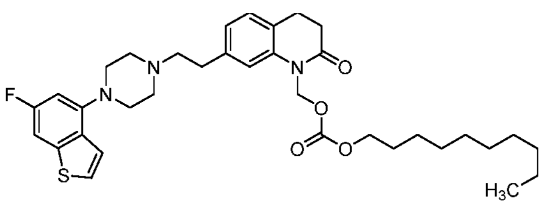

dodecyl ((7-(2-(4-(6-fluorobenzo[*b*]thiophen-4-yl)piperazin-1-yl)ethyl)-2-oxo-3,4-dihydroquinolin-1(2*H*)-yl)methyl) carbonate;

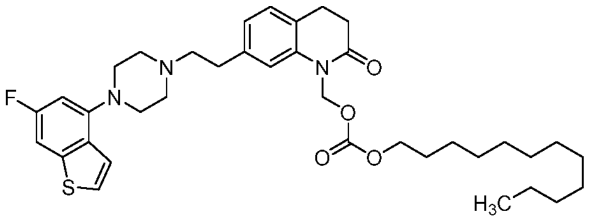

(30) (7-(2-(4-(6-fluorobenzo[*b*]thiophen-4-yl)piperazin-1-yl)ethyl)-2-oxo-3,4-dihydroquinolin-1(2*H*)-yl)methyl tetradecyl carbonate;

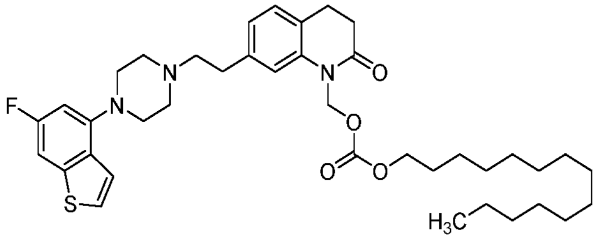

(31) (7-(2-(4-(6-fluorobenzo[*b*]thiophen-4-yl)piperazin-1-yl)ethyl)-2-oxo-3,4-dihydroquinolin-1(2*H*)-yl)methyl hexadecyl carbonate;

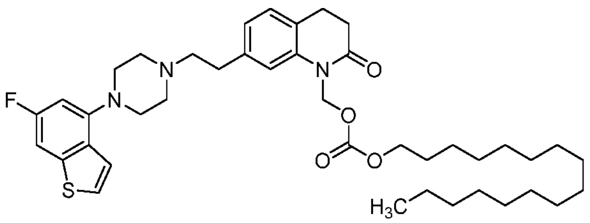

(32) (7-(2-(4-(6-fluorobenzo[*b*]thiophen-4-yl)piperazin-1-yl)ethyl)-2-oxo-3,4-dihydroquinolin-1(2*H*)-yl)methyl phenyl carbonate;

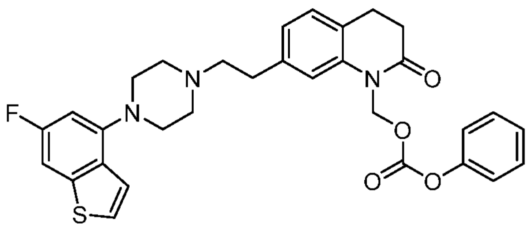

(33) (7-(2-(4-(6-fluorobenzo[*b*]thiophen-4-yl)piperazin-1-yl)ethyl)-2-oxoquinolin-1(2*H*)-yl)methyl phenyl carbonate;

(34)

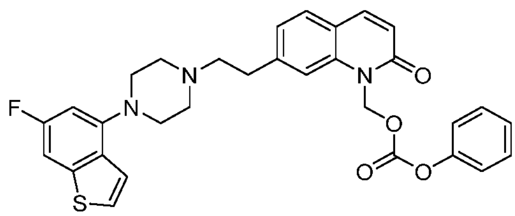

benzyl ((7-(2-(4-(6-fluorobenzo[*b*]thiophen-4-yl)piperazin-1-yl)ethyl)-2-oxo-3,4-dihydroquinolin-1(2*H*)-yl)methyl) carbonate;

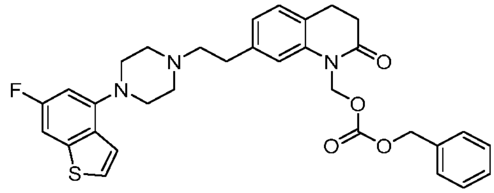

(35) (7-(2-(4-(6-fluorobenzo[*b*]thiophen-4-yl)piperazin-1-yl)ethyl)-2-oxo-3,4-dihydroquinolin-1(2*H*)-yl)methyl (2,2,2-trifluoroethyl) carbonate;

(36)

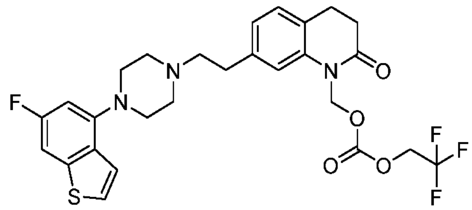

cyclohexyl ((7-(2-(4-(6-fluorobenzo[*b*]thiophen-4-yl)piperazin-1-yl)ethyl)-2-oxo-3,4-dihydroquinolin-1(2*H*)-yl) methyl) carbonate;

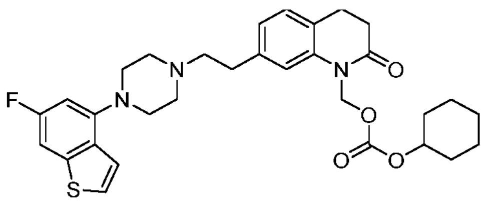

(37) (4-fluoro-7-(2-(4-(6-fluorobenzo[*b*]thiophen-4-yl)piperazin-1-yl)ethyl)-2-oxoquinolin-1(2*H*)-yl)methyl hexanoate;

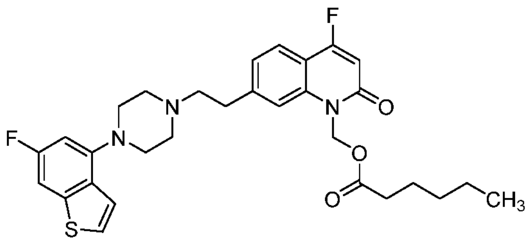

(38) (4-fluoro-7-(2-(4-(6-fluorobenzo[*b*]thiophen-4-yl)piperazin-1-yl)ethyl)-2-oxoquinolin-1(2*H*)-yl)methyl dodecanoate;

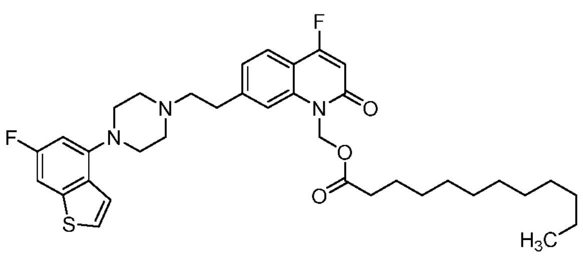

(39) (4-chloro-7-(2-(4-(6-fluorobenzo[*b*]thiophen-4-yl)piperazin-1-yl)ethyl)-2-oxoquinolin-1(2*H*)-yl)methyl hexanoate;

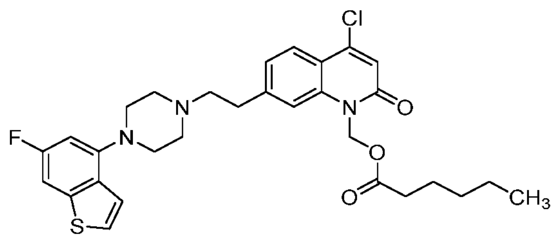

(40) (4-amino-7-(2-(4-(6-fluorobenzo[*b*]thiophen-4-yl)piperazin-1-yl)ethyl)-2-oxoquinolin-1(2*H*)-yl)methyl hexanoate;

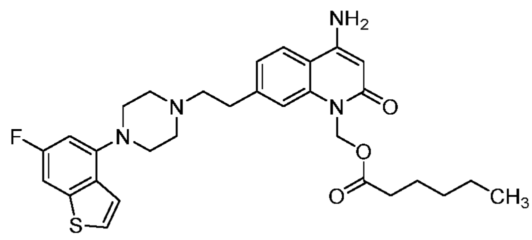

(41) (4-acetamido-7-(2-(4-(6-fluorobenzo[*b*]thiophen-4-yl)piperazin-1-yl)ethyl)-2-oxoquinolin-1(2*H*)-yl)methyl hexanoate;

(42)

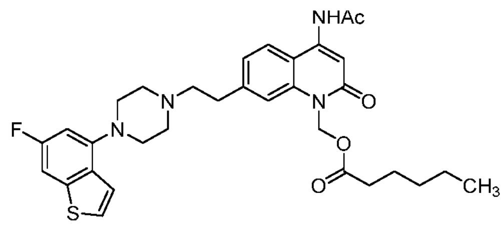

(7-(2-(4-(6-fluorobenzo[*b*]thiophen-4-yl)piperazin-1-yl)ethyl)-4,4-dimethyl-2-oxo-3,4-dihydroquinolin-1(2*H* )-yl) methyl hexanoate;

(43)

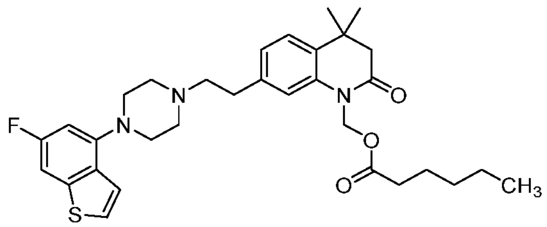

(7-(2-(4-(6-fluorobenzo[*b*]thiophen-4-yl)piperazin-1-yl)ethyl)-4,4-dimethyl-2-oxo-3,4-dihydroquinolin-1(2*H* )-yl) methyl dodecanoate;

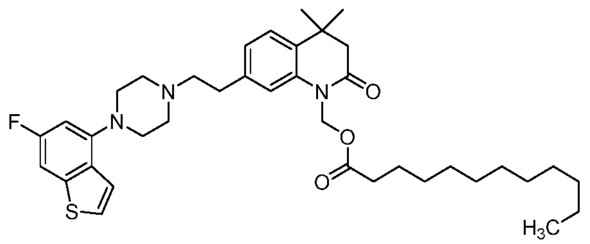

(44) (7-(2-(4-(6-fluorobenzo[*b*]thiophen-4-yl)piperazin-1-yl)ethyl)-4-methyl-2-oxoquinolin-1(2*H*)-yl)methyl hexanoate;

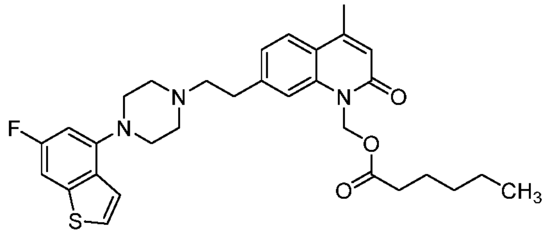

(45) (7-(2-(4-(6-fluorobenzo[*b*]thiophen-4-yl)piperazin-1-yl)ethyl)-3-methyl-2-oxoquinolin-1(2*H*)-yl)methyl hexanoate;

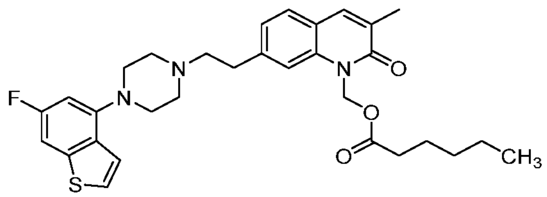

(46) (3-ethyl-7-(2-(4-(6-fluorobenzo[b]thiophen-4-yl)piperazin-1-yl)ethyl)-2-oxoquinolin-1(2H)-yl)methyl hexanoate

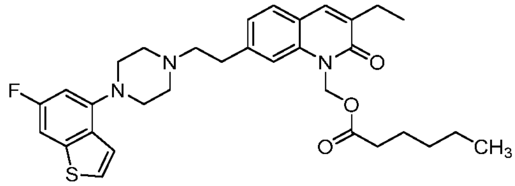

(47) (7-(2-(4-(6-fluorobenzo[b]thiophen-4-yl)piperazin-1-yl)ethyl)-3-hydroxy-2-oxoquinolin-1(2*H*)-yl)methyl hexanoate;

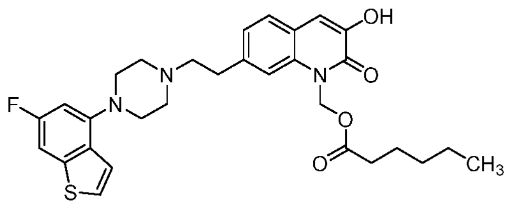

(48) (7-(2-(4-(6-fluorobenzo[*b*]thiophen-4-yl)piperazin-1-yl)ethyl)-4-hydroxy-2-oxoquinolin-1(2*H*)-yl)methyl hexanoate;

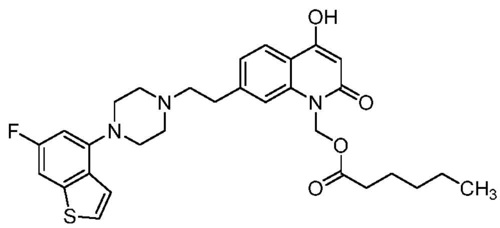

(49) (7-(2-(4-(6-fluorobenzo[b]thiophen-4-yl)piperazin-1-yl)ethyl)-4-hydroxy-2-oxoquinolin-1(2*H*)-yl)methyl dodecanoate;

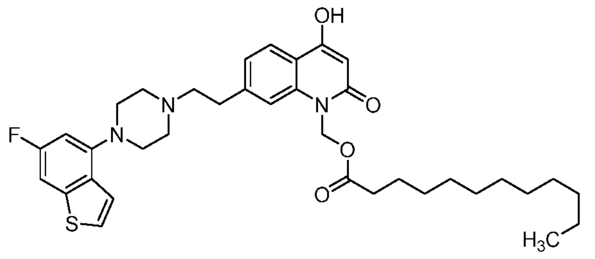

(50) (7-(2-(4-(6-fluorobenzo[*b*]thiophen-4-yl)piperazin-1-yl)ethyl)-2-oxoquinolin-1(2*H*)-yl-4-d)methyl hexanoate;

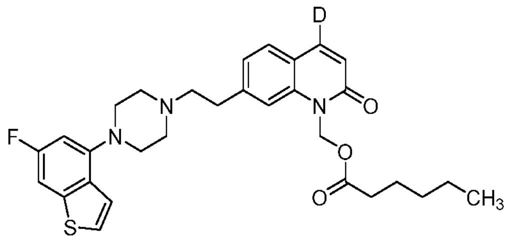

(51) (7-(2-(4-(6-fluorobenzo[*b*]thiophen-4-yl)piperazin-1-yl)ethyl)-2-oxoquinolin-1(2*H*)-yl-4-d)methyl dodecanoate;

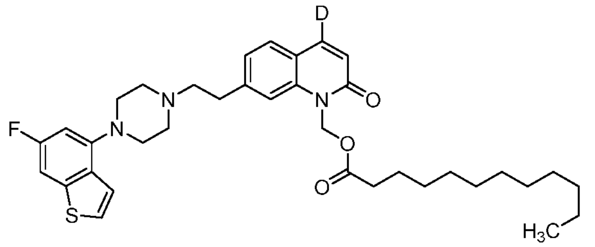

(52) 7-(2-(4-(6-fluorobenzo[b]thiophen-4-yl)piperazin-1-yl)ethyl)-1-(isopropoxymethyl)-3,4-dihydroquinolin-2(1*H*)-one;

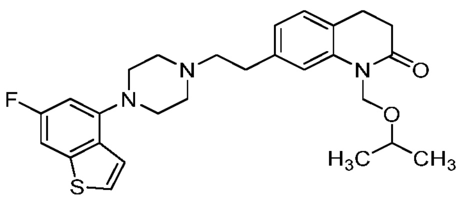

(53) 7-(2-(4-(6-fluorobenzo[*b*]thiophen-4-yl)piperazin-1-yl)ethyl)-1-(isopropoxymethyl)quinolin-2(1*H*)-one;

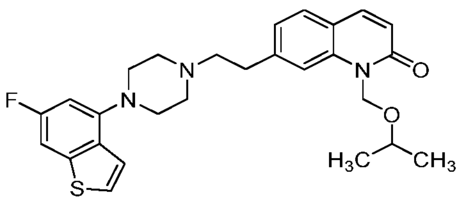

(54) 7-(2-(4-(6-fluorobenzo[*b*]thiophen-4-yl)piperazin-1-yl)ethyl)-1-((2,2,2-trifluoroethoxy)methyl)-3,4-dihydroq uinolin-2(1*H*)-one;

(55)

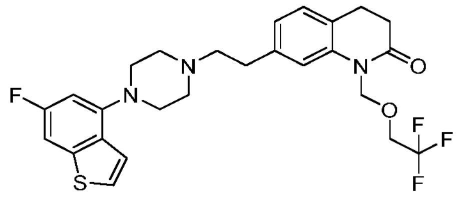

1-((benzyloxy)methyl)-7-(2-(4-(6-fluorobenzo[b]thiophen-4-yl)piperazin-1-yl)ethyl)-3,4-dihydroquinolin-2(1H )-one;

(56)

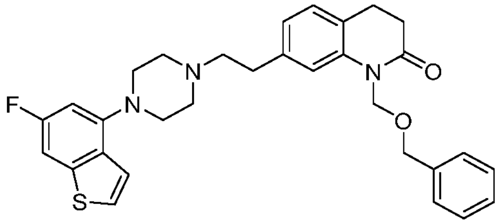

1-(((benzyloxy)methoxy)methyl)-7-(2-(4-(6-fluorobenzo[*b*]thiophen-4-yl)piperazin-1-yl)ethyl)-3,4-dihydroquinolin-2(1*H* )-one;

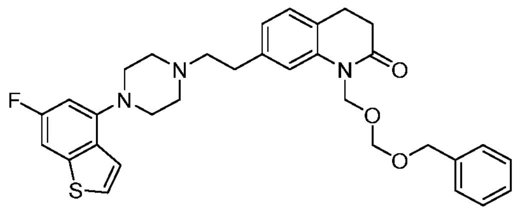

(57) (7-(2-(4-(6-fluorobenzo[*b*]thiophen-4-yl)piperazin-1-yl)ethyl)-2-oxo-3,4-dihydroquinolin-1(2*H*)-yl)methyl propionate;

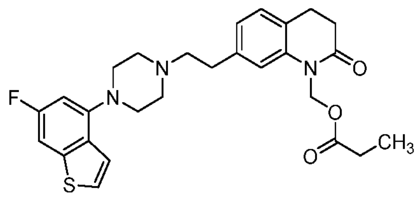

(58) (7-(2-(4-(6-fluorobenzo[*b*]thiophen-4-yl)piperazin-1-yl)ethyl)-2-oxo-3,4-dihydroquinolin-1(2*H*)-yl)methyl butyrate;

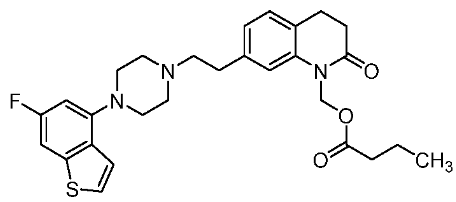

(59) (7-(2-(4-(6-fluorobenzo[*b*]thiophen-4-yl)piperazin-1-yl)ethyl)-2-oxo-3,4-dihydroquinolin-1(2*H*)-yl)methyl 2-methoxyacetate;

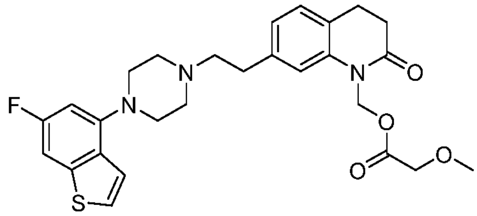

(60) (7-(2-(4-(6-fluorobenzo[*b*]thiophen-4-yl)piperazin-1-yl)ethyl)-2-oxo-3,4-dihydroquinolin-1(2*H*)-yl)methyl heptanoate;

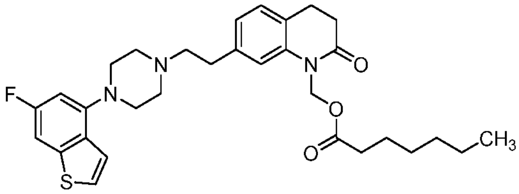

(61) (7-(2-(4-(6-fluorobenzo[*b*]thiophen-4-yl)piperazin-1-yl)ethyl)-2-oxo-3,4-dihydroquinolin-1(2*H*)-yl)methyl octanoate;

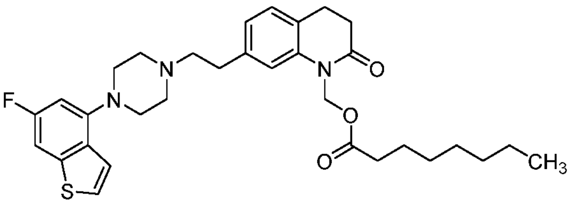

(62) (7-(2-(4-(6-fluorobenzo[*b*]thiophen-4-yl)piperazin-1-yl)ethyl)-2-oxo-3,4-dihydroquinolin-1(2*H*)-yl)methyl nonanoate;

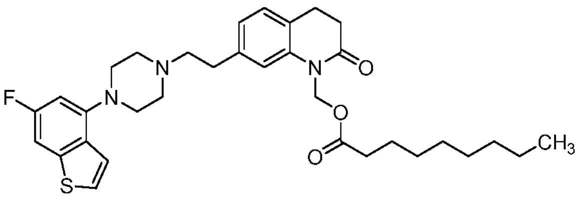

(63) (7-(2-(4-(6-fluorobenzo[*b*]thiophen-4-yl)piperazin-1-yl)ethyl)-2-oxo-3,4-dihydroquinolin-1(2*H*)-yl)methyl undecanoate;

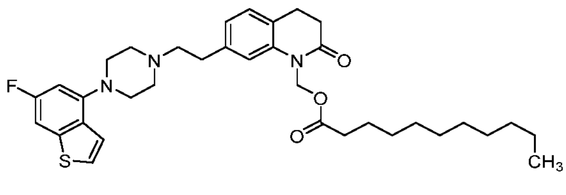

(64) (7-(2-(4-(6-fluorobenzo[*b*]thiophen-4-yl)piperazin-1-yl)ethyl)-2-oxo-3,4-dihydroquinolin-1(2*H*)-yl)methyl tridecanoate

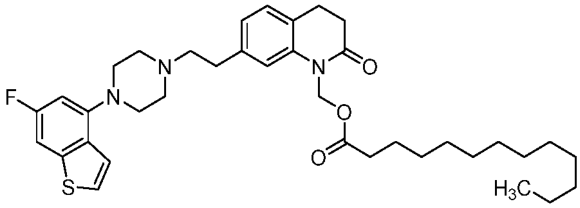

(65) (7-(2-(4-(6-fluorobenzo[*b*]thiophen-4-yl)piperazin-1-yl)ethyl)-2-oxo-3,4-dihydroquinolin-1(2*H*)-yl)methyl tetradecanoate;

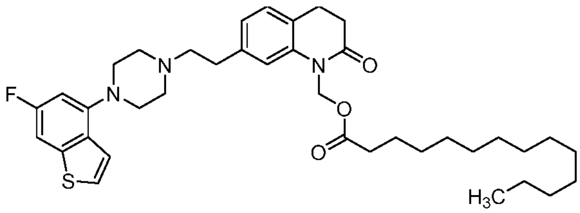

(66) (7-(2-(4-(6-fluorobenzo[*b*]thiophen-4-yl)piperazin-1-yl)ethyl)-2-oxo-3,4-dihydroquinolin-1(2*H*)-yl)methyl pentadecanoate;

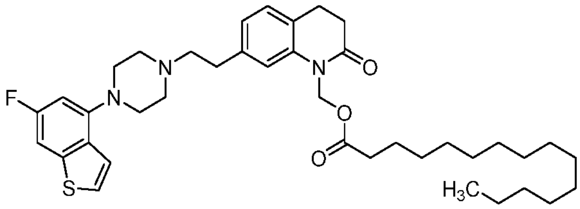

(67) (7-(2-(4-(6-fluorobenzo[*b*]thiophen-4-yl)piperazin-1-yl)ethyl)-2-oxo-3,4-dihydroquinolin-1(2*H*)-yl)methyl heptadecanoate;

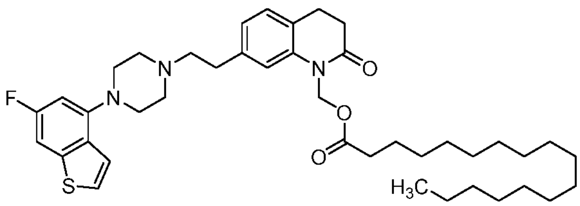

(68) (7-(2-(4-(6-fluorobenzo[*b*]thiophen-4-yl)piperazin-1-yl)ethyl)-2-oxo-3,4-dihydroquinolin-1(2*H*)-yl)methyl stearate;

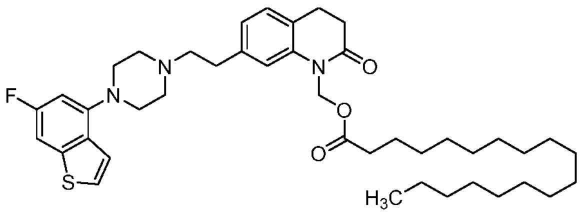

(69) (7-(2-(4-(6-fluorobenzo[*b*]thiophen-4-yl)piperazin-1-yl)ethyl)-2-oxo-3,4-dihydroquinolin-1(2*H*)-yl)methyl icosanoate;

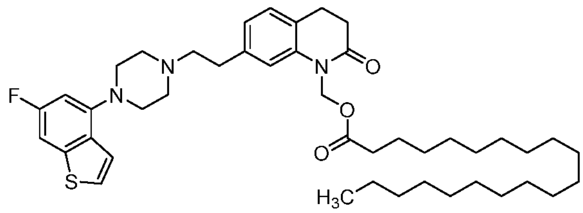

(70) (7-(2-(4-(6-fluorobenzo[*b*]thiophen-4-yl)piperazin-1-yl)ethyl)-2-oxo-3,4-dihydroquinolin-1(2*H*)-yl)methyl allylcarbamate;

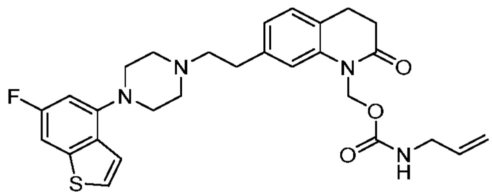

(71) (7-(2-(4-(6-fluorobenzo[*b*]thiophen-4-yl)piperazin-1-yl)ethyl)-2-oxo-3,4-dihydroquinolin-1(2*H*)-yl)methyl decylcarbamate;

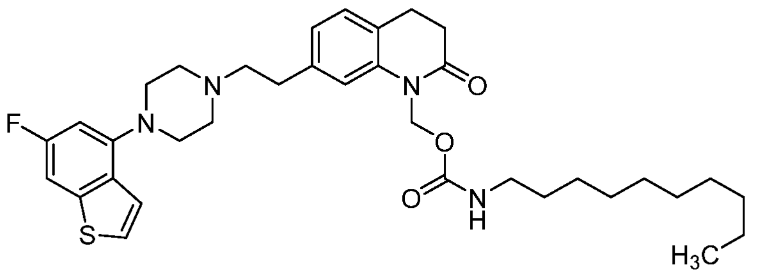

(72) (7-(2-(4-(6-fluorobenzo[*b*]thiophen-4-yl)piperazin-1-yl)ethyl)-2-oxo-3,4-dihydroquinolin-1(2*H*)-yl)methyl undec-10-enoate;

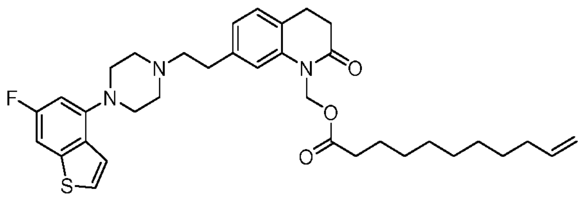

(73) (7-(2-(4-(6-fluorobenzo[*b*]thiophen-4-yl)piperazin-1-yl)ethyl)-2-oxo-3,4-dihydroquinolin-1(2*H*)-yl)methyl oleate;

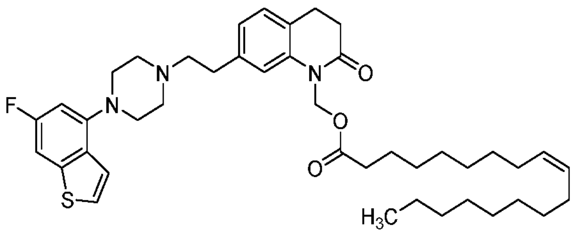

(74) (7-(2-(4-(6-fluorobenzo[*b*]thiophen-4-yl)piperazin-1-yl)ethyl)-2-oxo-3,4-dihydroquinolin-1(2*H*)-yl)methyl docosanoate

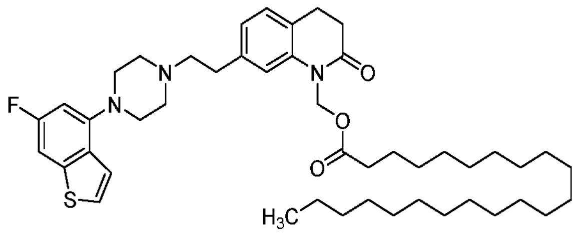

(75) (7-(2-(4-(6-fluorobenzo[*b*]thiophen-4-yl)piperazin-1-yl)ethyl)-2-oxo-3,4-dihydroquinolin-1(2*H*)-yl)methyl tetracosanoate;

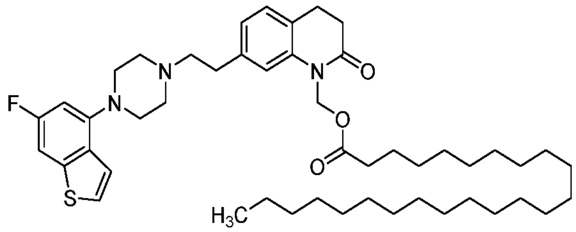

(76) (7-(2-(4-(6-fluorobenzo[*b*]thiophen-4-yl)piperazin-1-yl)ethyl)-2-oxo-3,4-dihydroquinolin-1(2*H*)-yl)methyl heptacosanoate;

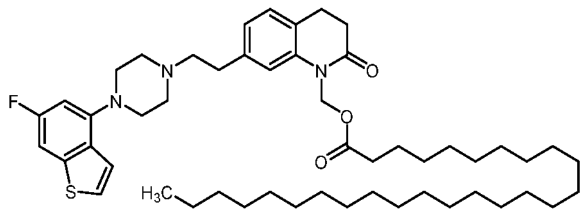

(77) (7-(2-(4-(6-fluorobenzo[*b*]thiophen-4-yl)piperazin-1-yl)ethyl)-2-oxoquinolin-1(2*H*)-yl)methyl oleate;

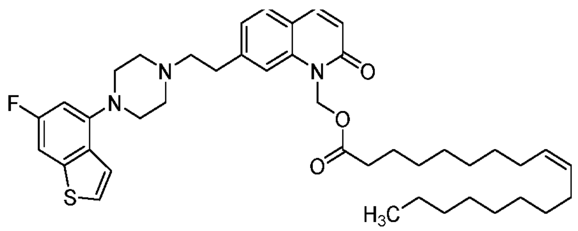

(78) (7-(2-(4-(6-fluorobenzo[*b*]thiophen-4-yl)piperazin-1-yl)ethyl)-2-oxo-3,4-dihydroquinolin-1(2*H*)-yl)methyl cyclobutanecarboxylate;

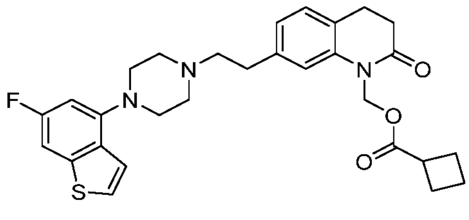

(79) (7-(2-(4-(6-fluorobenzo[*b*]thiophen-4-yl)piperazin-1-yl)ethyl)-2-oxo-3,4-dihydroquinolin-1(2*H*)-yl)methyl cyclopentanecarboxylate;

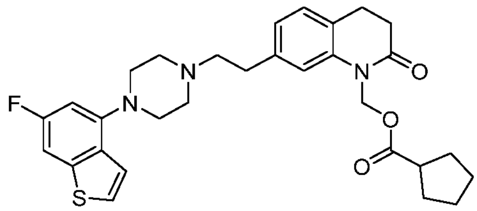

(80) (7-(2-(4-(6-fluorobenzo[*b*]thiophen-4-yl)piperazin-1-yl)ethyl)-2-oxo-3,4-dihydroquinolin-1(2*H*)-yl)methyl cyclohexanecarboxylate;

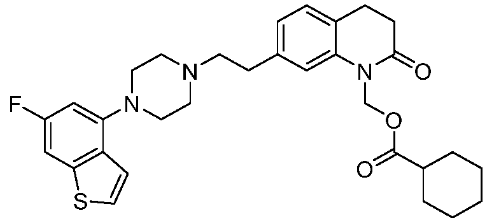

(81) (7-(2-(4-(6-fluorobenzo[*b*]thiophen-4-yl)piperazin-1-yl)ethyl)-2-oxo-3,4-dihydroquinolin-1(2*H*)-yl)methyl piperidine-1 -carboxylate;

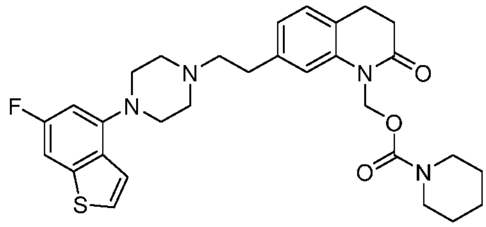

(82) (7-(2-(4-(6-fluorobenzo[*b*]thiophen-4-yl)piperazin-1-yl)ethyl)-2-oxo-3,4-dihydroquinolin-1(2*H*)-yl)methyl cycloheptanecarboxylate;

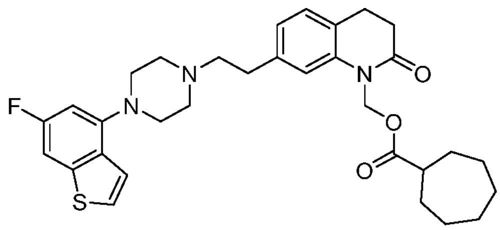

(83) (7-(2-(4-(6-fluorobenzo[*b*]thiophen-4-yl)piperazin-1-yl)ethyl)-2-oxo-3,4-dihydroquinolin-1(2*H*)-yl)methyl cyclohexylcarbamate;

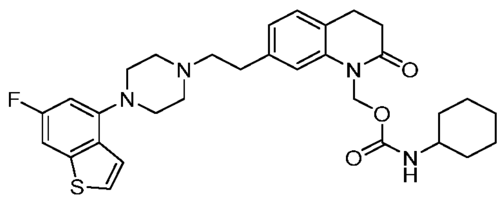

(84) (7-(2-(4-(6-fluorobenzo[*b*]thiophen-4-yl)piperazin-1-yl)ethyl)-2-oxo-3,4-dihydroquinolin-1(2*H*)-yl)methyl (3,3,3 -trifluoropropyl)carbamate;

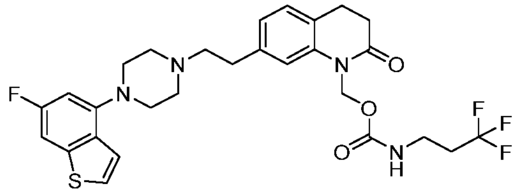

(85) (7-(2-(4-(6-fluorobenzo[*b*]thiophen-4-yl)piperazin-1-yl)ethyl)-2-oxo-3,4-dihydroquinolin-1(2*H*)-yl)methyl (cyclohexylmethyl)carbamate;

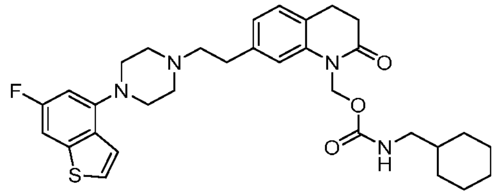

(86) (7-(2-(4-(6-fluorobenzo[*b*]thiophen-4-yl)piperazin-1-yl)ethyl)-2-oxo-3,4-dihydroquinolin-1(2*H*)-yl)methyl furan-2-carboxylate;

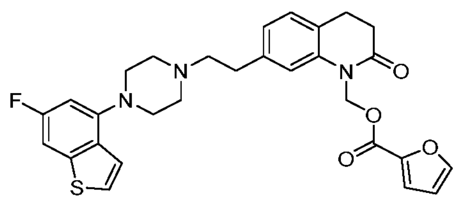

(87) (7-(2-(4-(6-fluorobenzo[*b*]thiophen-4-yl)piperazin-1-yl)ethyl)-2-oxo-3,4-dihydroquinolin-1(2*H*)-yl)methyl furan-3-carboxylate;

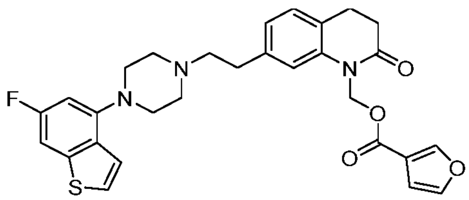

(88) (7-(2-(4-(6-fluorobenzo[*b*]thiophen-4-yl)piperazin-1-yl)ethyl)-2-oxoquinolin-1(2*H*)-yl)methyl furan-2-carboxylate;

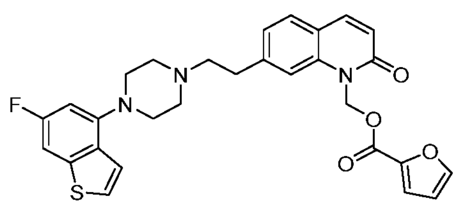

(89) (7-(2-(4-(6-fluorobenzo[*b*]thiophen-4-yl)piperazin-1-yl)ethyl)-2-oxo-3,4-dihydroquinolin-1(2*H*)-yl)methyl thiophene-2-carboxylate;

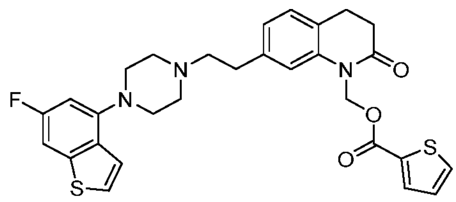

(90) (7-(2-(4-(6-fluorobenzo[*b*]thiophen-4-yl)piperazin-1-yl)ethyl)-2-oxo-3,4-dihydroquinolin-1(2*H*)-yl)methyl tetrahydro-2*H*-pyran-4-carboxylate;

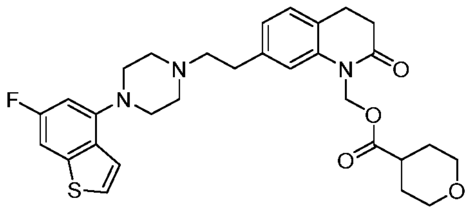

(91) (7-(2-(4-(6-fluorobenzo[*b*]thiophen-4-yl)piperazin-1-yl)ethyl)-2-oxo-3,4-dihydroquinolin-1(2*H*)-yl)methyl picolinate;

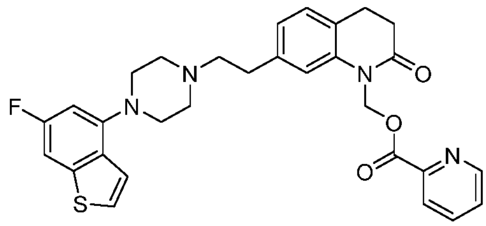

(92) (7-(2-(4-(6-fluorobenzo[*b*]thiophen-4-yl)piperazin-1-yl)ethyl)-2-oxo-3,4-dihydroquinolin-1(2*H*)-yl)methyl nicotinate;

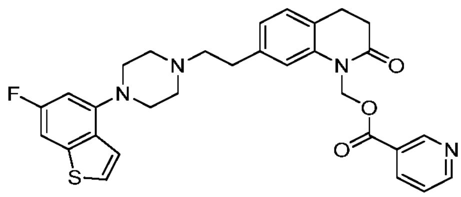

(93) (7-(2-(4-(6-fluorobenzo[*b*]thiophen-4-yl)piperazin-1-yl)ethyl)-2-oxo-3,4-dihydroquinolin-1(2*H*)-yl)methyl pyrimidine-5-carboxylate;

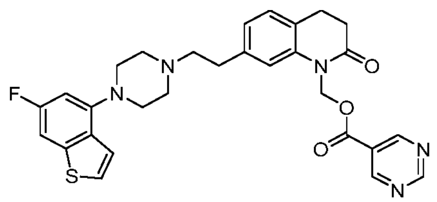

(94) (7-(2-(4-(6-fluorobenzo[*b*]thiophen-4-yl)piperazin-1-yl)ethyl)-2-oxo-3,4-dihydroquinolin-1(2*H*)-yl)methyl quinoline-6-carboxylate;

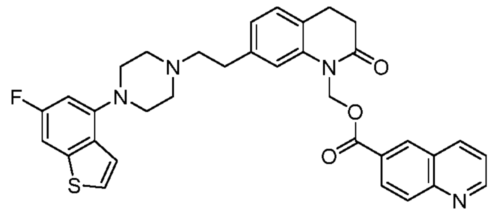

(95) (7-(2-(4-(6-fluorobenzo[*b*]thiophen-4-yl)piperazin-1-yl)ethyl)-2-oxo-3,4-dihydroquinolin-1(2*H*)-yl)methyl benzoate;

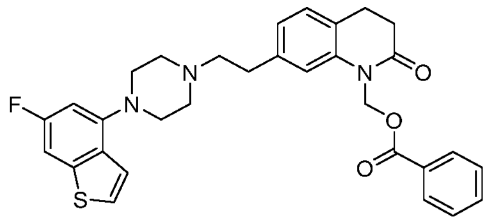

(96) (7-(2-(4-(6-fluorobenzo[*b*]thiophen-4-yl)piperazin-1-yl)ethyl)-2-oxo-3,4-dihydroquinolin-1(2*H*)-yl)methyl 2-phenylacetate;

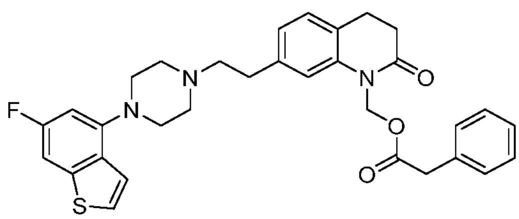

(97) (7-(2-(4-(6-fluorobenzo[*b*]thiophen-4-yl)piperazin-1-yl)ethyl)-2-oxo-3,4-dihydroquinolin-1(2*H*)-yl)methyl 4,4,4-trifluorobutanoate;

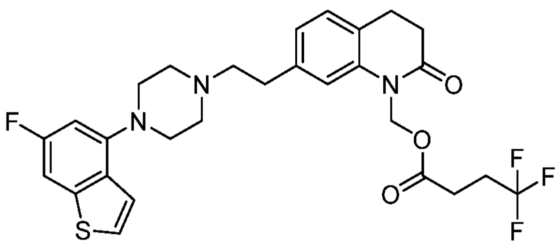

(98) (7-(2-(4-(6-fluorobenzo[*b*]thiophen-4-yl)piperazin-1-yl)ethyl)-2-oxo-3,4-dihydroquinolin-1(2*H*)-yl)methyl benzylcarbamate;

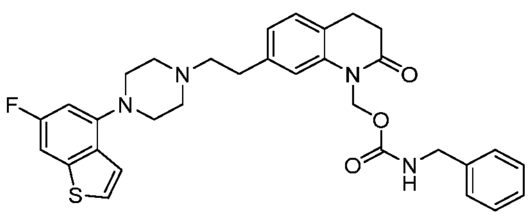

(99) (7-(2-(4-(6-fluorobenzo[*b*]thiophen-4-yl)piperazin-1-yl)ethyl)-2-oxo-3,4-dihydroquinolin-1(2*H*)-yl)methyl (2-hydroxyethyl)carbamate;

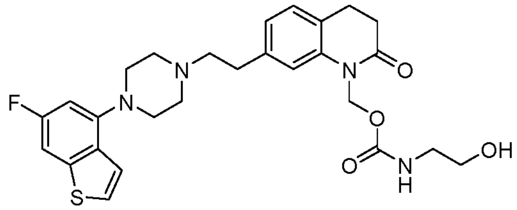

(100) (7-(2-(4-(6-fluorobenzo[*b*]thiophen-4-yl)piperazin-1-yl)ethyl)-2-oxo-3,4-dihydroquinolin-1(2*H*)-yl)methyl heptylcarbamate;

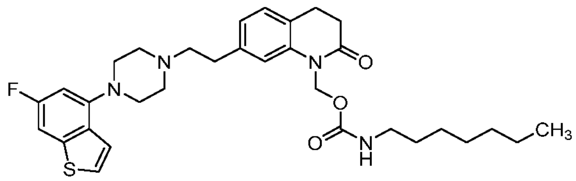

(101) (7-(2-(4-(6-fluorobenzo[*b*]thiophen-4-yl)piperazin-1-yl)ethyl)-2-oxo-3,4-dihydroquinolin-1(2*H*)-yl)methyl 2-(2-methoxyethoxy)acetate;

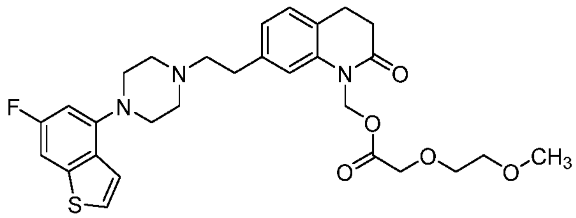

(102) (7-(2-(4-(6-fluorobenzo[*b*]thiophen-4-yl)piperazin-1-yl)ethyl)-2-oxo-3,4-dihydroquinolin-1(2*H*)-yl)methyl 2-(2-(2-methoxyethoxy)ethoxy)acetate;

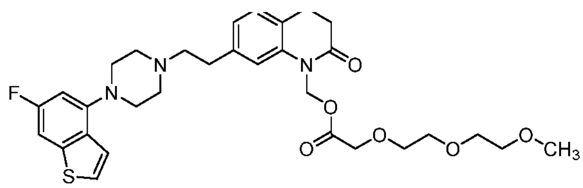

(103) (7-(2-(4-(6-fluorobenzo[*b*]thiophen-4-yl)piperazin-1-yl)ethyl)-2-oxo-3,4-dihydroquinolin-1(2*H*)-yl)methyl 2-(2-butoxyethoxy)acetate;

(104)

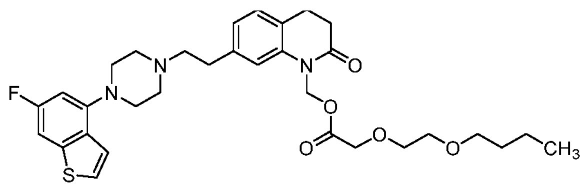

tert-butyl ((7-(2-(4-(6-fluorobenzo[*b*]thiophen-4-yl)piperazin-1-yl)ethyl)-2-oxo-3,4-dihydroquinolin-1(2*H*)-yl)methyl) malonate

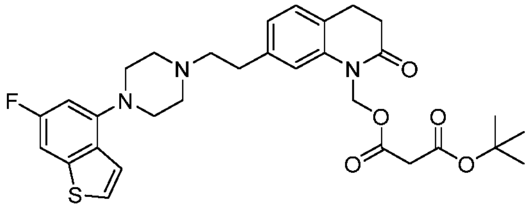

(105) (7-(2-(4-(6-fluorobenzo[*b*]thiophen-4-yl)piperazin-1-yl)ethyl)-2-oxo-3,4-dihydroquinolin-1(2*H*)-yl)methyl 2-methylbutanoate;

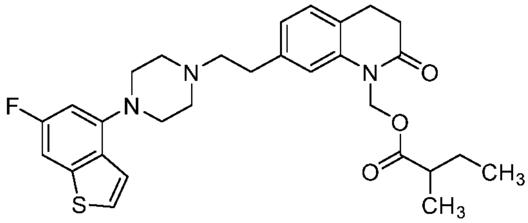

(106) (7-(2-(4-(6-fluorobenzo[*b*]thiophen-4-yl)piperazin-1-yl)ethyl)-2-oxo-3,4-dihydroquinolin-1(2*H*)-yl)methyl 4-methylpentanoate;

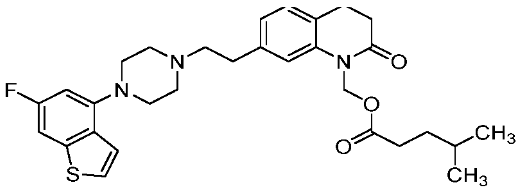

(107) (7-(2-(4-(6-fluorobenzo[*b*]thiophen-4-yl)piperazin-1-yl)ethyl)-2-oxo-3,4-dihydroquinolin-1(2*H*)-yl)methyl 2-methylpentanoate;

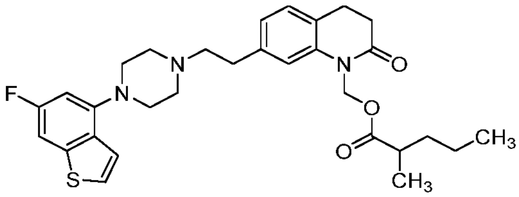

(108) (7-(2-(4-(6-fluorobenzo[*b*]thiophen-4-yl)piperazin-1-yl)ethyl)-2-oxo-3,4-dihydroquinolin-1(2*H*)-yl)methyl 2,2-dimethylhexanoate;

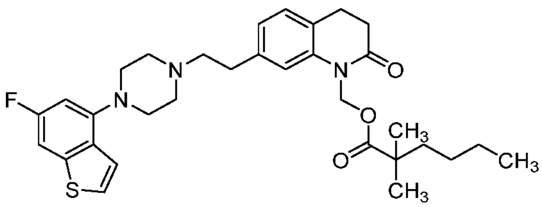

(109) (7-(2-(4-(6-fluorobenzo[*b*]thiophen-4-yl)piperazin-1-yl)ethyl)-2-oxo-3,4-dihydroquinolin-1(2*H*)-yl)methyl 2-methylhexanoate;

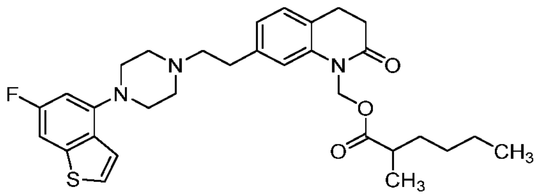

(110) (7-(2-(4-(6-fluorobenzo[*b*]thiophen-4-yl)piperazin-1-yl)ethyl)-2-oxo-3,4-dihydroquinolin-1(2*H*)-yl)methyl 2-methylheptanoate;

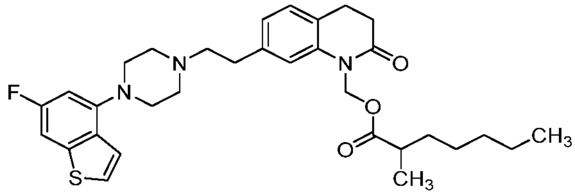

(111) (7-(2-(4-(6-fluorobenzo[b]thiophen-4-yl)piperazin-1-yl)ethyl)-2-oxoquinolin-1(2*H*)-yl)methyl pivalate;

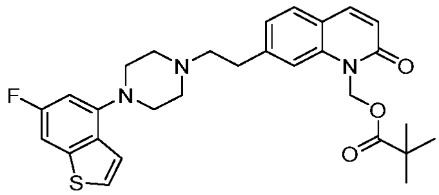

(112) (7-(2-(4-(6-fluorobenzo[*b*]thiophen-4-yl)piperazin-1-yl)ethyl)-2-oxo-3,4-dihydroquinolin-1(2*H*)-yl)methyl 2-pentylheptanoate;

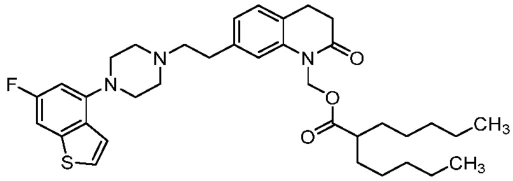

(113) (7-(2-(4-(6-fluorobenzo[*b*]thiophen-4-yl)piperazin-1-yl)ethyl)-2-oxoquinolin-1(2*H*)-yl)methyl 2-pentylheptanoate;

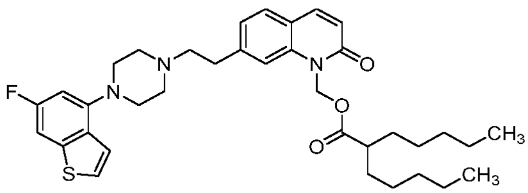

(114) (7-(2-(4-(6-fluorobenzo[*b*]thiophen-4-yl)piperazin-1-yl)ethyl)-2-oxo-3,4-dihydroquinolin-1(2*H*)-yl)methyl glycinate;

(115)

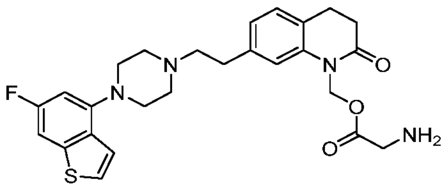

1-benzoyl-7-(2-(4-(6-fluorobenzo[*b*]thiophen-4-yl)piperazin-1-yl)ethyl)-3,4-dihydroquinolin-2(1*H*)-one;

(116)

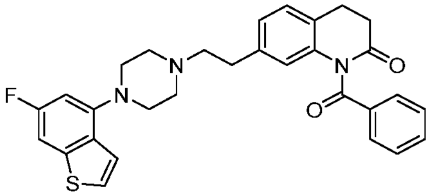

7-(2-(4-(6-fluorobenzo[*b*]thiophen-4-yl)piperazin-1-yl)ethyl)-1-(2-phenylacetyl)-3,4-dihydroquinolin-2(1*H*)-one;

(117)

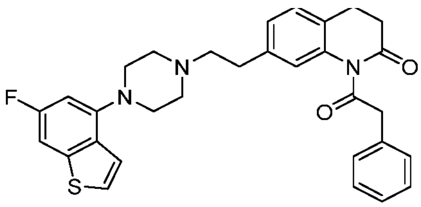

phenyl 7-(2-(4-(6-fluorobenzo[*b*]thiophen-4-yl)piperazin-1-yl)ethyl)-2-oxo-3,4-dihydroquinoline-1(2*H*)-carboxylate

(118)

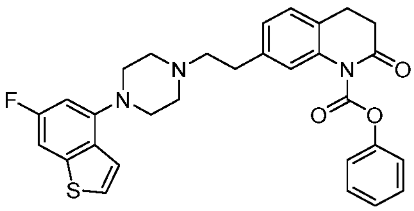

benzyl 7-(2-(4-(6-fluorobenzo[*b*]thiophen-4-yl)piperazin-1-yl)ethyl)-2-oxo-3,4-dihydroquinoline-1(2*H*)-carboxylate ;

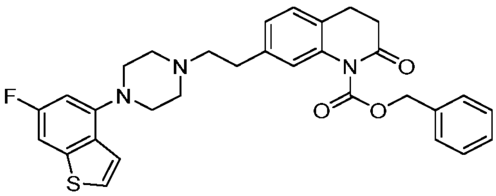

(119) 1-(cyclopentanecarbonyl)-7-(2-(4-(6-fluorobenzo[*b*]thiophen-4-yl)piperazin-1-yl)ethyl)-3,4-dihydroquinolin -2(1*H*)-one;

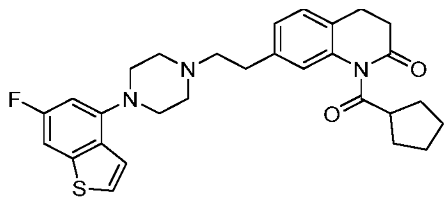

(120) 7-(2-(4-(6-fluorobenzo[*b*]thiophen-4-yl)piperazin-1-yl)ethyl)-1-(2-hydroxyacetyl)-3,4-dihydroquinolin-2(1*H*)-one;

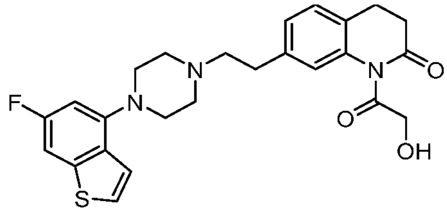

(121) 7-(2-(4-(6-fluorobenzo[*b*]thiophen-4-yl)piperazin-1-yl)ethyl)-1-glycyl-3,4-dihydroquinolin-2(1*H*)-one;

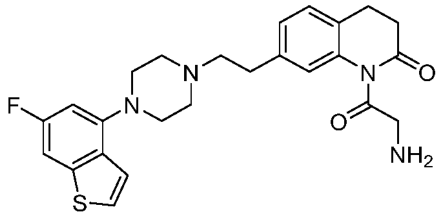

(122) 7-(2-(4-(6-fluorobenzo[*b*]thiophen-4-yl)piperazin-1-yl)ethyl)-*N*-(2-hydroxyethyl)-2-oxo-3,4-dihydroquinoline-1(2*H*) -carboxamide;

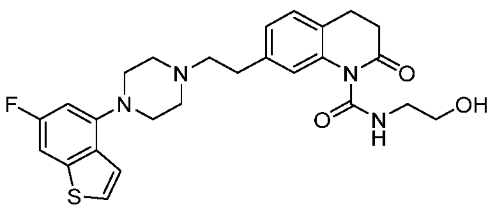

(123) 7-(2-(4-(6-fluorobenzo[*b*]thiophen-4-yl)piperazin-1-yl)ethyl)-2-oxo-*N*-propyl-3,4-dihydroquinoline-1(2*H*)-c arboxamide;

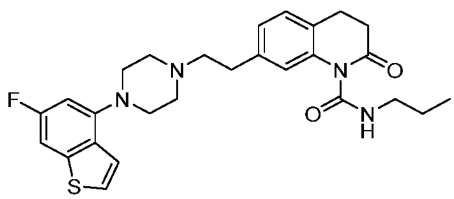

(124) 7-(2-(4-(6-fluorobenzo[*b*]thiophen-4-yl)piperazin-1-yl)ethyl)-1-(2-(2-methoxyethoxy)acetyl)-3,4-dihydroqui nolin-2(1*H*)-one;

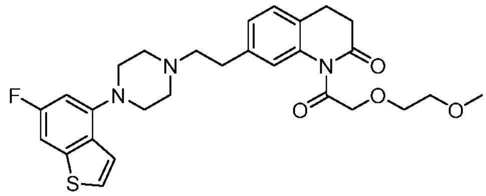

(125) 7-(2-(4-(6-fluorobenzo[*b*]thiophen-4-yl)piperazin-1-yl)ethyl)-1-(2-(2-methoxyethoxy)acetyl)quinolin-2(1*H*)-one;

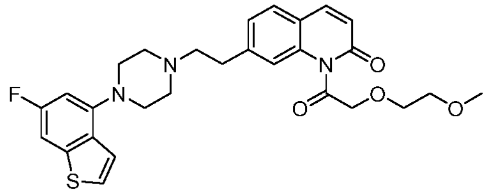

(126) 7-(2-(4-(6-fluorobenzo[*b*]thiophen-4-yl)piperazin-1-yl)ethyl)-1-(2-(2-(2-methoxyethoxy)ethoxy)acetyl)-3,4-dihydroquinolin-2(1*H*)-one;

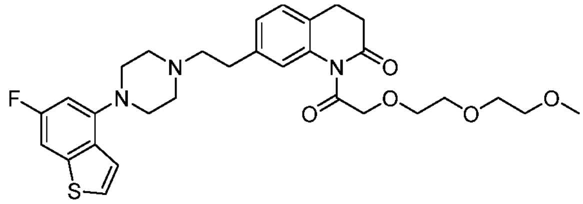

(127) *N*-benzyl-7-(2-(4-(6-fluorobenzo[*b*]thiophen-4-yl)piperazin-1-yl)ethyl)-2-oxo-3,4-dihydroquinoline-1(2*H*)-carboxamide;

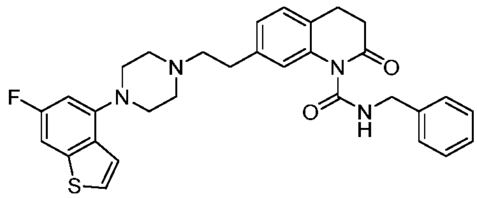

(128) 7-(2-(4-(6-fluorobenzo[*b*]thiophen-4-yl)piperazin-1-yl)ethyl)-1-(morpholine-4-carbonyl)-3,4-dihydroquinolin-2(1*H*) -one;

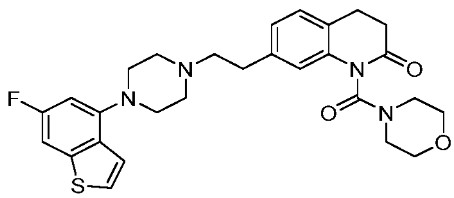

(129) 1-(cyclohexanecarbonyl)-7-(2-(4-(6-fluorobenzo[*b*]thiophen-4-yl)piperazin-1-yl)ethyl)-3,4-dihydroquinolin-2(1*H* )-one;

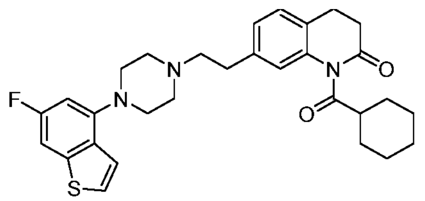

(130) 7-(2-(4-(6-fluorobenzo[*b*]thiophen-4-yl)piperazin-1-yl)ethyl)-1-(2-methoxyacetyl)-3,4-dihydroquinolin-2(1*H*)-one;

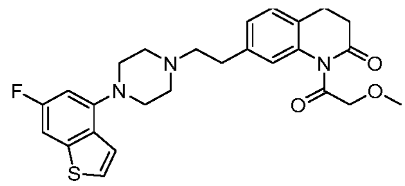

(131) 2-methoxyethyl 7-(2-(4-(6-fluorobenzo[*b*]thiophen-4-yl)piperazin-1-yl)ethyl)-2-oxo-3,4-dihydroquinoline-1(2*H*)-carboxylate;

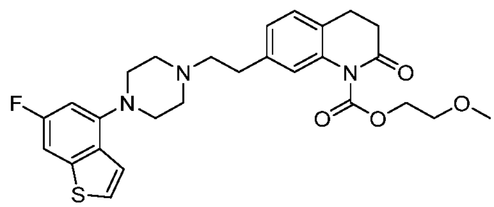

(132) 7-(2-(4-(6-fluorobenzo[*b*]thiophen-4-yl)piperazin-1-yl)ethyl)-1-(undec-10-enoyl)-3,4-dihydroquinolin-2(1*H*)-one

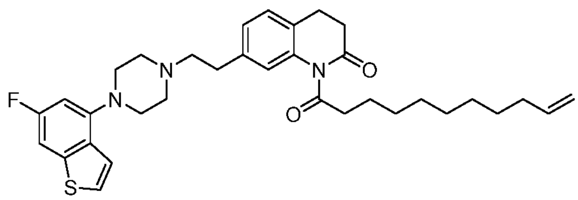

(133) (decanoyloxy)methyl 7-(2-(4-(6-fluorobenzo[*b*]thiophen-4-yl)piperazin-1-yl)ethyl)-2-oxo-3,4-dihydroquinoline-1(2*H*)-carboxylate ; and

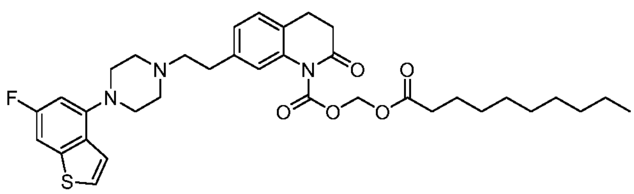

(134) (7-(2-(4-(6-fluorobenzo[*b*]thiophen-4-yl)piperazin-1-yl)ethyl)-2-oxoquinolin-1(2*H*)-yl)methyl dodecanoate;

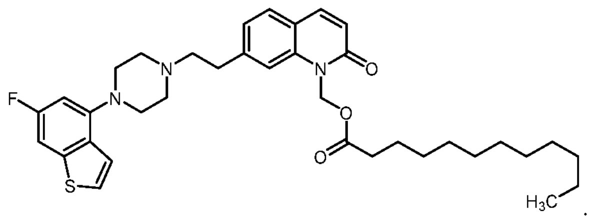

In a third aspect, the present invention provides a preparation method for the N-substituted quinolinone compound, or the pharmaceutically acceptable salt, the solvate, the stereoisomer, the geometric isomer, the isotopically labeled compound or the prodrug thereof described above, which is any one of methods 1 to 4.

Method 1:

subjecting a compound represented by formula (II) or a salt thereof to a N-alkylation reaction with a compound represented by formula (III) or a salt thereof,

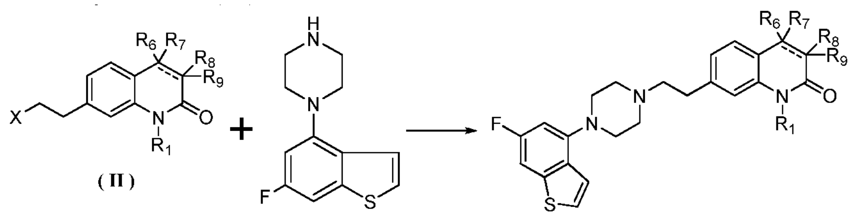

(II) (III) (I)

wherein, - - - - - -, $R_1$, $R_6$, $R_7$, $R_8$, and $R_9$ are as defined above;

X represents a leaving group which is halogen, C1-C6 alkylsulfonyloxy, benzenesulfonyloxy, or naphthalenesulfonyloxy, the C1-C6 alkylsulfonyloxy, benzenesulfonyloxy, and naphthalenesulfonyloxy described above being optionally substituted with one or more substituents selected from halogen, C1-C6 alkyl, C1-C6 alkoxy, nitro, hydroxy, amino, and C1-C6 alkanoyl; preferably, X is halogen, C1-C4 alkylsulfonyloxy, benzenesulfonyloxy, or naphthalenesulfonyloxy, the C1-C4 alkylsulfonyloxy, benzenesulfonyloxy, and naphthalenesulfonyloxy described above being optionally substituted with one or more substituents selected from halogen, C1-C4 alkyl, C1-C4 alkoxy, nitro, hydroxy, amino, and C1-C4 alkanoyl; more preferably, X is chlorine, bromine, methanesulfonyloxy, trifluoromethanesulfonyloxy, benzenesulfonyloxy, naphthalenesulfonyloxy, methylbenzenesulfonyloxy, nitrobenzenesulfonyloxy, aminobenzenesulfonyloxy, chlorobenzenesulfonyloxy, bromobenzenesulfonyloxy, or methoxybenzenesulfonyloxy.

The reaction described above is performed in the presence or absence of a solvent, preferably in a solvent with or without a base.

The solvent includes water; ethers, such as dioxane, tetrahydrofuran, diethyl ether, methyl tert-butyl ether, diisopropyl ether, diethylene glycol dimethyl ether, and ethylene glycol dimethyl ether; aromatic hydrocarbons, such as benzene, toluene, xylene, nitrobenzene, and chlorobenzene; alcohols, such as methanol, ethanol, isopropanol, butanol, tert-butanol, and ethylene glycol; ketones, such as acetone, methyl ethyl ketone, and 4-methyl-2-pentanone; amides, such as *N*,*N*-dimethylformamide, *N*,*N*-dimethylacetamide, and 1-methyl-2-pyrrolidone; halogenated hydrocarbons, such as chloroform, dichloromethane, dichloroethane, and carbon tetrachloride; esters, such as ethyl acetate, ethyl formate, methyl acetate, and isopropyl acetate; other types of solvents, such as dimethyl sulfoxide and acetonitrile; or mixtures of the solvents described above.

The base is selected from inorganic bases and organic bases, wherein the inorganic bases include alkali metal hydroxides, such as sodium hydroxide, potassium hydroxide, cesium hydroxide, and lithium hydroxide; alkali metal carbonates, such as sodium carbonate, potassium carbonate, cesium carbonate, and lithium carbonate; alkali metal bicarbonates, such as sodium bicarbonate, potassium bicarbonate, and lithium bicarbonate; alkali metals, such as potassium and sodium; and others, such as sodium amide, potassium amide, sodium hydride, and potassium hydride; the organic bases include sodium methoxide, sodium ethoxide, potassium methoxide, potassium ethoxide, sodium acetate, triethylamine, pyridine, diisopropylamine, diisopropylethylamine, tripropylamine, diethylamine, pyrimidine, quinoline, piperidine, piperazine, imidazole, dimethylaminopyridine, trimethylamine, N-ethyldiisopropylatnine, N-methylmorpholine, dimethylaniline, 1,8-diazabicyclo[5.4.0]undec-7-ene (DBU), 1,5-diazabicyclo[4.3.0]non-5-ene (DBN), and 1,4-diazabicyclo[2.2.2]octane (DABCO). These bases may be used alone or in combination of two or more.

Preferably, the reaction described above is performed in the presence of an alkali metal iodide. The alkali metal iodide acts as a reaction accelerant, including potassium iodide and sodium iodide.

Preferably, the reaction is performed at a temperature of room temperature to 200 °C, preferably from room temperature to 150 °C; the reaction is performed for a period of 1-120 h, preferably 5-80 h.

Method 2:

subjecting a compound represented by formula (IV) or a salt thereof to a coupling reaction with a compound represented by formula (V) or a salt thereof,

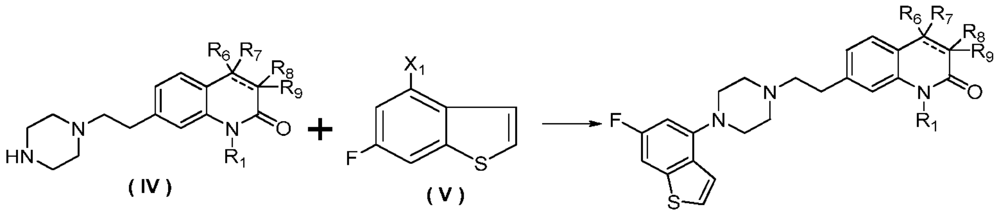

(IV) (V) (I)

wherein, <u>- - - - - -</u>, $R_1$, $R_6$, $R_7$, $R_8$, and $R_9$ are as defined above;

$X_1$ is halogen or trifluoromethanesulfonyloxy, preferably bromine, iodine, chlorine, or trifluoromethanesulfonyloxy; preferably, the coupling reaction is performed in the presence of a palladium catalyst and a base.

The palladium catalyst is one or more of palladium acetate ($Pd(OAc)_2$), bis(triphenylphosphine)palladium(II) dichloride ($(Ph_3P)_2PdCl_2$), bis(benzonitrile)palladium(II) chloride ($(PhCN)_2PdCl_2$), tetrakis(triphenylphosphine)palladium(0) ($Pd(PPh_3)_4$), bis(triphenylphosphine)palladium(II) diacetate ($(Ph_3P)_2Pd(OAc)_2$), [1,2-bis(diphenylphosphino)ethane]palladium(II) dichloride (PdClz(dppe)z), bis[1,2-bis(diphenylphosphino)ethane]palladium(0) ($Pd(dppe)_2$), bis(dibenzylideneacetone)palladium(0) ($Pd(dba)_2$), tris(dibenzylideneacetone)dipalladium(0) ($Pd_2(dba)_3$), [1,3-bis(diphenylphosphino)propane]palladium(II) chloride ($PdCl_2(dippp)$), and [1,1'-bis(diphenylphosphino)ferrocene]palladium(II) dichloride ($Pd(dppf)Cl_2$).

The base is one or more of sodium bis(trimethylsilyl)amide, potassium *tert*-butoxide, sodium *tert*-butoxide, cesium carbonate, potassium phosphate, sodium phosphate, sodium methoxide, sodium ethoxide, potassium hydroxide, sodium hydroxide, potassium fluoride, sodium fluoride, tetrabutylammonium fluoride (TBAF), sodium acetate, potassium acetate, cesium carbonate, potassium carbonate, and sodium carbonate.

The reaction solvent for the reaction described above is not particularly limited as long as it does not interfere with the reaction, and includes water; ethers, such as dioxane and tetrahydrofuran; aromatic hydrocarbons, such as toluene and xylene; alcohols, such as tert-butyl alcohol; ketones, such as acetone; amides, such as *N,N*-dimethylformamide; others, such as dimethyl sulfoxide and acetonitrile; or mixtures of the solvents described above.

Preferably, the reaction described above is performed in the presence of a ligand as an accelerant, which is one or more of 2,2'-bis(diphenylphosphino)-1,1'-binaphthalene (BINAP), tri-*tert*-butylphosphine (P(t-Bu)s), 1,1'-bis(diphenylphosphino)ferrocene (dppf), 2-dicyclohexylphosphino-2',4',6'-triisopropylbiphenyl (x-phos), 4,5-bis(diphenylphosphino)-9,9-dimethylxanthene (Xantphos), tri-*tert*-butylphosphine tetrafluoroborate, and tris(2-methylphenyl)phosphine ($P(o\text{-}tolyl)_3$).

Preferably, the reaction is performed at a temperature of room temperature to 200 °C, preferably room temperature to 150 °C; the reaction is performed for a period of 1-30 h, preferably 5-20 h.

Method 3:

subjecting a compound represented by formula (I-a) or a salt thereof to a substitution reaction with a compound represented by $R_1X$,

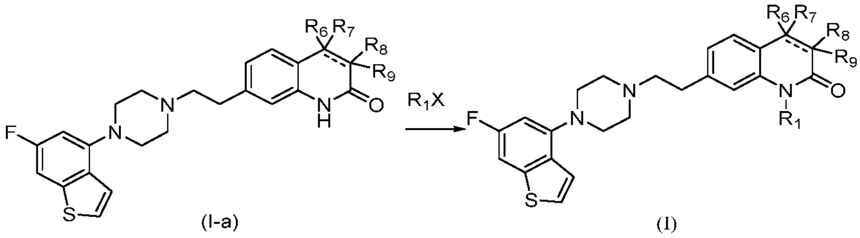


(I-a) (I)

wherein, <u>- - - - - -</u>, $R_1$, $R_6$, $R_7$, $R_8$, and $R_9$ are as defined above;

X is as defined in method 1;

the reaction conditions in method 3 are the same as those in method 1.

Method 4:

subjecting a compound represented by formula (1-b) or a salt thereof to a substitution reaction with a compound represented by $X-C(=O)-R_2$ or $X-O-C(=O)-O-R_3$ or $X-R_4$,

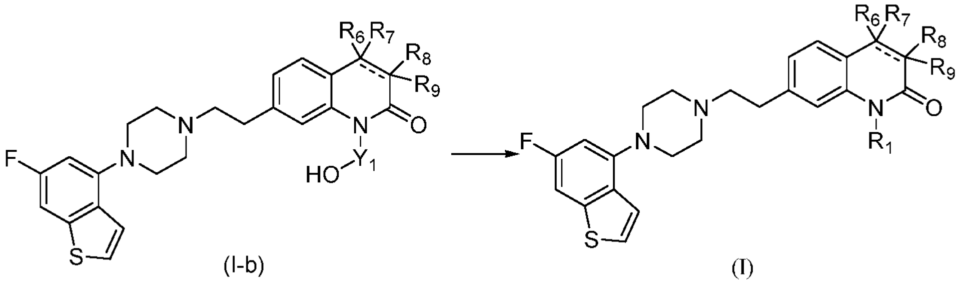

(I-b) (I)

wherein, $R_1$ is $-Y_1-O-C(=O)-R_2$, $-Y_1-O-C(=O)-O-R_3$, or $-Y_1-O-R_4$, and ------, $R_6$, $R_7$, $R_8$, $R_9$, $Y_1$, $R_2$, $R_3$, and $R_4$ are as defined above;

X is halogen, preferably bromine, iodine, or chlorine;

the reaction conditions in method 4 are the same as those in method 1.

The target compounds obtained according to the reaction schemes are isolated and purified from the reaction mixture by the following method: after cooling, isolating a crude product from the reaction mixture by methods such as filtration, extraction, or concentration, and then subjecting the crude product to purification by conventional methods such as column chromatography, slurrying, or recrystallization.

In a fourth aspect, the present invention provides a pharmaceutical composition comprising the N-substituted quinolinone compound, or the pharmaceutically acceptable salt, the solvate, stereoisomer, the geometric isomer, the isotopically labeled compound or the prodrug thereof described above, and at least one pharmaceutically acceptable carrier.

In a fifth aspect, the present invention provides a preparation method for a pharmaceutical composition, which comprises the following steps: mixing the N-substituted quinolinone compound, or the pharmaceutically acceptable salt, the solvate, stereoisomer, the geometric isomer, the isotopically labeled compound or the prodrug thereof described above with a pharmaceutically acceptable carrier.

The pharmaceutical composition of the present invention may be selected from various pharmaceutical formulation forms according to the therapeutic purpose, including but not limited to: tablets, pills, capsules, granules, suspensions, solutions, creams, ointments, powders, suppositories, aerosols, injections (e.g., fat-soluble or oil-soluble injections), and the like.

In a sixth aspect, the present invention provides use of the N-substituted quinolinone compound, or the pharmaceutically acceptable salt, the solvate, stereoisomer, the geometric isomer, the isotopically labeled compound or the prodrug thereof described above, or the pharmaceutical composition described above in preparing a medicament for the prevention and/or treatment of a central nervous system disease, condition, or disorder.

In a seventh aspect, the present invention provides a method for treating and/or preventing a central nervous system disease, condition, or disorder, which comprises administering to a human or animal an effective amount of the N-substituted quinolinone compound, or the pharmaceutically acceptable salt, the solvate, stereoisomer, the geometric isomer, the isotopically labeled compound or the prodrug thereof described above, or the pharmaceutical composition described above.

In an eighth aspect, the present invention provides the N-substituted quinolinone compound, or the pharmaceutically acceptable salt, the solvate, stereoisomer, the geometric isomer, the isotopically labeled compound or the prodrug thereof described above, or the pharmaceutical composition described above, for use as a $5-HT_{1A}$ receptor agonist/$5-HT_{2A}$ receptor antagonist/$D_2$ receptor antagonist/$D_3$ receptor agonist/5-HT transporter inhibitor.

The central nervous system disease, condition, or disorder is selected from schizophrenia (preferably uncontrollable, intractable, or chronic schizophrenia), affective disorder, mental disorder, mood disorder, bipolar I disorder, bipolar II disorder, depressive disorder (preferably intrinsic, severe, or uncontrollable depressive disorder), dysthymic disorder, cyclothymic disorder, panic attack, panic disorder, social phobia, obsessive-compulsive disorder, impulsive disorder, post-traumatic stress disorder, anxiety disorder, acute stress disorder, hysteria, anorexia nervosa, sleep disorder, adaptive disorder, cognitive disorder, autism, neuropathic headache, mania, Parkinson's disease, Huntington's chorea, Alzheimer's disease, dementia, memory disorder, hyperactivity disorder, attention deficit/hyperactivity disorder,

and tic disorder.

Effects of the Present Invention

In order to solve the problem of poor solubility of the compounds in an oily medium in the prior art, the inventors have conducted various studies and found that the liposolubility of the compounds can be significantly improved by introducing a substituent at a specific position of quinolinone. Based on this finding, the present invention has been completed. Beneficial Effects of the Present Invention:

1) Excellent oil solubility

The compounds of the present invention have significantly excellent solubility in oils (such as sesame oil and benzyl benzoate) and can be applied to oil injections. Compared with aqueous suspensions, the oil injections are more advantageous in the following aspects: sustained delivery of plasma concentration (controlled diffusion at the administration site via an oily base), shortened preparation time of a drug solution (no need for mixing and shaking), ensured sterilization by filtration (filterable oily base), avoidance of physical irritation at an administration site (oily base stability), improved accuracy of filling the syringe (container filled with an oily base), and the like.

2) Multi-target effect: good efficacy and few side effects

The compounds of the present invention or *in vivo* metabolites thereof have a multi-target effect on the 5-$HT_{2A}$ receptor, dopamine $D_{2/3}$ receptor, 5-$HT_{1A}$ receptor, and 5-HT transporter. Specifically, they have an antagonistic effect on the 5-$HT_{2A}$ receptor, the $D_2$ receptor, and the 5-HT transporter, and have a partial agonistic effect on the $D_3$ receptor and the 5-$HT_{1A}$ receptor, with $IC_{50}$ or $EC_{50}$ up to 0.1-10 nM, suggesting a significant antagonistic/agonistic effect.

The multi-target effect is characterized by its ability to regulate the DA/5-HT system, which is favorable for regulating the balance of neurotransmitters in the brain, and has relatively good efficacy on various central nervous system diseases. Meanwhile, the multi-target effect is characterized by its ability to reduce or avoid side effects caused by a single $D_2$ receptor antagonist or a $D_2$/5-$HT_{2A}$ receptor antagonist, such as extrapyramidal symptoms (EPS) and hyperprolactinemia.

The compounds of the present invention have a multi-target effect, are superior to the compounds in patent WO2015131856 in terms of druggability, and can be used in the treatment of various central nervous system diseases, especially depressive disorder, major depressive disorder (MDD), manic-depressive psychosis, schizophrenia, negative symptoms of schizophrenia, anxiety disorder, phobia, autism, Alzheimer's disease, bipolar disorder, cognitive dysfunction, Parkinson's disease, hysteria, obsessive-compulsive disorder, hyperactivity disorder, and the like.

The compounds of the present invention or *in-vivo* metabolites thereof have a good inhibitory effect on the 5-HT transporter, and can ameliorate comorbidities such as anxiety or depression of schizophrenia or other central nervous system diseases.

The compounds of the present invention or the *in-vivo* metabolites thereof have a good regulatory effect on the 5-$HT_{1A}$ receptor, can ameliorate negative symptoms and cognitive impairment of schizophrenia, and do not cause central or peripheral side effects caused by complete activation of the 5-$HT_{1A}$ receptor.

3) Better safety and tolerability

Compared to the compounds in patent WO2015131856, the compounds of the present invention have a lower hERG inhibitory effect and are expected to be more clinically safe.

The compounds of the present invention can slowly release active metabolites *in vivo,* with little fluctuation in plasma concentration, thereby improving the safety and tolerability of drugs. Since the compounds of the present invention can be converted into active metabolites having a multi-target effect in blood and are also excellent in maintaining the plasma concentration of active metabolites having a desired therapeutic effect for a long period of time, the compounds of the present invention exhibit excellent metabolic stability and plasma concentration persistence, and are more suitable for the preparation of long-acting formulations.

The compounds of the present invention are effective for oral administration, can be prepared into oral formulations or oil injections, have a long-acting effect, and have the characteristics of low drug effect dosage, small toxic and side effects, and the like, thereby improving the drug compliance of patients.

4) Easy crystallization and excellent stability

The compounds of the present invention are easy to crystallize, simple to operate, and have excellent chemical

stability.

5) Good effect in combination therapy

The compounds of the present invention can exert effects that cannot be achieved by conventional treatments, such as reducing the dosage, ameliorating side effects, and improving the therapeutic efficacy, by administering in combination with at least one clinically used drug selected from (1) mood stabilizers, (2) serotonin reuptake inhibitors, (3) norepinephrine reuptake inhibitors, (4) serotonin and norepinephrine reuptake inhibitors, and (5) antidepressants.

In summary, compared with the existing antipsychotic drugs, the compounds of the present invention have the advantages of excellent oil solubility, multi-target effect, lower drug effect dosage, less toxic and side effect, better safety and tolerability, and the like, has good general druggability, and has good clinical application prospects.

## BRIEF DESCRIPTION OF THE DRAWINGS

FIG. 1 is a graph of time-active metabolite concentration curves after intramuscular injection of compounds 3, 5, and 7 in SD rats.

FIG. 2 is a graph of time-active metabolite concentration curves after intramuscular injection of compounds 2, 6, and 8 in SD rats.

## DETAILED DESCRIPTION

The definitions of groups in the present invention are as follows:
Halogen refers to fluorine, chlorine, bromine, and iodine, preferably fluorine, chlorine, or bromine; more preferably fluorine or chlorine.

In the present invention, Cn-Cm indicates that there are n-m carbon atoms, and the following carbon numbers are each representative examples.

Alkyl refers to linear or branched saturated hydrocarbyl, C1-C30 alkyl refers to linear or branched saturated hydrocarbyl containing 1-30 carbon atoms, e.g., C1-C16 alkyl, C1-C12 alkyl, or C1-C6 alkyl, e.g., C1-C30 linear alkyl or C1-C26 linear alkyl, examples of which include methyl, ethyl, *n*-propyl, n-butyl, *n*-pentyl, *n*-hexyl, undecyl, pentadecyl, nonadecyl, tricosyl, and the like, e.g., C1-C30 branched alkyl or C1-C12 branched alkyl, examples of which include isopropyl, isobutyl, *tert*-butyl, *sec*-butyl, 1-methylpropyl, 1-ethylpropyl, isopentyl, neopentyl, 1-methylbutyl, 3-methylbutyl, isohexyl, 1-methylpentyl, 3-methylpentyl, 1,1-dimethylpentyl, *n*-hexyl, 1-methylhexyl, 1-pentylhexyl, and the like, preferably methyl, ethyl, *n*-propyl, isopropyl, *n*-butyl, isobutyl, or *n*-hexadecyl.

C1-C6 alkylene refers to a divalent alkyl group containing 1-6 carbon atoms, e.g., $-CH_2-$, $-CH(CH_3)-$, $-C(CH_3)_2-$, $-CH_2CH_2-$, $-CH_2CH(CH_3)-$, $-CH_2C(CH_3)_2-$, $-CH_2CH_2CH_2-$, $-(CH_2)_6-$, or the like.

Halogenated C1-C30 alkyl refers to linear or branched saturated hydrocarbyl containing 1-30 carbon atoms with hydrogen atoms substituted with 1 or more identical or different halogen atoms, e.g., halogenated C1-C16 alkyl, halogenated C1-C12 alkyl, or halogenated C1-C6 alkyl, examples of which include trifluoromethyl, fluoromethyl, difluoromethyl, chloromethyl, bromomethyl, dichlorofluoromethyl, chloroethyl, bromopropyl, 2-chlorobutyl, pentafluoroethyl, $-(CH_2)_{15}CF_3$, or the like.

C1-C30 alkoxy refers to linear or branched alkoxy containing 1-30 carbon atoms, e.g., C1-C16 alkoxy, C1-C12 alkoxy, or C1-C6 alkoxy, examples of which include methoxy, ethoxy, *n*-propoxy, isopropoxy, *n*-butoxy, isobutoxy, *tert*-butoxy, *sec*-butoxy, *n*-pentoxy, isopentoxy, neopentoxy, isohexoxy, 3-methylpentoxy, *n*-hexoxy, or the like, preferably methoxy, ethoxy, *n*-propoxy, isopropoxy, *n*-butoxy, isobutoxy, or *n*-hexadecyloxy.

Halogenated C1-C30 alkoxy refers to linear or branched alkoxy containing 1-30 carbon atoms with hydrogen atoms substituted with 1 or more identical or different halogen atoms, e.g., halogenated C1-C16 alkoxy, halogenated C1-C12 alkoxy, or halogenated C1-C6 alkoxy, examples of which include, $-OCF_3$, $-OCH_2CH_2Cl$, $-OCHBrCH_2Cl$, $-OCF_2CF_3$, $-O(CH_2)_{15}CF_3$, or the like.

C2-C30 alkenyl refers to linear or branched unsaturated hydrocarbyl containing 1, 2, or 3 double bonds and 2-16 carbon atoms, including both cis- and trans-configurations, e.g., C2-C16 alkenyl, C2-C12 alkenyl, or C2-C6 alkenyl, examples of which include vinyl, 1-propenyl, 2-propenyl, 1-methyl-1-propenyl, 2-methyl-1-propenyl, 2-methyl-2-propenyl, 1-butenyl, 2-butenyl, 3-butenyl, 1-pentenyl, 2-pentenyl, 3-pentenyl, 4-pentenyl, 1,3-butadienyl, 1,3-pentadienyl, 1-hexenyl, 2-hexenyl, 3-hexenyl, 4-hexenyl, 5-hexenyl, 3,3-dimethyl-1-propenyl, 2-ethyl-1-propenyl, or the like.

Phenyl C1-C30 alkyl refers to linear or branched saturated hydrocarbyl containing 1-30 carbon atoms with one carbon atom connected with a carbon atom of phenyl, e.g., phenyl C1-C16 alkyl, phenyl C1-C12 alkyl, or phenyl C1-C6 alkyl, examples of which include benzyl, phenethyl, phenylpropyl, or the like.

Phenyl C1-C30 alkoxy refers to linear or branched alkoxy containing 1-30 carbon atoms with one carbon atom connected with a carbon atom of phenyl, e.g., phenyl C1-C16 alkoxy, phenyl C1-C12 alkoxy, or phenyl C1-C6 alkoxy, examples of which include benzyloxy, $-OCH(CH_3)Ph$, phenethyloxy, phenylpropyloxy, or the like.

Amino substituted with C1-C30 alkyl refers to amino with hydrogen atoms substituted with 1 or 2 identical or different C1-C30 alkyl, e.g., -NHMe, -NHEt, -N(Me)Et, $-NEt_2$, $-NH(CH_2)_{15}CH_3$, or the like.

Amino substituted with C2-C30 alkenyl refers to amino with hydrogen atoms substituted with 1 or 2 identical or different C2-C30 alkenyl, e.g., $-NHCH=CH_2$, $-NHCH=CHCH_3$, $-NHCH=CHCH_2CH_3$, $-NHCH=CH(CH_2)_{13}CH_3$, or the like.

Amino substituted with halogenated C1-C30 alkyl refers to amino with hydrogen atoms substituted with 1 or 2 identical or different halogenated C1-C30 alkyl, e.g., $-NHCH_2CF_3$, $-NHCF_2CF_3$, $-N(CH_2CF_3)_2$, $-NH(CH_2)_{15}CF_3$, or the like.

Amino substituted with phenyl C1-C30 alkyl refers to amino with hydrogen atoms substituted with 1 or 2 identical or different phenyl C1-C30 alkyl, e.g., $-NHCH_2Ph$, $-N(CH_2Ph)_2$, $-NH(CH_2)_{15}Ph$, or the like. Amino substituted with hydroxy C1-C30 alkyl refers to amino with hydrogen atoms substituted with 1 or 2 identical or different hydroxy C1-C30 alkyl, e.g., $-NHCH_2CH_2OH$, $-N(CH_2CH_2OH)_2$, or $-NH(CH_2)_{16}OH$. Amino substituted with C3-C10 cycloalkyl refers to amino with hydrogen atoms substituted with 1 or 2 identical or different C3-C10 cycloalkyl, e.g., $-NHCH(CH_2)_2$, $-NHCH(CH_2)_4$, or the like.

Amino substituted with C3-C10 cycloalkyl C1-C6 alkyl refers to amino with hydrogen atoms substituted with 1 or 2 identical or different C3-C10 cycloalkyl C1-C6 alkyl, e.g., $-NHCH_2CH(CH_2)_2$, $-NHCH_2CH_2CH(CH_2)_2$, $-NHCH_2CH(CH_2)_4$, or the like.

C1-C30 alkoxycarbonyl C1-C30 alkyl refers to C1-C30 alkoxycarbonyl with carbonyl carbon atoms connected with C1-C30 alkyl, e.g., $-CH_2(CO)OCH_2CH_3$, $-CH_2CH_2(CO)OCH_2CH_3$, $-CH_2(CO)O(CH_2)_{15}CH_3$, or the like.

C3-C10 cycloalkyl refers to saturated cyclohydrocarbyl containing 3-10 carbon atoms, e.g., cyclopropyl, cyclobutyl, cyclopentyl, cyclohexyl, cycloheptyl, cyclooctyl, or the like.

C3-C10 cycloalkoxy refers to hydroxy with a hydrogen atom substituted with C3-C10 cycloalkyl, e.g., $-OCH(CH_2)_2$, $-OCH(CH_2)_3$, $-OCH(CH_2)_4$, or the like.

C1-C30 alkoxy C1-C30 alkyl refers to C1-C30 alkoxy and C1-C30 alkyl which are connected through a single bond, e.g., $-CH_2OCH_2CH_3$, $-CH_2CH_2OCH_2CH_3$, $-CH_2O(CH_2)_{15}CH_3$, or the like.

C1-C30 alkoxy C1-C30 alkoxy refers to C1-C30 alkoxy and C1-C30 alkoxy which are connected through a single bond, e.g., $-OCH_2OCH_2CH_3$, $-OCH_2CH_2OCH_2CH_3$, $-OCH_2O(CH_2)_{15}CH_3$, or the like.

Phenyl C1-C30 alkoxy C1-C30 alkyl refers to phenyl, C1-C30 alkoxy, and C1-C30 alkyl which are sequentially connected through a single bond, e.g., $-CH_2OCH_2CH_2Ph$, $-CH_2CH_2OCH_2CH_2Ph$, $-CH_2O(CH_2)_{16}Ph$, or the like.

C1-C30 alkoxy C1-C30 alkoxy C1-C30 alkyl refers to C1-C30 alkoxy, C1-C30 alkoxy, and C1-C30 alkyl which are sequentially connected through a single bond, e.g., $-CH_2OCH_2CH_2OCH_2CH_3$, $-CH_2CH_2OCH_2CH_2OCH_2CH_3$, $-CH_2OCH_2CH_2O(CH_2)_{15}CH_3$, or the like.

C1-C30 alkoxy C1-C30 alkoxy C1-C30 alkoxy C1-C30 alkyl refers to C1-C30 alkoxy, C1-C30 alkoxy, C1-C30 alkoxy, and C1-C30 alkyl which are sequentially connected through a single bond, e.g., $-CH_2OCH_2CH_2OCH_2CH_2OCH_2CH_3$, $-CH_2CH_2OCH_2CH_2OCH_2CH_2OCH_2CH_3$, $-CH_2OCH_2CH_2OCH_2CH_2O(CH_2)_{15}CH_3$, or the like.

Hydroxy C1-C30 alkyl refers to linear or branched alkyl containing 1-30 carbon atoms with one carbon atom connected with hydroxy, e.g., $-CH_2OH$, $-CH_2CH_2OH$, $-CH(OH)CH_3$, $-CH_2CH_2CH_2OH$, $-CH_2CH_2CH_2CH_2OH$, $-CH_2CH(CH_3)CH_2OH$, $-(CH_2)_{15}CH_2OH$, or the like.

Amino C1-C30 alkyl refers to linear or branched alkyl containing 1-30 carbon atoms with one carbon atom connected with amino, e.g., $-CH_2NH_2$, $-CH_2CH_2NH_2$, $-CH(NH_2)CH_3$, $-CH_2CH_2CH_2NH_2$, $-(CH_2)_{15}CH_2NH_2$, or the like.

C6-C12 aryl refers to a monocyclic or polycyclic aromatic ring group containing 6-12 ring atoms but no heteroatoms in the ring atoms, e.g., phenyl or naphthyl.

C6-C12 aryl C1-C30 alkyl refers to linear or branched saturated hydrocarbyl containing 1-30 carbon atoms with one carbon atom connected to a carbon atom of C6-C12 aryl, e.g., benzyl, naphthylmethyl, naphthylethyl, or the like.

Heterocyclyl includes "heterocycloalkyl" and "heteroaryl" and refers to a monocyclic or polycyclic group containing at least one heteroatom selected from nitrogen, oxygen, and sulfur as ring members, which may be aromatic or non-aromatic; 5- to 10-membered heterocyclyl refers to heterocyclyl containing 5-10 ring atoms, e.g., pyridinyl, piperidinyl, morpholinyl, furanyl, thienyl, thiazolyl, imidazolyl, pyrrolyl, pyrazinyl, pyridazinyl, pyrimidinyl, or the like.

The term "pharmaceutically acceptable salt" refers to salts of the compounds of the present invention which are, within the scope of sound medical judgment, suitable for use in contact with the tissues of humans and lower animals without undue toxicity, irritation, allergic response, and the like, commensurate with a reasonable benefit/risk ratio, and effective for their intended use where possible, e.g., pharmaceutically acceptable acid addition salts and pharmaceutically acceptable base addition salts.

The term "solvate" refers to a variable stoichiometric amount of a complex formed by a solute (e.g., the compound of the present invention) and a solvent (e.g., water, ethanol, or acetic acid). This physical association may involve varying degrees of ionic and covalent bonding, including hydrogen bonding. In certain cases, the solvate can be isolated, e.g.,

when one or more solvent molecules are incorporated into the crystal lattice of the crystalline solid. Generally, such solvents selected for the present invention do not interfere with the biological activity of the solute. Representative solvates include hydrates, ethanolates, methanolates, and the like. The term "hydrate" means that the solvent is a solvate of water.

The term "stereoisomer" or "optical isomer" refers to a stable isomer that has at least one chiral element, e.g., a chiral atom or a perpendicular asymmetric plane due to restricted rotation (e.g., certain biphenyls, allenes, and spiro compounds), and can enable the rotation of plane-polarized light. The compounds of the present invention (and derivative forms such as salts, solvates, or prodrugs thereof) contain asymmetric carbon atoms and thus may exist as individual stereoisomers, racemates, and mixtures of enantiomers and diastereomers.

The term "geometric isomer" or "cis-trans isomer" refers to a stable isomer resulting from a limited rotational degree of freedom in the double bond (e.g., *cis*-2-butene and *trans*-2-butene) or ring structure (e.g., cis-1,3-dichlorocyclobutane and *trαns*-1,3-dichlorocyclobutane). The specific configuration of the isomers is indicated using the cis/trans convention or using the E or Z system, where the term "E" or "E-form" means that substituents of the higher order are on opposite sides of the double bond, and the term "Z" or "Z-form" means that substituents of the higher order are on the same side of the double bond. The determination of the E- and Z-isomers can be performed by analytical methods such as X-ray crystallography, $^{1}$H-NMR, and $^{13}$C-NMR.

The term "isotopically labeled compound" refers to a derivative compound formed by replacing a specific atom in the compounds of the present invention with its isotopic atom. Unless otherwise indicated, the compounds of the present invention include various isotopes of H, C, N, O, F, P, S, and Cl, such as $^{2}$H (D), $^{3}$H (T), $^{13}$C, $^{14}$C, $^{15}$N, $^{17}$O, $^{18}$O, $^{18}$F, $^{31}$P, $^{32}$P, $^{35}$S, $^{36}$S, and $^{37}$Cl.

The term "prodrug" or "prodrug derivative" refers to a covalently bonded derivative or carrier of a parent compound or active drug substance that undergoes at least some biotransformation before exhibiting one or more of its pharmacological effects. Generally, such prodrugs have metabolically cleavable groups and are rapidly converted *in vivo* to produce the parent compound. Prodrugs are typically formulated with the goal of improving chemical stability, improving the acceptance and compliance of an individual, improving bioavailability, prolonging duration of action, improving organ selectivity, improving formulation (e.g., increased water solubility), and/or reducing side effects (e.g., toxicity). Generally, prodrugs are themselves weakly or not biologically active and are stable under normal conditions, and may be readily prepared from the parent compound by methods known in the art.

The compounds of formula (II), formula (III), formula (IV), formula (V), and formula (I-a) of the present invention are commercially available compounds or compounds prepared according to methods known in the art or prepared according to synthetic methods of similar compounds.

Starting compounds used in the reaction schemes of the present invention may be pharmaceutically acceptable salts thereof, including alkali metal salts and alkaline earth metal salts, such as sodium salts, potassium salts, calcium salts, and magnesium salts; organic base salts such as pyridine salts and triethylamine salts; inorganic acid salts such as hydrochloride, hydrobromide, hydroiodide, sulfate, nitrate, and phosphate; organic acid salts such as formate, acetate, propionate, glycolate, oxalate, malonate, succinate, fumarate, maleate, lactate, malate, citrate, tartrate, picrate, glutamate, methanesulfonate, and benzenesulfonate.

Further, the starting compounds used in the reaction schemes of the present invention may be in the form of solvates such as hydrates and alcoholates thereof.

The N-substituted quinolinone compound represented by general formula (I) and the stereoisomers thereof of the present invention may also be in the form of solvates such as hydrates and alcoholates thereof, and such solvates are included in the scope of the present invention.

The pharmaceutically acceptable salts of the N-substituted quinolinone compound represented by formula (I) and the stereoisomers thereof of the present invention means that the N-substituted quinolinone compound represented by formula (I) or the stereoisomers thereof are treated with suitable acids, which convert them into non-toxic adduct salt forms with therapeutic activity. The salts include hydrochloride, hydrobromide, hydroiodide, sulphate, bisulphate, nitrate, phosphate, acid phosphate, perchlorate, formate, acetate, trifluoroacetate, propionate, pyruvate, glycolate, oxalate, malonate, succinate, glutarate, maleate, fumarate, lactate, malate, citrate, tartrate, picrate, glutamate, benzoate, methanesulfonate, ethanesulfonate, benzenesulfonate, p-toluenesulfonate, salicylate, ascorbate, camphorate, camphorsulfonate, or the like. Conversely, the salt form may also be converted to the free base form by treatment with a base.

The term "pharmaceutically acceptable salt" as used above may also be a solvate thereof, and the solvate is included in the scope of the present invention. Examples of the solvate include hydrates and alcoholates. The term "room temperature" as used herein refers to 10-30 °C.

Abbreviations and definitions in the present invention are shown in the following table:

| Abbreviation | Definition |
|---|---|
| DMF | *N,N*-Dimethylfonnamide |
| DCM | Dichloromethane |

(continued)

| Abbreviation | Definition |
|---|---|
| TLC | Thin layer chromatography |
| PE | Petroleum ether |
| EA | Ethyl acetate |
| DMSO | Dimethyl sulfoxide |

The following examples and experimental examples further illustrate the present invention, but do not limit the scope of the present invention. The starting materials, reagents, methods, and the like used in examples and experimental examples were those conventional in the art, and all of them were commercially available, unless otherwise specified.

## Preparation Examples

### Example 1 Preparation of 7-(2-(4-(6-fluorobenzo[*b*]thiophen-4-yl)piperazin-1-yl)ethyl)-1-(hydroxymethyl)-3,4-dihydroquinolin-2 (1*H*)-one

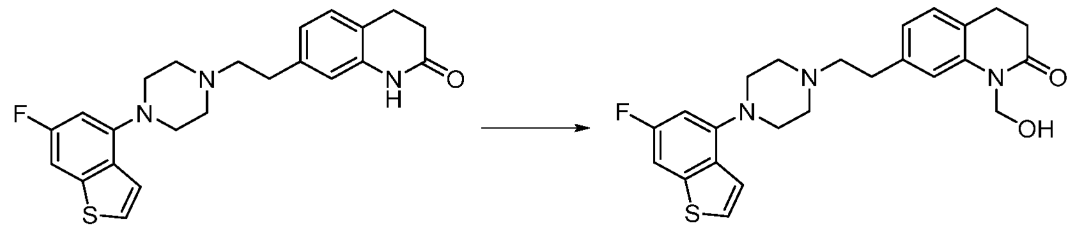

1-a 1

To a 100 mL single-necked flask were added compound 1-a (4.8 g, 1 eq), 37% aqueous formaldehyde solution (15 mL), triethylamine (593 mg, 0.5 eq), and DMF (15 mL), and the mixture was reacted at 85 °C for 3 h. The reaction process was monitored by TLC. Post-treatment: The reaction solution was diluted with 100 mL of DCM, and the dilution was washed with water (100 mL × 4) and concentrated under reduced pressure to obtain a crude product. The crude product was slurried with acetonitrile (10 mL) at room temperature, and the slurry was filtered to obtain 3.6 g of wet product. The wet product was slurried with DCM (6 mL) at 0-10 °C, and the slurry was filtered to obtain 2.3 g of wet product. The wet product was further slurried at 0-10 °C with DCM (4.6 mL), and the slurry was filtered and dried to obtain 2 g of the target compound (white solid, HPLC purity: 98%).

$^1$HNMR (400MHz, DMSO-$d_6$): δ ppm 7.66(d, J=5.5 Hz, 1H), 7.49(dd, J=8.6, 1.7 Hz, 1H), 7.37(d, J=5.5 Hz, 1H), 7.19(s, 1H), 7.11(d, J=7.6 Hz, 1H), 6.90(d, J=7.4 Hz, 1H), 6.75(dd, J=11.5, 2.0 Hz, 1H), 6.15(t, J=6.8 Hz, 1H), 5.23(d, J=6.8 Hz, 2H), 3.10(br, 4H), 2.78(m, 4H), 2.69(br, 4H), 2.62(m, 2H), 2.51(m, 2H). ESI-MS (*m*/*z*): 440.28 $[M+H]^+$.

### Example 2 Preparation of (7-(2-(4-(6-fluorobenzo[*b*]thiophen-4-yl)piperazin-1-yl)ethyl)-2-oxo-3,4-dihydroquinolin-1(2*H*)-yl)meth yl isopropyl carbonate

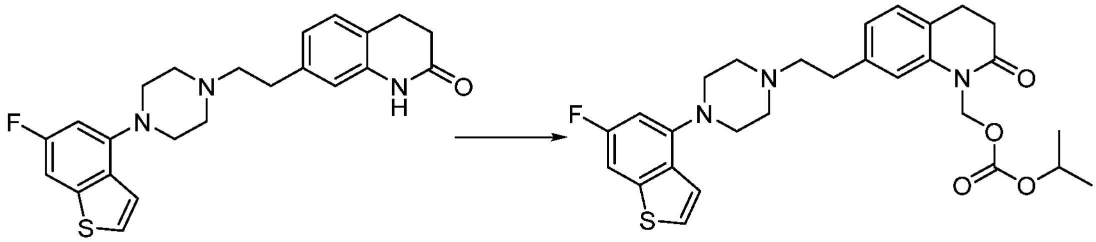

1-a 2

Compound 1-a (400 mg, 1 eq) was dissolved in DMF (10 mL), sodium hydride (4 eq) was added with the temperature controlled to be about 20 °C under nitrogen atmosphere, and the mixture was reacted at room temperature for 30 min. Chloromethyl isopropyl carbonate (4 eq) was added dropwise, and the mixture was heated to 60-65 °C and reacted for 2 h. After a small amount of the starting material remained as monitored by TLC (thin layer chromatography), the reaction was stopped. The reaction solution was poured into ethyl acetate and ice water (30 mL each), and the resulting mixture was stirred, left to stand, and subjected to liquid separation. The organic phase was washed sequentially with water and saturated brine (30 mL each), dried over anhydrous sodium sulfate, concentrated under reduced pressure, and purified by column chromatography to give 440 mg of an oil.

$^1$HNMR (400MHz, $CDCl_3$): δ ppm 7.33(m, 2H), 7.21(dd, J=8.2, 1.9 Hz, 1H), 7.11(d, J=7.6 Hz, 1H), 6.98(s, 1H), 6.94(d, J=7.6 Hz, 1H), 6.66(dd, J=11.1, 2.1 Hz, 1H), 5.96(s, 2H), 4.94(m, 1H), 3.20(br, 4H), 2.89(m, 2H), 2.85(m, 2H), 2.77(br, 4H), 2.70(m, 4H), 1.31(d, J=6.3 Hz, 6H).

ESI-MS (*m*/*z*): 526.50 $[M+H]^+$.

**Example 3 Preparation of (7-(2-(4-(6-fluorobenzo[*b*]thiophen-4-yl)piperazin-1-yl)ethyl)-2-oxo-3,4-dihydroquinolin-1(2*H*)-yl)meth yl dodecanoate**

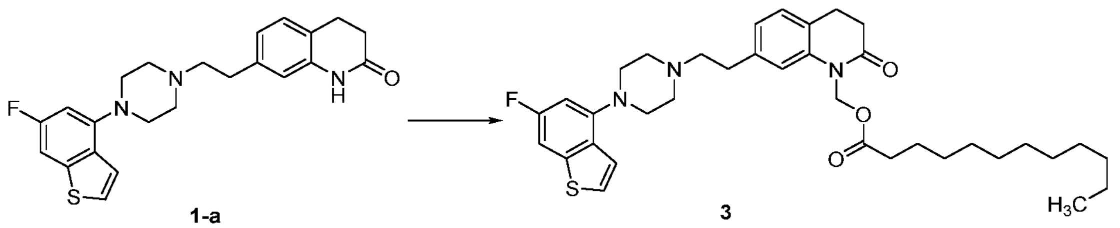

1-a 3

Compound 1-a (500mg, 1 eq) was dissolved in DMF (10 mL), sodium hydride (4 eq) was added with the temperature controlled to be about 20 °C under nitrogen atmosphere, and the mixture was reacted at room temperature for 30 min. Chloromethyl laurate (4 eq) was added dropwise, and the mixture was heated to 60-65 °C and reacted for 2 h. After a small amount of the starting material remained as monitored by TLC (thin layer chromatography), the reaction was stopped. The reaction solution was poured into ethyl acetate and ice water (30 mL each), and the resulting mixture was stirred, left to stand, and subjected to liquid separation. The organic phase was washed sequentially with water and saturated brine (30 mL each), dried over anhydrous sodium sulfate, concentrated, and purified by column chromatography to give 670 mg of an oil. Isopropyl ether (6.7 mL) was added to obtain a clear solution, which was frozen and crystallized (-40 °C), filtered, and dried to give 500 mg of a white solid.

$^1$HNMR (400MHz, $CDCl_3$): δ ppm 7.33(m, 2H), 7.21(dd, J=8.2, 1.9 Hz, 1H), 7.11(d, J=7.6 Hz, 1H), 6.94(d, J=7.6 Hz, 1H), 6.90(s, 1H), 6.66(dd, J=11.1, 2.1 Hz, 1H), 5.94(s, 2H), 3.20(br, 4H), 2.90(m, 2H), 2.85(m, 2H), 2.77(br, 4H), 2.70(m, 4H), 2.36(t, J=7.6 Hz, 2H), 1.64(m, 2H), 1.35-1.17(m, 16H), 0.86(t, J=6.9 Hz, 3H).

ESI-MS (*m*/*z*): 622.52 $[M+H]^+$.

**Example 4 Preparation of isopropyl 7-(2-(4-(6-fluorobenzo[*b*]thiophen-4-yl)piperazin-1-yl)ethyl)-2-oxo-3,4-dihydroquinoline-1(2*H*)-carbox ylate**

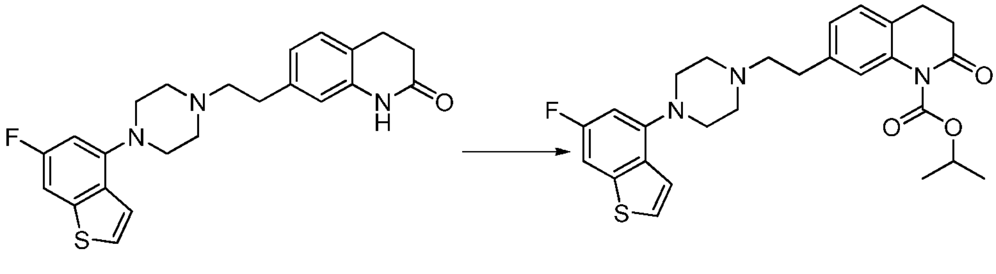

1-a 4

Compound 1-a (400 mg, 1 eq) was dissolved in DMF (10 mL), sodium hydride (4 eq) was added with the temperature controlled to be about 20 °C under nitrogen atmosphere, and the mixture was reacted at room temperature for

30 min. Isopropyl chloroformate (4 eq) was added dropwise, and the mixture was heated to 60-65 °C and reacted for 2 h. After a small amount of the starting material remained as monitored by TLC (thin layer chromatography), the reaction was stopped. The reaction solution was poured into ethyl acetate and ice water (30 mL each), and the resulting mixture was stirred, left to stand, and subjected to liquid separation. The organic phase was washed sequentially with water and saturated brine (30 mL each), dried over anhydrous sodium sulfate, concentrated, and purified by column chromatography to give 440 mg of an oil.

$^1$HNMR (400MHz, $CDCl_3$): δ ppm 7.33(m, 2H), 7.21(dd, J=8.2, 2.0 Hz, 1H), 7.11(d, J=7.6 Hz, 1H), 6.95(dd, J=7.6, 1.1 Hz, 1H), 6.84(s, 1H), 6.66(dd, J=11.1, 2.1 Hz, 1H), 5.24(m, 1H), 3.20(br, 4H), 2.93(m, 2H), 2.82(m, 2H), 2.77(br, 4H), 2.67(m, 4H), 1.41(d, J=6.3 Hz, 6H).

ESI-MS (*m*/*z*): 496.25 $[M+H]^+$.

**Example 5 Preparation of (7-(2-(4-(6-fluorobenzo[*b*]thiophen-4-yl)piperazin-1-yl)ethyl)-2-oxo-3,4-dihydroquinolin-1(2*H*)-yl)meth yl acetate**

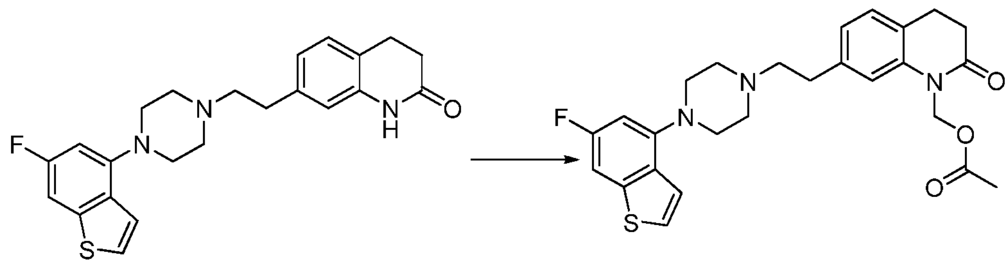

1-a 5

To a 25 mL reaction flask were added compound 1-a (200 mg, 1 eq) and DMF (4 mL). 60% sodium hydride (78 mg, 4 eq) was added with the temperature controlled to be < 30 °C under nitrogen atmosphere, and the mixture was stirred at room temperature for 30 min. Chloromethyl acetate (212 mg, 4 eq) and sodium iodide (73 mg, 1 eq) were sequentially added, and the mixture was reacted at room temperature for 2-3 h. The reaction was quenched with water (1 mL). The resulting mixture was diluted with ethyl acetate (8 mL), and the dilution was washed with water three times (8 mL × 3). The organic phase was concentrated and purified by column chromatography to obtain an oil. Isopropyl ether was added, and the resulting mixture was stirred at room temperature for crystallization to obtain 115 mg of a white solid with a yield of 49%.

$^1$HNMR (400MHz, $CDCl_3$): δ ppm 7.33(m, 2H), 7.21(dd, J=8.2, 1.8 Hz, 1H), 7.12(m, 1H), 6.95(d, J=7.6 Hz, 1H), 6.91(s, 1H), 6.66(dd, J=11.1, 2.1 Hz, 1H), 5.93(s, 2H), 3.21(br, 4H), 2.93-2.82(m, 4H), 2.78(br, 4H), 2.70(m, 4H), 2.12(s, 3H).

ESI-MS (*m*/*z*): 482.29 $[M+H]^+$.

**Example 6 Preparation** of **(7-(2-(4-(6-fluorobenzo[*b*]thiophen-4-yl)piperazin-1-yl)ethyl)-2-oxo-3,4-dihydroquinolin-1(2*H*-yl)meth yl pentanoate**

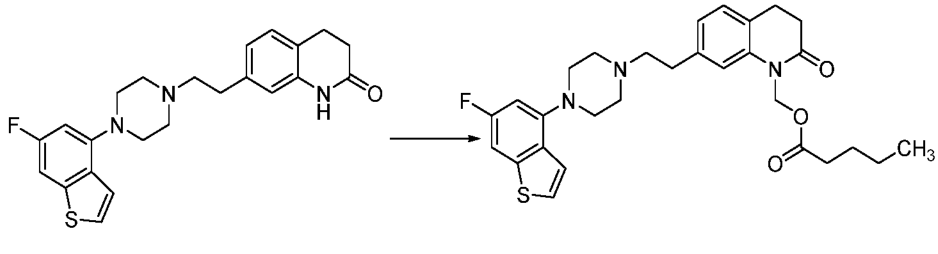

1-a 6

To a 25 mL reaction flask were added compound 1-a (200 mg, 1 eq) and DMF (4 mL). 60% sodium hydride (78 mg, 4 eq) was added with the temperature controlled to be < 30 °C under nitrogen atmosphere, and the mixture was stirred at room temperature for 30 min. Chloromethyl *n*-valerate (294mg, 4 eq) and sodium iodide (73 mg, 1 eq) were sequentially

added, and the mixture was reacted at room temperature for 2-3 h. The reaction was quenched with water (1 mL). The resulting mixture was diluted with ethyl acetate (8 mL), and the dilution was washed with water three times (8 mL × 3). The organic phase was concentrated and purified by column chromatography to obtain an oil. Isopropyl ether was added, and the resulting mixture was stirred at a low temperature for crystallization to obtain 100 mg of a white solid with a yield of 39%.

$^1$HNMR (400MHz, $CDCl_3$): δ ppm 7.33(m, 2H), 7.21(dd, J=8.2, 1.9 Hz, 1H), 7.12(m, 1H), 6.95(d, J=7.6 Hz, 1H), 6.89(s, 1H), 6.66(dd, J=11.1, 2.1 Hz, 1H), 5.94(s, 2H), 3.21(br, 4H), 2.94-2.81(m, 4H), 2.77(br, 4H), 2.69(m, 4H), 2.37(t, J=7.5 Hz, 2H), 1.63(m, 2H), 1.35(m, 2H), 0.90(t, J=7.3 Hz, 3H).

ESI-MS (*m/z*): 524.31 $[M+H]^+$.

**Example 7 Preparation of (7-(2-(4-(6-fluorobenzo[*b*]thiophen-4-yl)piperazin-1-yl)ethyl)-2-oxo-3,4-dihydroquinolin-1(2*H*-yl)meth yl dodecanoate**

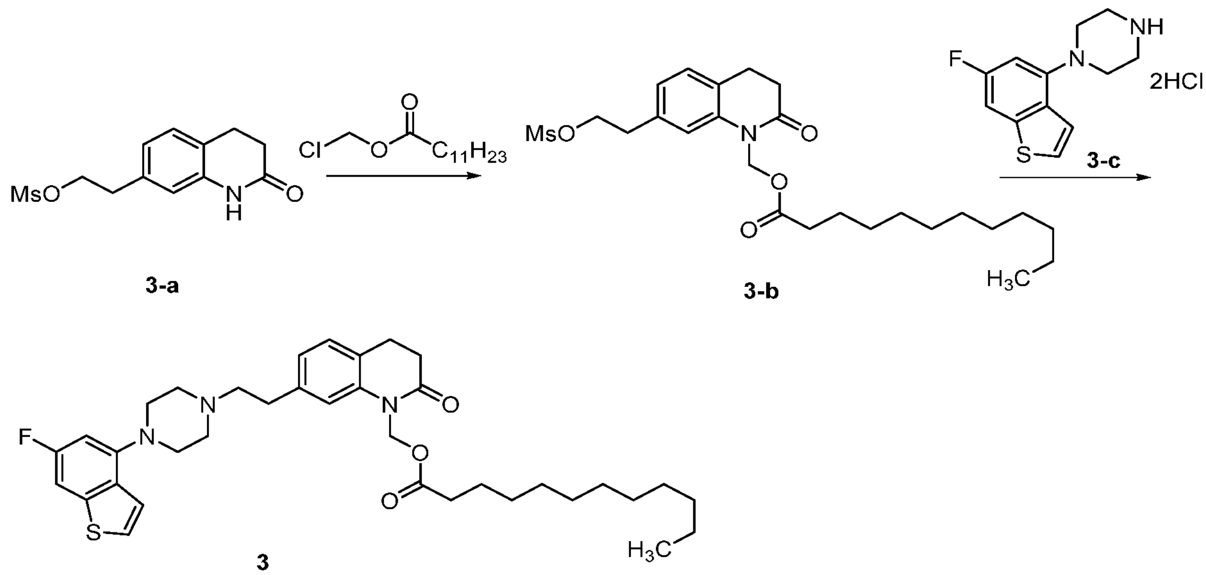

3-a
3-b
3

**Step 1:**

Reference was made to patent WO2015131856 for the preparation of compound 3-a. To a 25 mL reaction flask were added a starting material 3-a (250 mg, 1 eq) and DMF (5 mL, 20 V). 60% sodium hydride (56 mg, 1.5 eq) was added with the temperature controlled to be < 30 °C under nitrogen atmosphere, and the mixture was stirred at room temperature for 30 min. Chloromethyl dodecanoate (347 mg, 1.5 eq) and sodium iodide (140 mg, 1 eq) were sequentially added, and the mixture was reacted at room temperature for 2-3 h. The reaction was quenched with water (1 mL) to stop the reaction. The resulting mixture was diluted with ethyl acetate (10 mL) and washed three times with water (10 mL × 3). The organic phase was concentrated and purified by column chromatography (PE/EA = 30/1-20/1) to give 300 mg of an oil (intermediate 3-b) with a yield of 67%.

Step 2:

To a 25 mL reaction flask were added 3-c (161 mg, 1 eq), 3-b (300 mg, 1.2 eq), sodium bicarbonate (218 mg, 5 eq), sodium iodide (78 mg, 1 eq), and DMF (3.2 mL, 20 V), and the mixture was reacted at 85 °C for 2 h under nitrogen atmosphere. The reaction solution was diluted with dichloromethane (6.4 mL), and the dilution was washed with water (6.4 mL × 4), concentrated, and purified by column chromatography (PE/EA = 5/1-2/1) to give the title compound (white solid, 200 mg, yield: 62%).

**Example 8 Preparation of (7-(2-(4-(6-fluorobenzo[*b*]thiophen-4-yl)piperazin-1-yl)ethyl)-2-oxo-3,4-dihydroquinolin-1(2*H*-yl)meth yl decanoate**

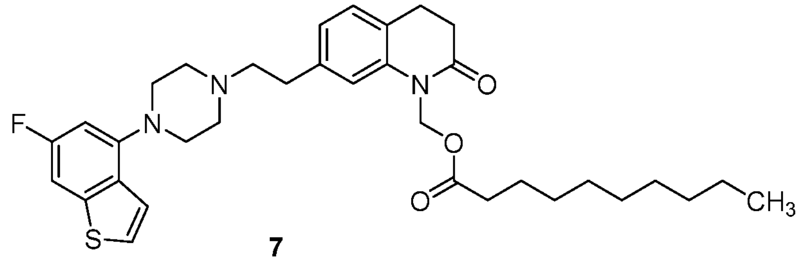

7

Referring to the above examples, the title compound (yield: 52%, white solid) was prepared with compound 1-a and chloromethyl decacarboxylate as starting materials.

$^1$HNMR (400MHz, $CDCl_3$): δ ppm 7.33(m, 2H), 7.21(dd, J=8.2, 1.8 Hz, 1H), 7.11(d, J=7.6 Hz,1H), 6.94(d, J=7.6 Hz, 1H), 6.90(s, 1H), 6.66(dd, J=11.1, 2.0 Hz, 1H), 5.94(s, 2H), 3.21(br, 4H), 2.91(m, 2H), 2.85(m, 2H), 2.77(br, 4H), 2.69(m, 4H), 2.35(t, J=7.6 Hz, 2H), 1.64(m, 2H), 1.25(br, 12H), 0.85(t, J=6.8 Hz, 3H). ESI-MS (*m*/*z*): 594.43 [M+H]$^+$.

### Example 9 Preparation of (7-(2-(4-(6-fluorobenzo[*b*]thiophen-4-yl)piperazin-1-yl)ethyl)-2-oxo-3,4-dihydroquinolin-1(2*H*)-yl)meth yl palmitate

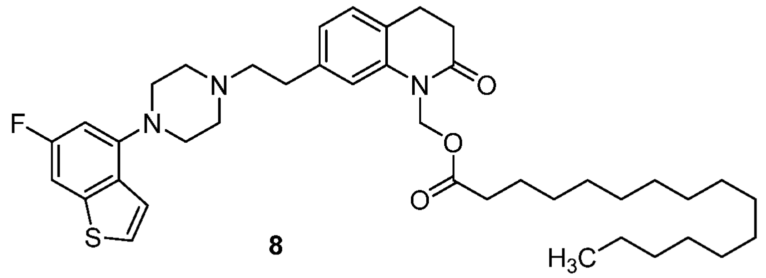

8

Referring to the above examples, the title compound (yield: 38%, white solid) was prepared with compound 1-a and chloromethyl hexadecanoate as starting materials.

$^1$HNMR (400MHz, $CDCl_3$): δ ppm 7.33(m, 2H), 7.21(dd, J=8.2, 1.9 Hz, 1H), 7.12(m, 1H), 6.95(d, J=7.6 Hz, 1H), 6.89(s, 1H), 6.66(dd, J=11.1, 2.1 Hz, 1H), 5.94(s, 2H), 3.21(br, 4H), 2.90(m, 2H), 2.85(m, 2H), 2.77(br, 4H), 2.69(m, 4H), 2.36(t, J=7.5 Hz, 2H), 1.63(m, 2H), 1.25(br, 24H), 0.87(t, J=7.3 Hz, 3H).

ESI-MS (*m*/*z*): 678.51 [M+H]$^+$.

### Example 10 Preparation of (7-(2-(4-(6-fluorobenzo[*b*]thiophen-4-yl)piperazin-1-yl)ethyl)-2-oxo-3,4-dihydroquinolin-1(2*H*)-yl)meth yl docosanoate

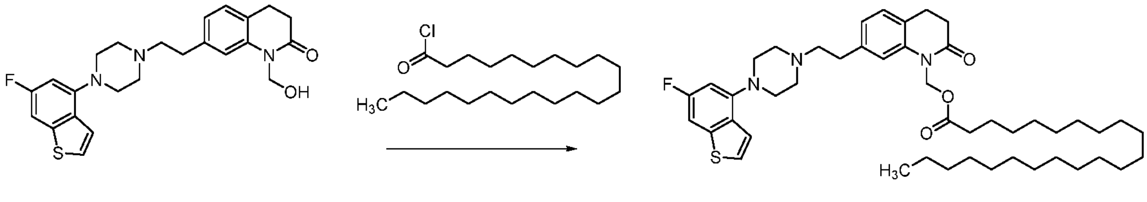

1 74

Thionyl chloride (10 mL) was added to 1.0 g of docosanoic acid (1.5 eq), a small amount of DMF was added for catalysis, and the mixture was refluxed for 3 h. After the reaction was completed as detected by TLC, the reaction solution was concentrated under reduced pressure to obtain docosanoyl chloride.

To another reaction flask was added compound 1 (220 mg, 1.0 eq), dichloromethane (5 mL), and triethylamine (150 mg, 3 eq). The newly prepared docosanoyl chloride was added to the system, and the mixture was stirred for reaction for 15 min. The reaction was quenched with water. The resulting mixture was subjected to liquid separation. The organic phase was retained, concentrated under reduced pressure, and purified by column chromatography to obtain 127 mg of

**compound 74** as an off-white solid.

$^1$HNMR (400MHz, $CDCl_3$): δ ppm 7.67(m, 1H), 7.50(m,1H), 7.36(m, 1H), 7.16(d, J=8.0 Hz, 1H), 7.10-6.97(m, 2H), 6.75(m, 1H), 5.86(s, 2H), 3.10(m, 4H), 2.86-2.60(m, 12H), 2.32(t, J=8.0 Hz, 2H), 1.51(m, 2H), 1.22-1.17(m, 36H), 0.84(t, t, J=8.0 Hz, 3H).

ESI-MS (*m*/*z*): 763.41 $[M+H]^+$.

**Example 11 Preparation of (7-(2-(4-(6-fluorobenzo[*b*]thiophen-4-yl)piperazin-1-yl)ethyl)-2-oxo-3,4-dihydroquinolin-1(2*H*-yl)meth yl heptacosanoate**

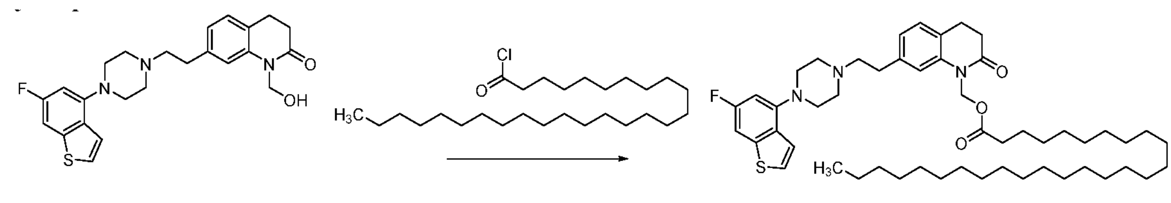

1 76

Thionyl chloride (3 mL) was added to 0.3 g of heptacosanoic acid (1.5 eq), a small amount of DMF was added for catalysis, and the mixture was refluxed for 2 h. After the reaction was completed as detected by TLC, the reaction solution was concentrated under reduced pressure to obtain heptacosanoyl chloride.

To another reaction flask was added compound 1 (150 mg, 1.0 eq), dichloromethane (3 mL), and triethylamine (100mg, 3 eq). The newly prepared heptacosanoyl chloride was added to the system, and the mixture was stirred for reaction for 15 min. The reaction was quenched with water. The resulting mixture was subjected to liquid separation. The organic phase was retained, concentrated under reduced pressure, and purified by column chromatography to obtain 167 mg of **compound 76** as an off-white solid.

$^1$HNMR (400MHz, $CDCl_3$): δ ppm 7.35-7.32(m, 2H), 7.24-7.21(m,1H), 7.12(d, J=8.0 Hz, 1H), 6.95(d, J=8.0 Hz, 1H), 6.90(s,1H), 6.69-6.65(m, 1H), 5.95(s, 2H), 3.20(m, 4H), 2.92-2.70(m, 12H), 2.36(t, J=16 Hz, 2H), 1.66-1.61(m, 2H), 1.28-1.22(m, 48H), 0.88(t, t, J=12.0 Hz, 3H).

ESI-MS (*m*/*z*): 833.21 $[M+H]^+$.

The compounds shown in the following table were prepared by the same methods as in the above examples, except that starting materials and intermediates corresponding to the final products were used.

| Compound No. | Name of the compounds | Structure | ESI-MS (*m*/*z*) |
|---|---|---|---|
| 9 | (7-(2-(4-(6-Fluorobenzo[*b*] thiophe n-4-yl)piperazin-1-yl)ethyl)-2-oxo quinolin-1(2*H*)-yl)methyl palmitate | 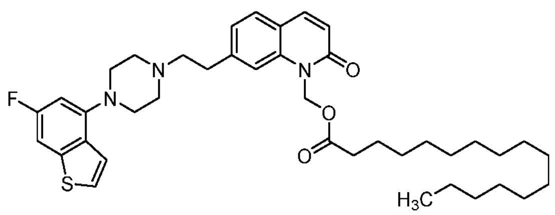 | 676.9 $[M+H]^+$ |
| 10 | (7-(2-(4-(6-Fluorobenzo[*b*] thiophe n-4-yl)piperazin-1-yl)ethyl)-2-oxo quinolin-1(2*H*)-yl)methyl decanoate | 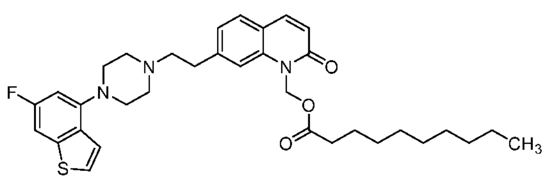 | 592.8 $[M+H]^+$ |
| 11 | 1-Acetyl-7-(2-(4-(6-fluorobenzo[*b*] thiophen-4-yl)piperazin-1-yl)ethyl) -3,4-dihydroquinolin-2(1*H*)-one | 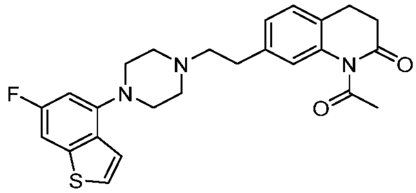 | 452.5 $[M+H]^+$ |

(continued)

| Compound No. | Name of the compounds | Structure | ESI-MS (*m/z*) |
|---|---|---|---|
| 12 | (7-(2-(4-(6-Fluorobenzo[*b*] thiophe n-4-yl)piperazin-1-yl)ethyl)-2-oxo-3,4-dihydroquinolin-1(2*H*)-yl)meth yl benzoate | 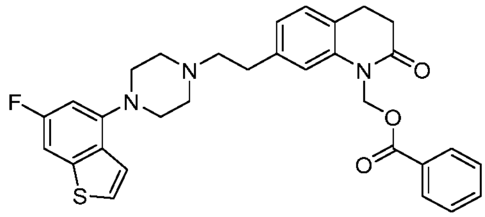 | 544.6 [M+H]+ |
| 13 | 1-(7-(2-(4-(6-Fluorobenzo [*b*]thiop hen-4-yl)piperazin-1-yl)ethyl)-2-ox o-3,4-dihydroquinolin-1(2*H*)-yl)eth yl dodecanoate | 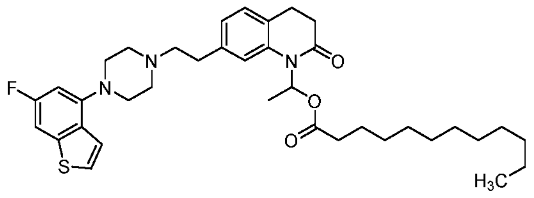 | 636.8 [M+H]+ |
| 14 | (7-(2-(4-(6-Fluorobenzo[*b*] thiophe n-4-yl)piperazin-1-yl)ethyl)-2-oxo-3,4-dihydroquinolin-1(2*H*)-yl)methyl undecyl carbonate | 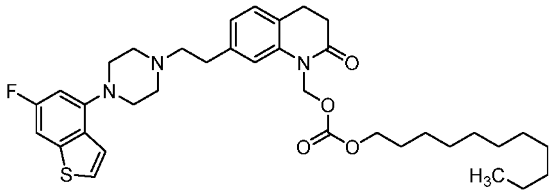 | 638.8 [M+H]+ |
| 15 | 1-(7-(2-(4-(6-Fluorobenzo [*b*]thiop hen-4-yl)piperazin-1-yl)ethyl)-2-ox o-3,4-dihydroquinolin-1(2*H*)-yl)eth yl palmitate | 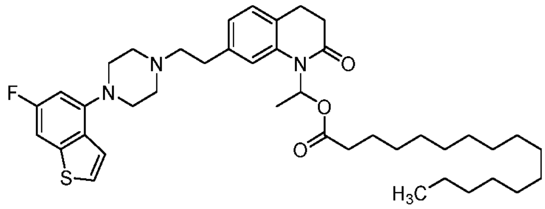 | 692.9 [M+H]+ |
| 16 | 1-(7-(2-(4-(6-Fluorobenzo [*b*]thiop hen-4-yl)piperazin-1-yl)ethyl)-2-ox o-3,4-dihydroquinolin-1(2*H*)-yl)eth yl isobutyrate | 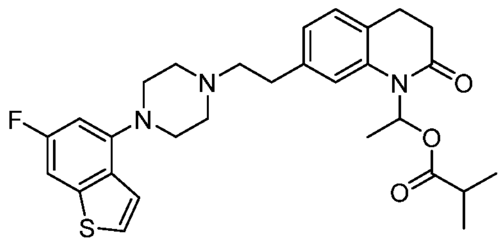 | 524.6 [M+H]+ |
| 17 | (7-(2-(4-(6-Fluorobenzo[b] thiophe n-4-yl)piperazin-1-yl)ethyl)-2-oxo-3,4-dihydroquinolin-1(2*H*)-yl)meth yl pivalate | 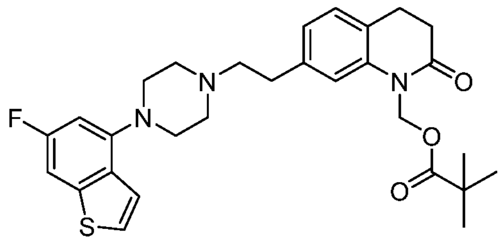 | 524.6 [M+H]+ |
| 18 | (7-(2-(4-(6-Fluorobenzo[b] thiophe n-4-yl)piperazin-1-yl)ethyl)-2-oxo-3,4-dihydroquinolin-1(2*H*)-yl)meth yl hexanoate | 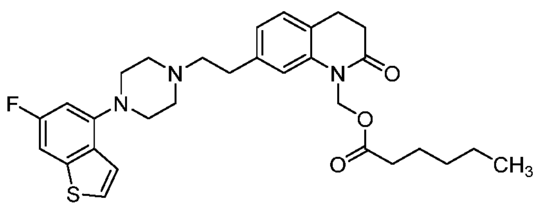 | 538.7 [M+H]+ |

(continued)

| Compound No. | Name of the compounds | Structure | ESI-MS (*m*/*z*) |
|---|---|---|---|
| 19 | (7-(2-(4-(6-Fluorobenzo[*b*] thiophe n-4-yl)piperazin-1-yl)ethyl)-2-oxo-3,4-dihydroquinolin-1(2*H*)-yl)meth yl methyl carbonate | 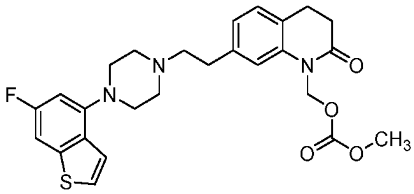 | 498.6 $[M+H]^+$ |
| 20 | (7-(2-(4-(6-Fluorobenzo[b] thiophe n-4-yl)piperazin-1-yl)ethyl)-2-oxo quinolin-1(2*H*)-yl)methyl methyl carbonate | 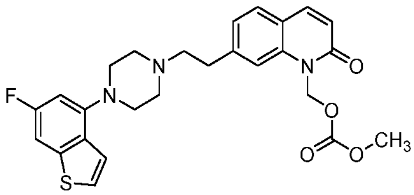 | 496.6 $[M+H]^+$ |
| 21 | Ethyl ((7-(2-(4-(6-fluorobenzo[*b*]thiophe n-4-yl)piperazin-1-yl)ethyl)-2-oxo-3,4-dihydroquinolin-1(2*H*)-yl)meth yl) carbonate | 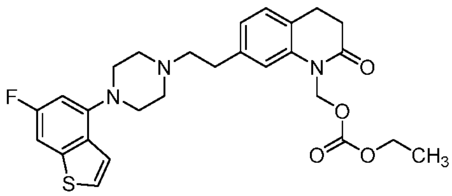 | 512.6 $[M+H]^+$ |
| 22 | (7-(2-(4-(6-Fluorobenzo[*b*] thiophe n-4-yl)piperazin-1-yl)ethyl)-2-oxo-3,4-dihydroquinolin-1(2*H*)-yl)meth yl propyl carbonate | 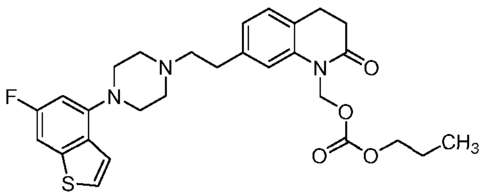 | 526.6 $[M+H]^+$ |
| 23 | Butyl ((7-(2-(4-(6-fluorobenzo[*b*]thiophe n-4-yl)piperazin-1-yl)ethyl)-2-oxo-3,4-dihydroquinolin-1(2*H*)-yl)meth vl) carbonate | 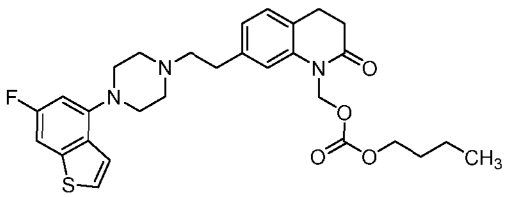 | 540.6 $[M+H]^+$ |
| 24 | (7-(2-(4-(6-Fluorobenzo[*b*] thiophe n-4-yl)piperazin-1-yl)ethyl)-2-oxo-3,4-dihydroquinolin-1(2*H*)-yl)meth yl hexyl carbonate | 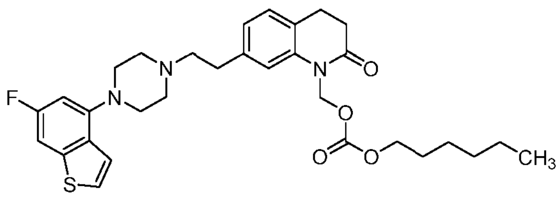 | 568.7 $[M+H]^+$ |
| 25 | (7-(2-(4-(6-Fluorobenzo[*b*] thiophe n-4-yl)piperazin-1-yl)ethyl)-2-oxo-3,4-dihydroquinolin-1(2*H*)-yl)meth yl heptyl carbonate | 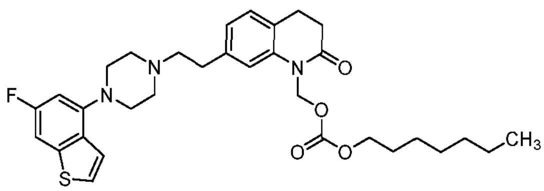 | 582.7 $[M+H]^+$ |
| 26 | (7-(2-(4-(6-Fluorobenzo[*b*] thiophe n-4-yl)piperazin-1-yl)ethyl)-2-oxo-3,4-dihydroquinolin-1(2*H*)-yl)meth vl octvl carbonate | 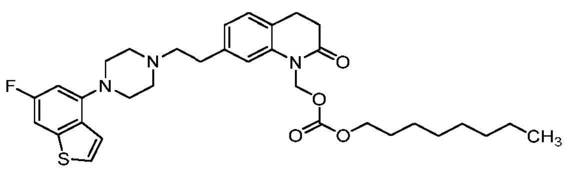 | 596.7 $[M+H]^+$ |

(continued)

| Compound No. | Name of the compounds | Structure | ESI-MS (*m*/*z*) |
|---|---|---|---|
| 27 | (7-(2-(4-(6-Fluorobenzo[*b*] thiophe n-4-yl)piperazin-1-yl)ethyl)-2-oxo-3,4-dihydroquinolin-1(2*H*)-yl)meth yl nonyl carbonate | 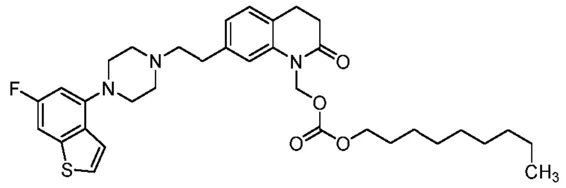 | 610.8 $[M+H]^+$ |
| 28 | Decyl ((7-(2-(4-(6-fluorobenzo[*b*]thiophe n-4-yl)piperazin-1-yl)ethyl)-2-oxo-3,4-dihydroquinolin-1(2*H*)-yl)meth yl)carbo-nate | 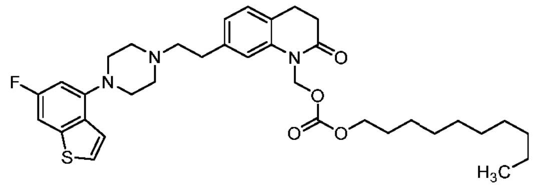 | 624.8 $[M+H]^+$ |
| 29 | Dodecyl ((7-(2-(4-(6-fluorobenzo[*b*]thiophe n-4-yl)piperazin-1-yl)ethyl)-2-oxo-3,4-dihydroquinolin-1(2*H*)-yl)meth yl) carbo-nate | 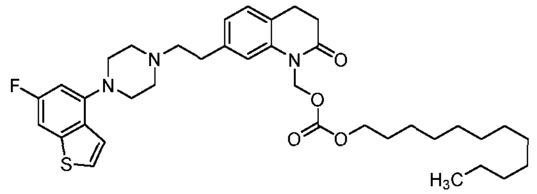 | 652.8 $[M+H]^+$ |
| 30 | (7-(2-(4-(6-Fluorobenzo[*b*] thiophe n-4-yl)piperazin-1-yl)ethyl)-2-oxo-3,4-dihydroquinolin-1(2*H*)-yl)meth yl tetradecvl carbonate | 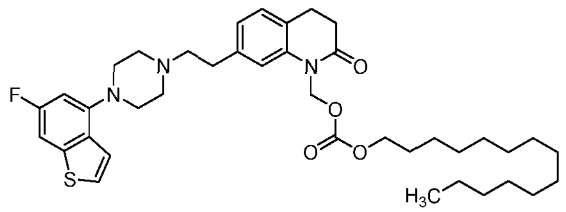 | 680.9 $[M+H]^+$ |
| 31 | (7-(2-(4-(6-Fluorobenzo[b] thiophe n-4-yl)piperazin-1-yl)ethyl)-2-oxo-3,4-dihydroquinolin-1(2*H*)-yl)meth yl hexadecyl carbonate | 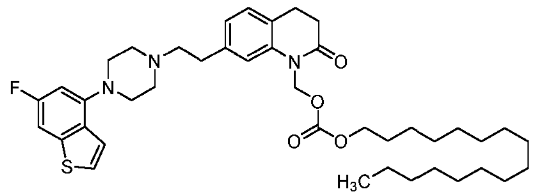 | 709.0 $[M+H]^+$ |
| 32 | (7-(2-(4-(6-Fluorobenzo[b] thiophe n-4-yl)piperazin-1-yl)ethyl)-2-oxo-3,4-dihydroquinolin-1(2*H*)-yl)meth yl phenyl carbonate | 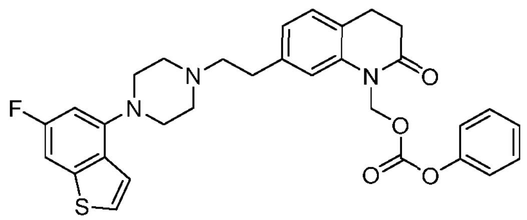 | 560.7 $[M+H]^+$ |
| 33 | (7-(2-(4-(6-Fluorobenzo[*b*] thiophe n-4-yl)piperazin-1-yl)ethyl)-2-oxo quinolin-1(2*H*)-yl)methyl phenyl carbonate | 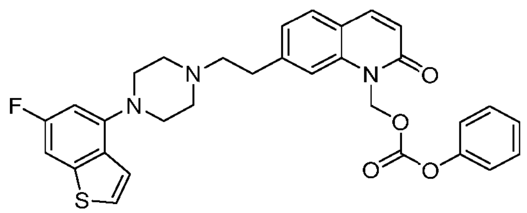 | 558.6 $[M+H]^+$ |

(continued)

| Compound No. | Name of the compounds | Structure | ESI-MS (*m*/*z*) |
|---|---|---|---|
| 34 | Benzyl ((7-(2-(4-(6-fluorobenzo[*b*]thiophe n-4-yl)piperazin-1-yl)ethyl)-2-oxo-3,4-dihydroquinolin-1(2*H*)-yl)meth yl) carbo-nate | 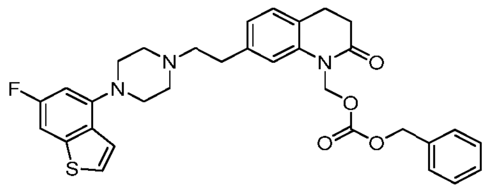 | 574.7 [M+H]+ |
| 35 | (7-(2-(4-(6-Fluorobenzo[*b*] thiophe n-4-yl)piperazin-1-yl)ethyl)-2-oxo-3,4-dihydroquinolin-1(2*H*)-yl)meth yl (2,2,2-trifluoroethyl) carbo-nate | 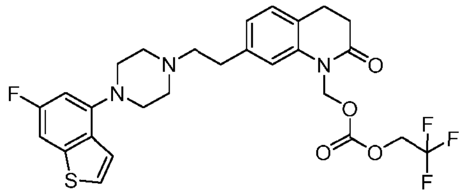 | 566.6 [M+H]+ |
| 36 | Cyclohexyl ((7-(2-(4-(6-fluorobenzo[*b*]thiophe n-4-yl)piperazin-1-yl)ethyl)-2-oxo-3,4-dihydroquinolin-1(2*H*)-yl)meth yl) carbo-nate | 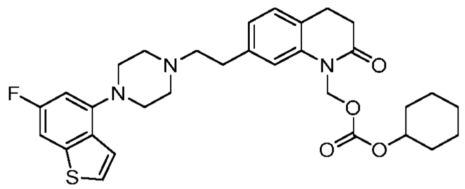 | 566.7 [M+H]+ |
| 37 | (4-Fluoro-7-(2-(4-(6-fluorobenzo[*b* ]thiophen-4-yl)piperazin-1-yl)ethyl )-2-oxoquinolin-1(2*H*)-yl)methyl hexanoate | 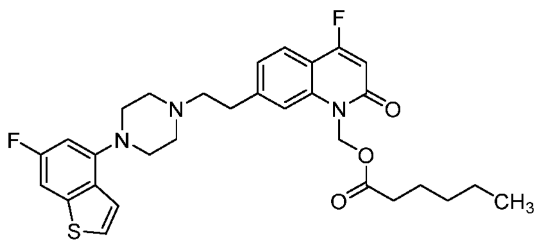 | 554.7 [M+H]+ |
| 38 | (4-Fluoro-7-(2-(4-(6-fluorobenzo[*b* ]thiophen-4-yl)piperazin-1-yl)ethyl )-2-oxoquinolin-1(2*H*)-yl)methyl do-decanoate | 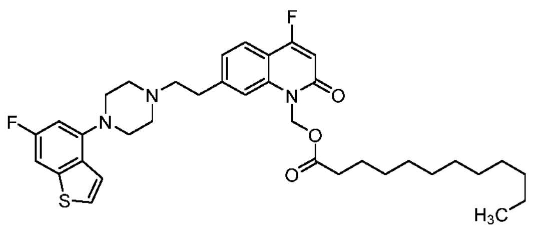 | 638.8 [M+H]+ |
| 39 | (4-Chloro-7-(2-(4-(6-fluorobenzo[ *b*]thiophen-4-yl)piperazin-1-yl)eth yl)-2-oxoquinolin-1(2*H*)-yl)methyl hexanoate | 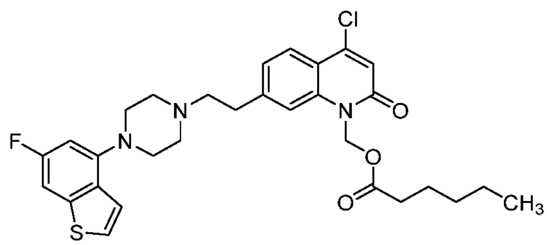 | 571.1 [M+H]+ |
| 40 | (4-Amino-7-(2-(4-(6-fluorobenzo[ *b*]thiophen-4-yl)piperazin-1-yl)eth yl)-2-oxoquinolin-1(2*H*)-yl)methyl hexanoate | 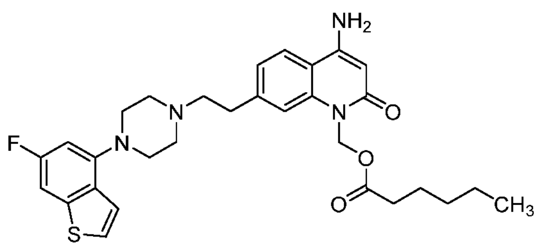 | 551.7 [M+H]+ |

(continued)

| Compound No. | Name of the compounds | Structure | ESI-MS (*m/z*) |
|---|---|---|---|
| 41 | (4-Acetamido-7-(2-(4-(6-fluoroben zo[*b*]thiophen-4-yl)piperazin-1-yl) ethyl)-2-oxoquinolin-1(2*H*)-yl)met hyl hexanoate | 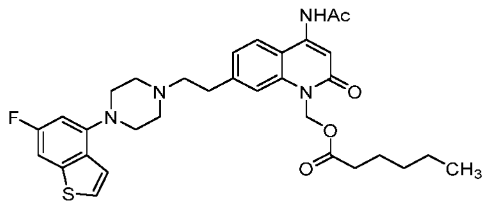 | 593.7 [M+H]+ |
| 42 | (7-(2-(4-(6-Fluorobenzo[*b*] thiophe n-4-yl)piperazin-1-yl)ethyl)-4,4-di methyl-2-oxo-3,4-dihydroquinolin-1(2*H*)-yl)methyl hexano-ate | 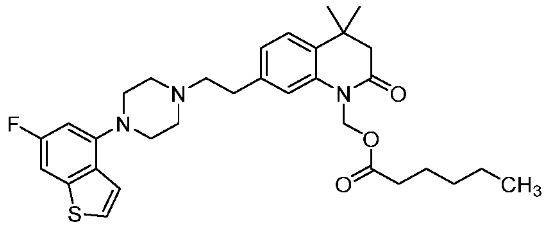 | 566.7 [M+H]+ |
| 43 | (7-(2-(4-(6-Fluorobenzo[*b*] thiophe n-4-yl)piperazin-1-yl)ethyl)-4,4-di methy l-2-oxo-3,4-dihydroquinolin-1(2*H*)-yl)methyl dode-canoate | 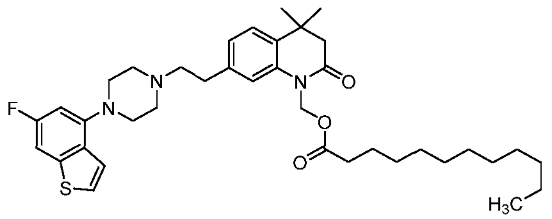 | 650.9 [M+H]+ |
| 44 | (7-(2-(4-(6-Fluorobenzo[*b*] thiophe n-4-yl)piperazin-1-yl)ethyl)-4-met hyl-2-oxoquinolin-1(2*H*)-yl)methyl hexanoate | 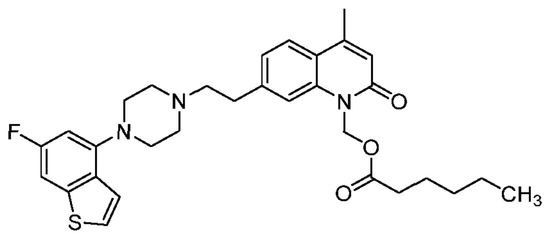 | 550.7 [M+H]+ |
| 45 | (7-(2-(4-(6-Fluorobenzo[b] thiophe n-4-yl)piperazin-1 -yl)ethyl)-3-met hyl-2-oxoquinolin-1(2*H*)-yl)methyl hexanoate | 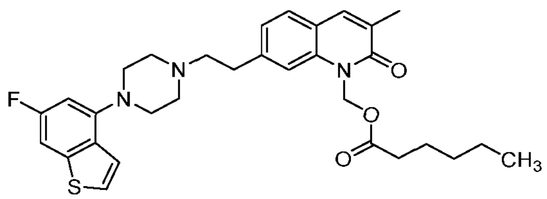 | 550.7 [M+H]+ |
| 46 | (3-Ethyl-7-(2-(4-(6-fluorobenzo[*b*] thiophen-4-yl)piperazin-1-yl)ethyl) -2-oxoquinolin-1(2*H*)-yl)methyl hexanoate | 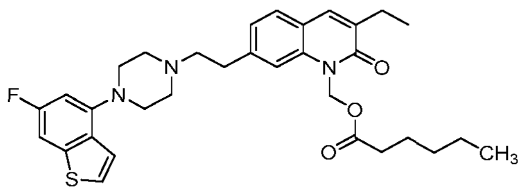 | 550.7 [M+H]+ |
| 47 | (7-(2-(4-(6-Fluorobenzo[b] thiophe n-4-yl)piperazin-1-yl)ethyl)-3-hydr oxy-2-oxoquinolin-1(2*H*)-yl)methy 1 hexanoate | 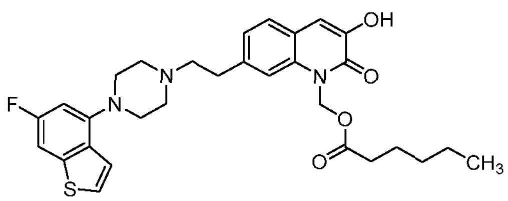 | 564.7 [M+H]+ |

(continued)

| Compound No. | Name of the compounds | Structure | ESI-MS (*m/z*) |
|---|---|---|---|
| 48 | (7-(2-(4-(6-Fluorobenzo[*b*] thiophe n-4-yl)piperazin-1-yl)ethyl)-4-hydr oxy-2-oxoquinolin-1(2*H*)-yl)methy l hexanoate | 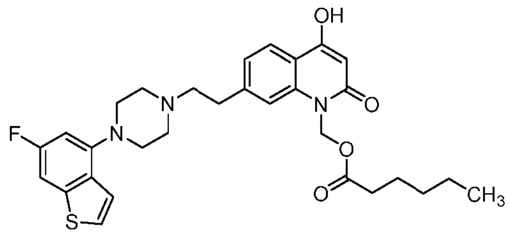 | 552.7 [M+H]$^+$ |
| 49 | (7-(2-(4-(6-Fluorobenzo[*b*] thiophe n-4-yl)piperazin-1-yl)ethyl)-4-hydr oxy-2-oxoquinolin-1(2*H*)-yl)methy 1 dodecanoate | 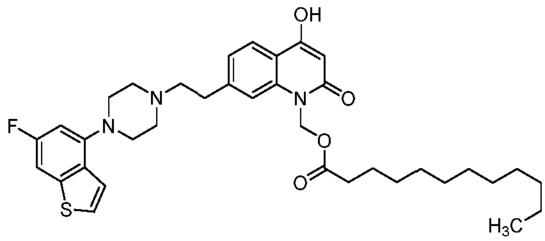 | 636.8 [M+H]$^+$ |
| 50 | (7-(2-(4-(6-Fluorobenzo[*b*] thiophe n-4-yl)piperazin-1-yl)ethyl)-2-oxo quinolin-1(2*H*)-yl-4-d)methylhex-anoate | 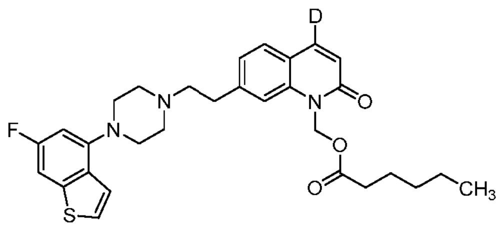 | 537.7 [M+H]$^+$ |
| 51 | (7-(2-(4-(6-Fluorobenzo[*b*] thiophe n-4-yl)piperazin-1-yl)ethyl)-2-oxo quinolin-1(2*H*)-yl-4-d)methyl do-decanoate | 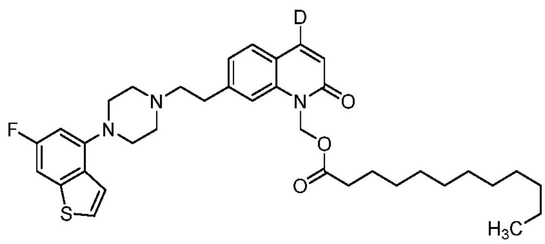 | 621.8[M+H]$^+$ |
| 52 | 7-(2-(4-(6-Fluorobenzo[*b*] thiophen -4-yl)piperazin-1-yl)ethyl)-1-(isopr opoxymethyl)-3,4-dihydroquinolin -2(1*H*)-one | 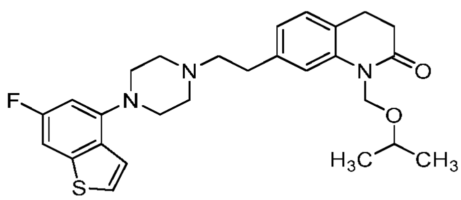 | 482.6[M+H]$^+$ |
| 53 | 7-(2-(4-(6-Fluorobenzo[*b*] thiophen -4-yl)piperazin-1-yl)ethyl)-1-(isopr opoxymethyl)quinolin-2(1*H*)-one | 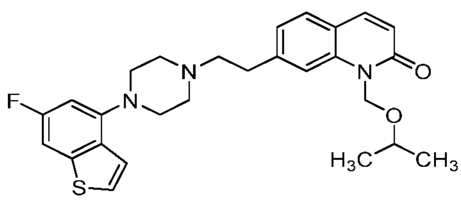 | 480.6[M+H]$^+$ |
| 54 | 7-(2-(4-(6-Fluorobenzo[*b*] thiophen -4-yl)piperazin-1-yl)ethyl)-1-((2,2, 2-trifluoroethoxy)methyl)-3,4-dihy droquinolin-2(1*H*)-one | 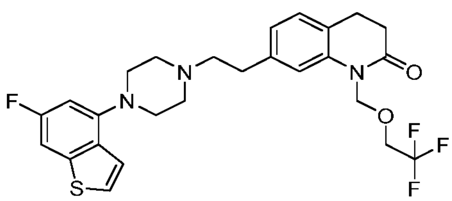 | 522.6[M+H]$^+$ |

(continued)

| Compound No. | Name of the compounds | Structure | ESI-MS (*m/z*) |
|---|---|---|---|
| 55 | 1-((Benzyloxy) methyl)-7-(2-(4-(6-fluorobenzo[b]thiophen-4-yl)piper azin-1-yl)ethyl)-3,4-dihydroquinoli n-2(1H)-one | 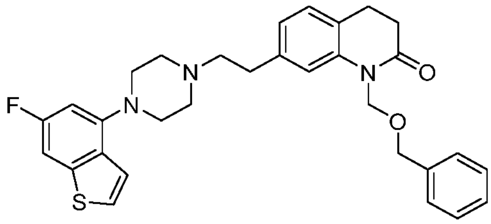 | 530.7[M+H]+ |
| 56 | 1-(((Benzyloxy)methoxy) methyl)-7-(2-(4-(6-fluorobenzo[*b*]thiophen-4-yl)piperazin-1-yl)ethyl)-3,4-dihy droquinolin-2(1*H*)-one | 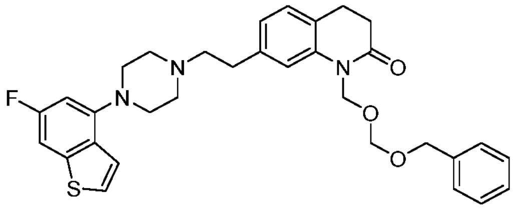 | 560.7[M+H]+ |
| 57 | (7-(2-(4-(6-Fluorobenzo[*b*] thiophe n-4-yl)piperazin-1-yl)ethyl)-2-oxo-3,4-dihydroquinolin-1(2*H*)-yl)meth vl propionate | 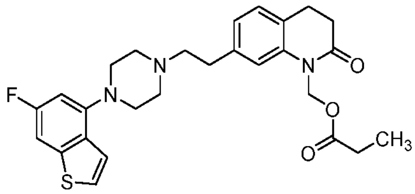 | 496.6[M+H]+ |
| 58 | (7-(2-(4-(6-Fluorobenzo[*b*] thiophe n-4-yl)piperazin-1-yl)ethyl)-2-oxo-3,4-dihydroquinolin-1(2*H*)-yl)methyl bu-tyrate | 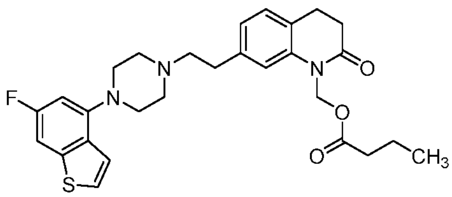 | 510.6[M+H]+ |
| 59 | (7-(2-(4-(6-Fluorobenzo[*b*] thiophe n-4-yl)piperazin-1-yl)ethyl)-2-oxo-3,4-dihydroquinolin-1(2*H*)-yl)meth yl 2-methoxyacetate | 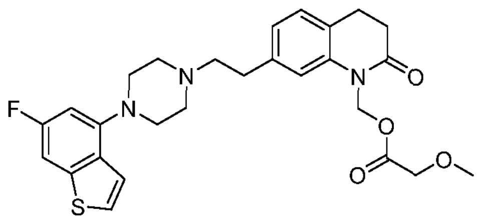 | 512.6[M+H]+ |
| 60 | (7-(2-(4-(6-Fluorobenzo[*b*] thiophe n-4-yl)piperazin-1-yl)ethyl)-2-oxo-3,4-dihydroquinolin-1(2*H*)-yl)meth yl heptanoate | 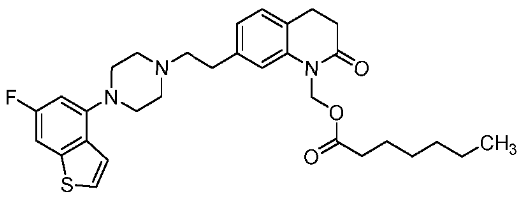 | 552.7[M+H]+ |
| 61 | (7-(2-(4-(6-Fluorobenzo[b] thiophe n-4-yl)piperazin-1-yl)ethyl)-2-oxo-3,4-dihydroquinolin-1(2*H*)-yl)meth yl octanoate | 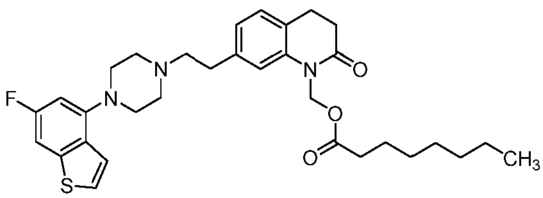 | 566.7[M+H]+ |

(continued)

| Compound No. | Name of the compounds | Structure | ESI-MS (*m/z*) |
|---|---|---|---|
| 62 | (7-(2-(4-(6-Fluorobenzo[*b*] thiophe n-4-yl)piperazin-1-yl)ethyl)-2-oxo-3,4-dihydroquinolin-1(2*H*)-yl)meth yl nonanoate | 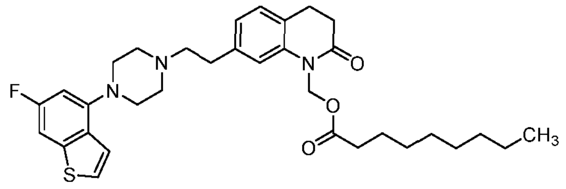 | 580.8[M+H]+ |
| 63 | (7-(2-(4-(6-Fluorobenzo[b] thiophe n-4-yl)piperazin-1-yl)ethyl)-2-oxo-3,4-dihydroquinolin-1(2*H*)-yl)meth yl undecanoate | 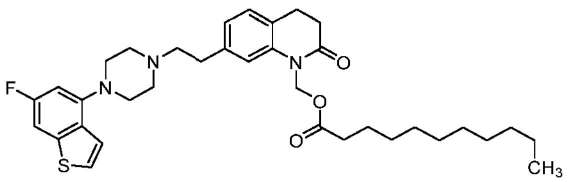 | 608.8[M+H]+ |
| 64 | (7-(2-(4-(6-Fluorobenzo[*b*] thiophe n-4-yl)piperazin-1-yl)ethyl)-2-oxo-3,4-dihydroquinolin-1(2*H*)-yl)meth yl tri-decanoate | 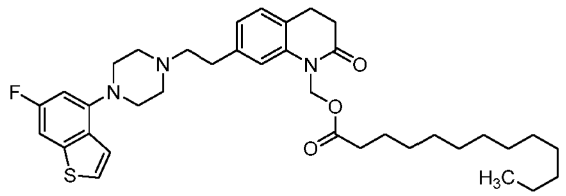 | 636.9[M+H]+ |
| 65 | (7-(2-(4-(6-Fluorobenzo[*b*] thiophe n-4-yl)piperazin-1-yl)ethyl)-2-oxo-3,4-dihydroquinolin-1(2*H*)-yl)meth yl tetradecanoate | 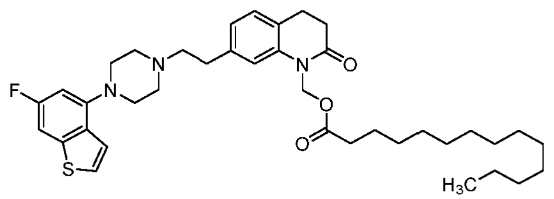 | 650.9[M+H]+ |
| 66 | (7-(2-(4-(6-Fluorobenzo[*b*] thiophe n-4-yl)piperazin-1-yl)ethyl)-2-oxo-3,4-dihydroquinolin-1(2*H*)-yl)meth yl pentadecanoate | 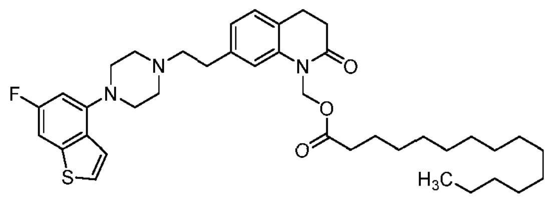 | 664.9[M+H]+ |
| 67 | (7-(2-(4-(6-Fluorobenzo[*b*] thiophe n-4-yl)piperazin-1-yl)ethyl)-2-oxo-3,4-dihydroquinolin-1(2*H*)-yl)meth yl heptadecanoate | 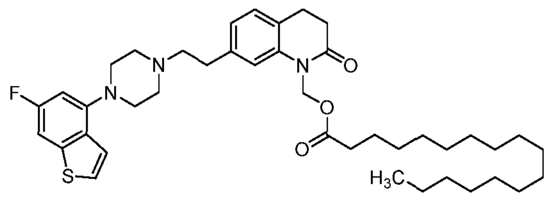 | 693.0[M+H]+ |
| 68 | (7-(2-(4-(6-Fluorobenzo[b] thiophe n-4-yl)piperazin-1-yl)ethyl)-2 -oxo-3,4-dihydroquinolin-1(2*H*)-yl)meth yl stearate | 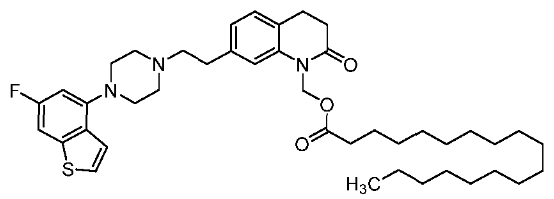 | 707.0[M+H]+ |

(continued)

| Compound No. | Name of the compounds | Structure | ESI-MS (*m/z*) |
|---|---|---|---|
| 69 | (7-(2-(4-(6-Fluorobenzo[*b*] thiophe n-4-yl)piperazin-1-yl)ethyl)-2-oxo-3,4-dihydroquinolin-1(2*H*)-yl)meth yl icosanoate | 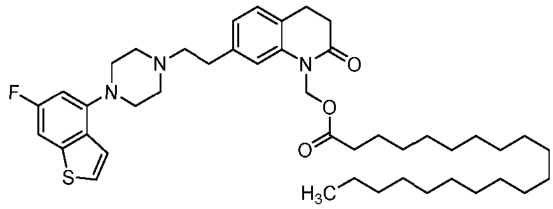 | 735.1[M+H]+ |
| 70 | (7-(2-(4-(6-Fluorobenzo[b] thiophe n-4-yl)piperazin-1-yl)ethyl)-2-oxo-3,4-dihydroquinolin-1(2*H*)-yl)meth yl al-lylcarbamate | 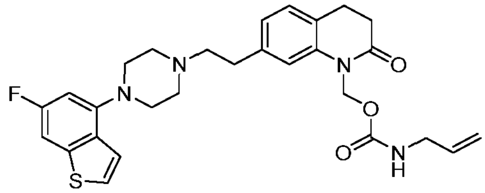 | 523.6[M+H]+ |
| 71 | (7-(2-(4-(6-Fluorobenzo[*b*] thiophe n-4-yl)piperazin-1-yl)ethyl)-2-oxo-3,4-dihydroquinolin-1(2*H*)-yl)meth yl decylcarbamate | 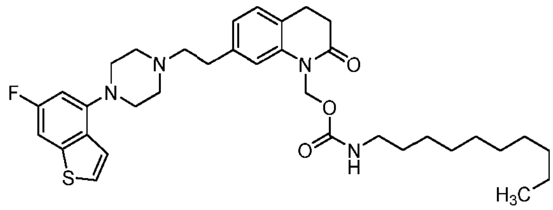 | 623.8[M+H]+ |
| 72 | (7-(2-(4-(6-Fluorobenzo[*b*] thiophe n-4-yl)piperazin-1-yl)ethyl)-2-oxo-3,4-dihydroquinolin-1(2*H*)-yl)meth yl undec-10-enoate | 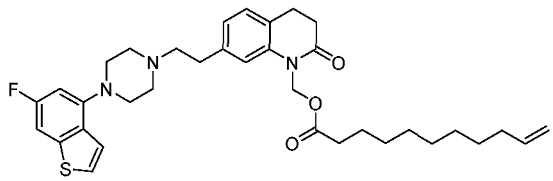 | 606.8[M+H]+ |
| 73 | (7-(2-(4-(6-Fluorobenzo[*b*] thiophe n-4-yl)piperazin-1-yl)ethyl)-2-oxo-3,4-dihydroquinolin-1(2*H*)-yl)methyl oleate | 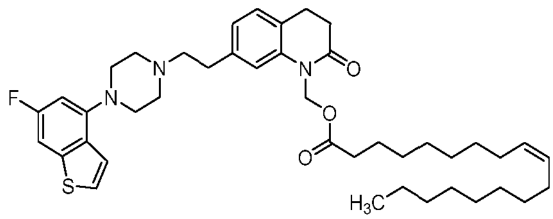 | 705.0[M+H]+ |
| 75 | (7-(2-(4-(6-Fluorobenzo[*b*] thiophe n-4-yl)piperazin-1-yl)ethyl)-2-oxo-3,4-dihydroquinolin-1(2*H*)-yl)meth yl tetracosanoate | 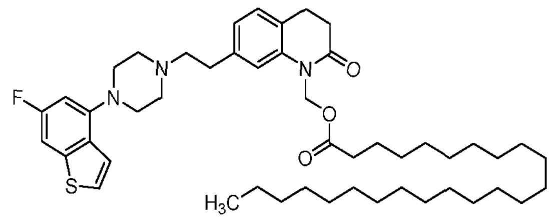 | 791.2[M+H]+ |
| 77 | (7-(2-(4-(6-Fluorobenzo[*b*] thiophe n-4-yl)piperazin-1-yl)ethyl)-2-oxo quinolin-1(2*H*)-yl)methyl oleate | 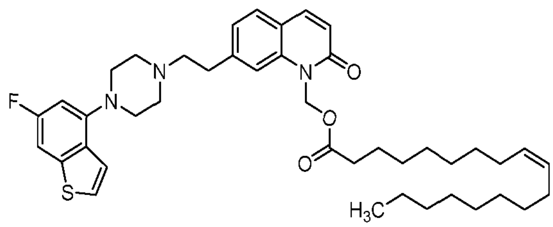 | 703.0[M+H]+ |

(continued)

| Compound No. | Name of the compounds | Structure | ESI-MS (*m/z*) |
|---|---|---|---|
| 78 | (7-(2-(4-(6-Fluorobenzo[b] thiophe n-4-yl)piperazin-1-yl)ethyl)-2-oxo-3,4-dihydroquinolin-1(2*H*)-yl)meth yl cyclobutanecarboxylate | 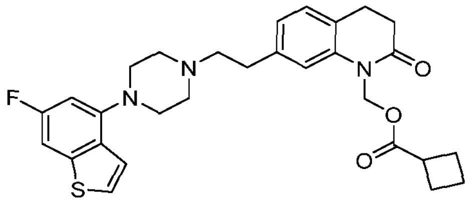 | 522.7[M+H]+ |
| 79 | (7-(2-(4-(6-Fluorobenzo[*b*] thiophe n-4-yl)piperazin-1-yl)ethyl)-2-oxo-3,4-dihydroquinolin-1(2*H*)-yl)meth yl cyclopentanecarboxylate | 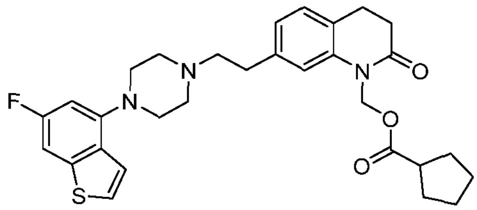 | 536.7[M+H]+ |
| 80 | (7-(2-(4-(6-Fluorobenzo[*b*] thiophe n-4-yl)piperazin-1-yl)ethyl)-2-oxo-3,4-dihydroquinolin-1(2*H*)-yl)meth yl cyclohexanecarboxylate | 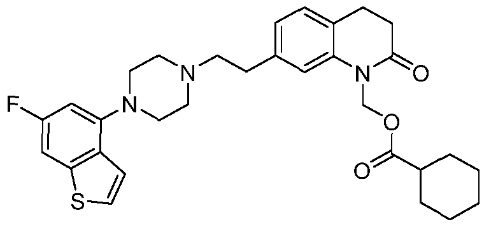 | 550.7[M+H]+ |
| 81 | (7-(2-(4-(6-Fluorobenzo[*b*] thiophe n-4-yl)piperazin-1-yl)ethyl)-2-oxo-3,4-dihydroquinolin-1(2*H*)-yl)meth yl piperidine-1-carboxylate | 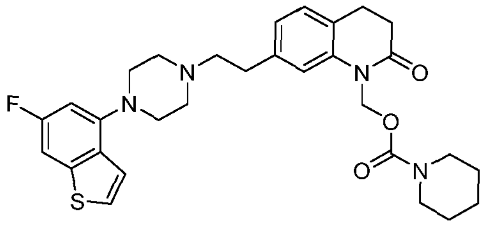 | 551.7[M+H]+ |
| 82 | (7-(2-(4-(6-Fluorobenzo[*b*] thiophe n-4-yl)piperazin-1-yl)ethyl)-2-oxo-3,4-dihydroquinolin-1(2*H*)-yl)meth yl cycloheptanecarboxylate | 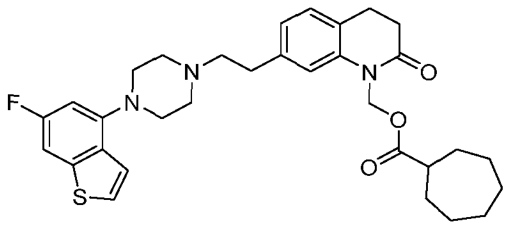 | 564.7[M+H]+ |
| 83 | (7-(2-(4-(6-Fluorobenzo[*b*] thiophe n-4-yl)piperazin-1-yl)ethyl)-2-oxo-3,4-dihydroquinolin-1(2*H*)-yl)meth yl cyclohexylcarbamate | 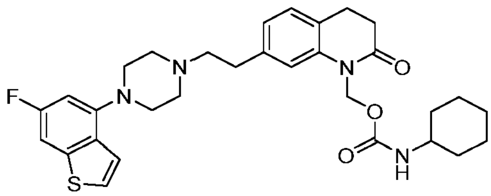 | 565.7[M+H]+ |
| 84 | (7-(2-(4-(6-Fluorobenzo[*b*] thiophe n-4-yl)piperazin-1-yl)ethyl)-2-oxo-3,4-dihydroquinolin-1(2*H*)-yl)meth yl (3,3,3-trifluoropropyl)carbamate | 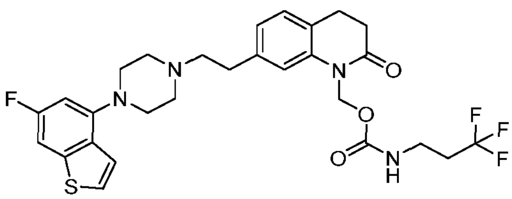 | 579.6[M+H]+ |

(continued)

| Compound No. | Name of the compounds | Structure | ESI-MS (*m/z*) |
|---|---|---|---|
| 85 | (7-(2-(4-(6-Fluorobenzo[*b*] thiophe n-4-yl)piperazin-1-yl)ethyl)-2 -oxo-3,4-dihydroquinolin-1(2*H*)-yl)meth yl (cyclohexvlmethyl)carbamate | 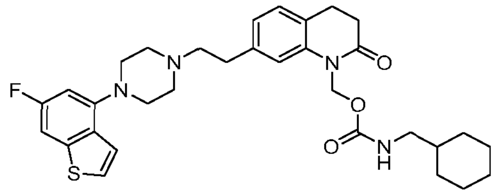 | 579.7[M+H]$^+$ |
| 86 | (7-(2-(4-(6-Fluorobenzo[*b*] thiophe n-4-yl)piperazin-1-yl)ethyl)-2-oxo-3,4-dihydroquinolin-1(2*H*)-yl)meth yl furan-2-carboxylate | 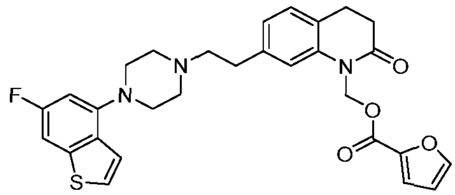 | 534.6[M+H]$^+$ |
| 87 | (7-(2-(4-(6-Fluorobenzo[*b*] thiophe n-4-yl)piperazin-1-yl)ethyl)-2-oxo-3,4-dihydroquinolin-1(2*H*)-yl)meth yl furan-3-carboxylate | 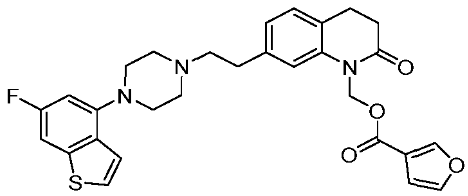 | 534.6[M+H]$^+$ |
| 88 | (7-(2-(4-(6-Fluorobenzo[*b*] thiophe n-4-yl)piperazin-1-yl)ethyl)-2-oxo quinolin-1(2*H*)-yl)methyl furan-2-carboxylate | 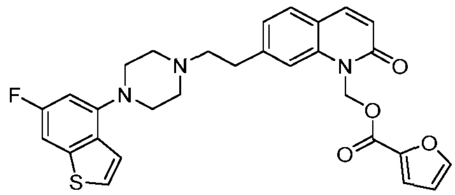 | 532.6[M+H]$^+$ |
| 89 | (7-(2-(4-(6-Fluorobenzo[*b*] thiophe n-4-yl)piperazin-1-yl)ethyl)-2-oxo-3,4-dihydroquinolin-1(2*H*)-yl)meth yl thiophene-2-carboxylate | 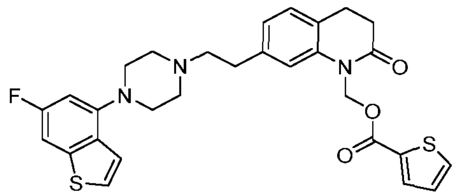 | 550.7[M+H]$^+$ |
| 90 | (7-(2-(4-(6-Fluorobenzo[*b*] thiophe n-4-yl)piperazin-1-yl)ethyl)-2-oxo-3,4-dihydroquinolin-1(2*H*)-yl)methyl tetrahydro-2*H*-pyran-4-carboxylate | 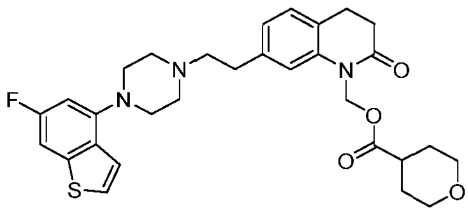 | 552.7[M+H]$^+$ |
| 91 | (7-(2-(4-(6-Fluorobenzo[*b*] thiophe n-4-yl)piperazin-1-yl)ethyl)-2-oxo-3,4-dihydroquinolin-1(2*H*)-yl)meth yl picolinate | 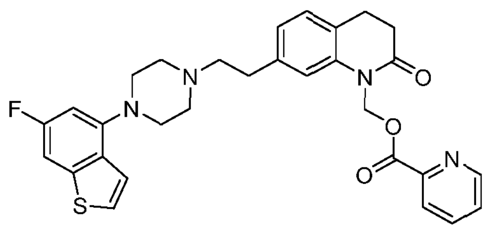 | 545.6[M+H]$^+$ |

(continued)

| Compound No. | Name of the compounds | Structure | ESI-MS (*m/z*) |
|---|---|---|---|
| 92 | (7-(2-(4-(6-Fluorobenzo[*b*] thiophe n-4-yl)piperazin-1-yl)ethyl)-2-oxo-3,4-dihydroquinolin-1(2*H*)-yl)meth yl ni-cotinate | 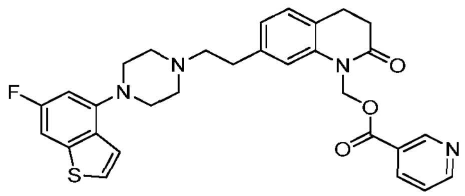 | 545.6[M+H]+ |
| 93 | (7-(2-(4-(6-Fluorobenzo[*b*] thiophe n-4-yl)piperazin-1-yl)ethyl)-2-oxo-3,4-dihydroquinolin-1(2*H*)-yl)meth yl pyrimidine-5-carboxylate | 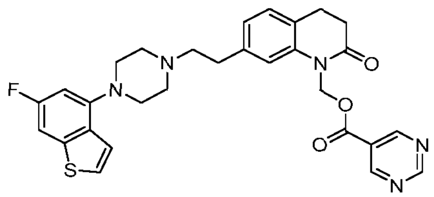 | 546.6[M+H]+ |
| 94 | (7-(2-(4-(6-Fluorobenzo[*b*] thiophe n-4-yl)piperazin-1-yl)ethyl)-2-oxo-3,4-dihydroquinolin-1(2*H*)-yl)meth yl quinoline-6-carboxylate | 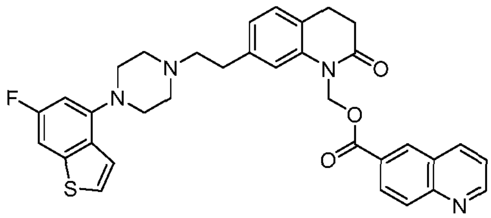 | 595.7[M+H]+ |
| 95 | (7-(2-(4-(6-Fluorobenzo[*b*] thiophe n-4-yl)piperazin-1-yl)ethyl)-2-oxo-3,4-dihydroquinolin-1(2*H*)-yl)meth yl benzoate | 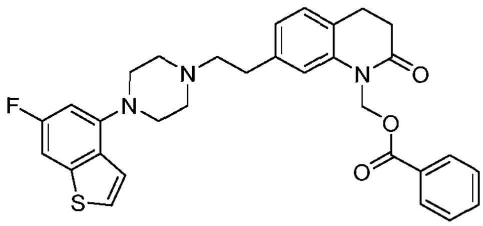 | 544.6[M+H]+ |
| 96 | (7-(2-(4-(6-Fluorobenzo[*b*] thiophe n-4-yl)piperazin-1-yl)ethyl)-2-oxo-3,4-dihydroquinolin-1(2*H*)-yl)meth yl 2-phenylacetate | 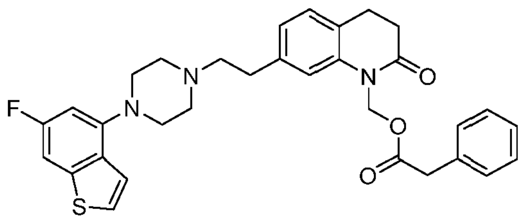 | 558.7[M+H]+ |
| 97 | (7-(2-(4-(6-Fluorobenzo[*b*] thiophe n-4-yl)piperazin-1-yl)ethyl)-2-oxo-3,4-dihydroquinolin-1(2*H*)-yl)meth yl 4,4,4-trifluorobutanoate | 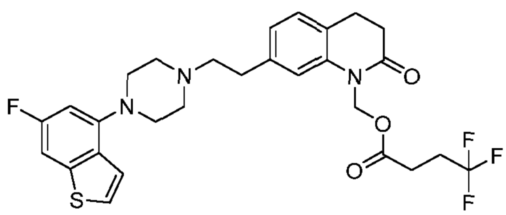 | 564.6[M+H]+ |
| 98 | (7-(2-(4-(6-Fluorobenzo[*b*] thiophe n-4-yl)piperazin-1-yl)ethyl)-2-oxo-3,4-dihydroquinolin-1(2*H*)-yl)meth yl benzylcarbamate | 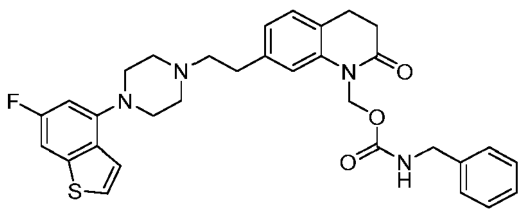 | 573.9[M+H]+ |

(continued)

| Compound No. | Name of the compounds | Structure | ESI-MS (*m/z*) |
|---|---|---|---|
| 99 | (7-(2-(4-(6-Fluorobenzo[*b*] thiophe n-4-yl)piperazin-1-yl)ethyl)-2-oxo-3,4-dihydroquinolin-1(2*H*)-yl)meth yl (2-hydroxyethyl)carbamate | 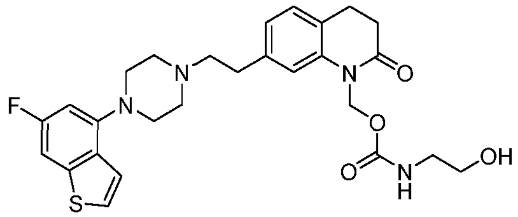 | 527.6[M+H]+ |
| 100 | (7-(2-(4-(6-Fluorobenzo[*b*] thiophe n-4-yl)piperazin-1-yl)ethyl)-2 -oxo-3,4-dihydroquinolin-1(2*H*)-yl)meth yl heptulcarbamate | 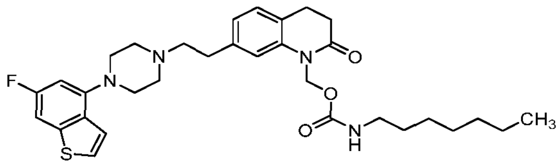 | 581.8[M+H]+ |
| 101 | (7-(2-(4-(6-Fluorobenzo[*b*] thiophe n-4-yl)piperazin-1-yl)ethyl)-2-oxo-3,4-dihydroquinolin-1(2*H*)-yl)meth yl 2-(2-methoxyethoxy)acetate | 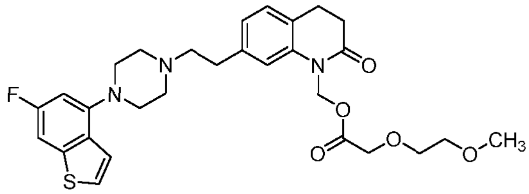 | 556.7[M+H]+ |
| 102 | (7-(2-(4-(6-Fluorobenzo[*b*] thiophe n-4-yl)piperazin-1-yl)ethyl)-2-oxo-3,4-dihydroquinolin-1(2*H*)-yl)meth yl 2-(2-(2-methoxyethoxy) ethoxy)ace tate | 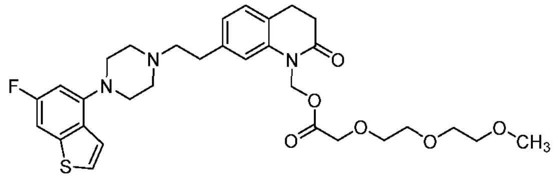 | 600.7[M+H]+ |
| 103 | (7-(2-(4-(6-Fluorobenzo[*b*] thiophe n-4-yl)piperazin-1-yl)ethyl)-2-oxo-3,4-dihydroquinolin-1(2*H*)-yl)meth yl 2-(2-butoxyethoxy)acetate | 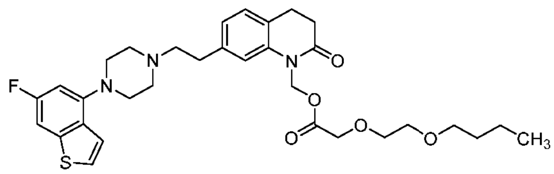 | 598.7[M+H]+ |
| 104 | *Tert*-butyl ((7-(2-(4-(6-fluorobenzo[*b*]thiophe n-4-yl)piperazin-1-yl)ethyl)-2-oxo-3,4-dihydroquinolin-1(2*H*)-yl)meth yl) malonate | 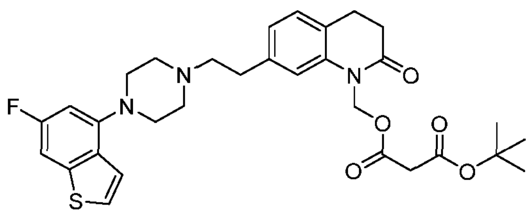 | 582.7[M+H]+ |
| 105 | (7-(2-(4-(6-Fluorobenzo[*b*] thiophe n-4-yl)piperazin-1-yl)ethyl)-2-oxo-3,4-dihydroquinolin-1(2*H*)-yl)meth yl 2-methylbutanoate | 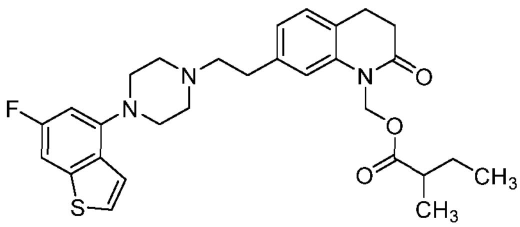 | 524.7[M+H]+ |

(continued)

| Compound No. | Name of the compounds | Structure | ESI-MS (*m*/*z*) |
|---|---|---|---|
| 106 | 7-(2-(4-(6-Fluorobenzo[*b*] thiophen -4-yl)piperazin-1-yl)ethyl)-2-oxo-3 ,4-dihydroquinolin-1(2*H*)-yl)methy 14-methylpentanoate | 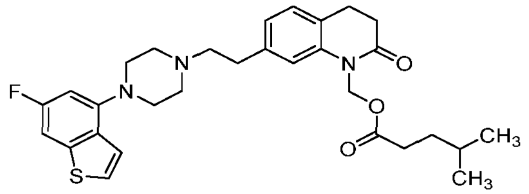 | 538.7[M+H]+ |
| 107 | (7-(2-(4-(6-Fluorobenzo[*b*] thiophe n-4-yl)piperazin-1-yl)ethyl)-2 -oxo-3,4-dihydroquinolin-1(2*H*)-yl)meth yl 2-methylpentanoate | 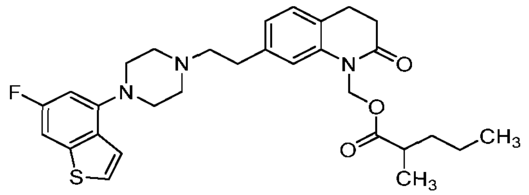 | 538.7[M+H]+ |
| 108 | (7-(2-(4-(6-Fluorobenzo[*b*] thiophe n-4-yl)piperazin-1-yl)ethyl)-2-oxo-3,4-dihydroquinolin-1(2*H*)-yl)meth yl 2,2-dimethylhexanoate | 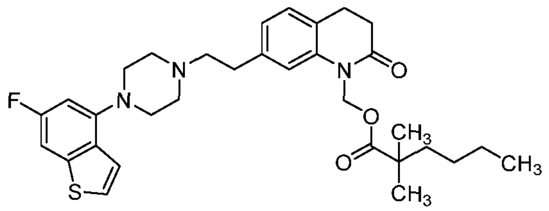 | 566.7[M+H]+ |
| 109 | (7-(2-(4-(6-Fluorobenzo[*b*] thiophe n-4-yl)piperazin-1-yl)ethyl)-2-oxo-3,4-dihydroquinolin-1(2*H*)-yl)meth yl 2-methylhexanoate | 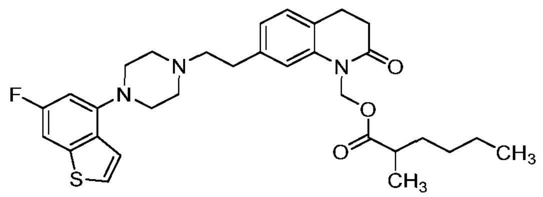 | 552.7[M+H]+ |
| 110 | (7-(2-(4-(6-Fluorobenzo[*b*] thiophe n-4-yl)piperazin-1-yl)ethyl)-2-oxo-3,4-dihydroquinolin-1(2*H*)-yl)meth yl 2-methylheptanoate | 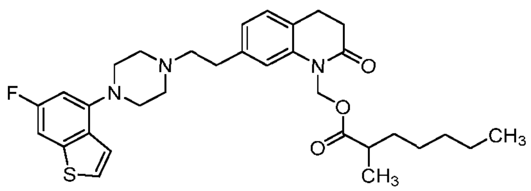 | 566.7[M+H]+ |
| 111 | (7-(2-(4-(6-Fluorobenzo[*b*] thiophe n-4-yl)piperazin-1-yl)ethyl)-2-oxo quinolin-1(2*H*)-yl)methyl pivalate | 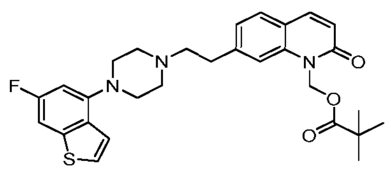 | 522.7[M+H]+ |
| 112 | (7-(2-(4-(6-Fluorobenzo[*b*] thiophe n-4-yl)piperazin-1-yl)ethyl)-2-oxo-3,4-dihydroquinolin-1(2*H*)-yl)meth yl 2-pentvlheptanoate | 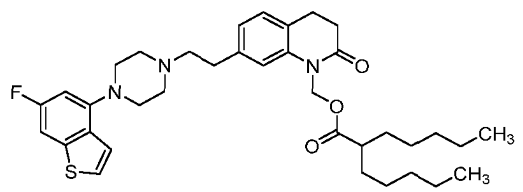 | 622.9[M+H]+ |
| 113 | (7-(2-(4-(6-Fluorobenzo[*b*] thiophe n-4-yl)piperazin-1-yl)ethyl)-2-oxo quinolin-1(2*H*)-yl)methyl 2-pentyl-heptanoate | 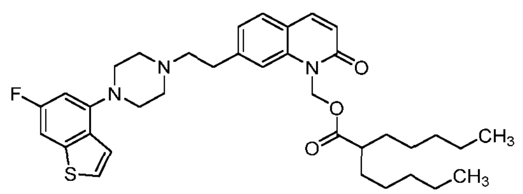 | 620.8[M+H]+ |

(continued)

| Compound No. | Name of the compounds | Structure | ESI-MS (*m*/*z*) |
|---|---|---|---|
| 114 | (7-(2-(4-(6-Fluorobenzo[*b*] thiophe n-4-yl)piperazin-1-yl)ethyl)-2-oxo-3,4-dihydroquinolin-1(2*H*)-yl)meth yl glycinate | 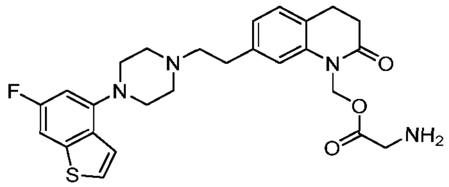 | 497.6[M+H]+ |
| 115 | 1-Benzoyl-7-(2-(4-(6-fluorobenzo[ *b*]thiophen-4-yl)piperazin-1-yl)eth yl)-3,4-dihydroquinolin-2(1*H*)-one | 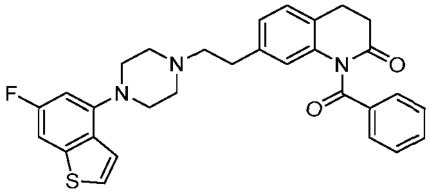 | 514.6[M+H]+ |
| 116 | 7-(2-(4-(6-Fluorobenzo[*b*] thiophen -4-yl)piperazin-1-yl)ethyl)-1-(2-ph enylacetyl)-3,4-dihydroquinolin-2( 1*H*)-one | 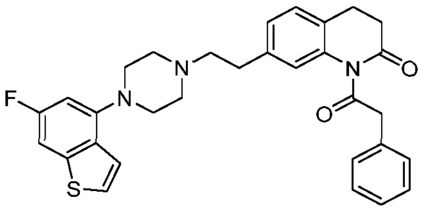 | 528.7[M+H]+ |
| 117 | Phenyl 7-(2-(4-(6-fluorobenzo [*b*]thiophen-4-yl)piperazin-1-yl)ethyl)-2-oxo-3, 4-dihydroquinoline-1(2*H*)-carboxy late | 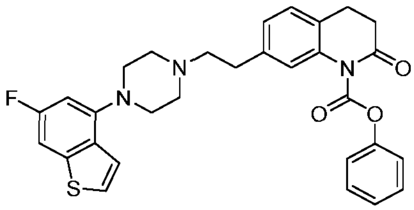 | 530.6[M+H]+ |
| 118 | Benzyl 7-(2-(4-(6-fluorobenzo [*b*]thiophen-4-yl)piperazin-1-yl)ethyl)-2-oxo-3, 4-dihydroquinoline-1(2*H*)-carboxy late | 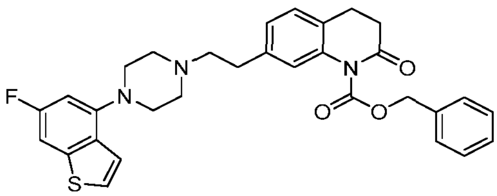 | 544.7[M+H]+ |
| 119 | 1-(Cyclopentanecarbonyl)-7-(2-(4-(6-fluorobenzo [*b*]thiophen-4-yl)pip erazin-1-yl)ethyl)-3,4-dihydroquin olin-2(1*H*)-one | 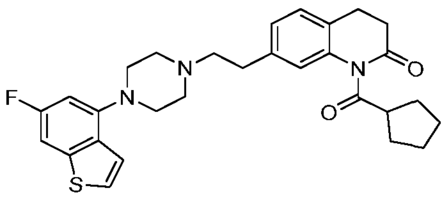 | 506.7[M+H]+ |
| 120 | 7-(2-(4-(6-Fluorobenzo[*b*] thiophen -4-yl)piperazin-1-yl)ethyl)-1-(2-hy droxyacetyl)-3,4-dihydroquinolin-2 (1*H*)-one | 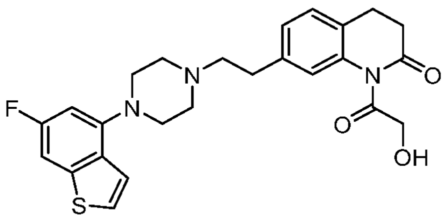 | 468.6[M+H]+ |

(continued)

| Compound No. | Name of the compounds | Structure | ESI-MS (*m/z*) |
|---|---|---|---|
| 121 | 7-(2-(4-(6-Fluorobenzo[*b*] thiophen -4-yl)piperazin-1-yl)ethyl)-1-glycy 1-3,4-dihydroquinolin-2(1*H*)-one | 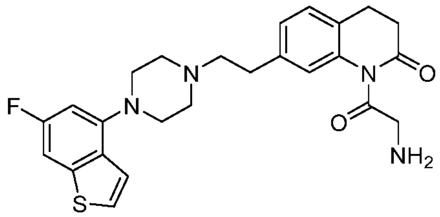 | 467.6[M+H]+ |
| 122 | 7-(2-(4-(6-Fluorobenzo[*b*] thiophen -4-yl)piperazin-1-yl)ethyl)-*N*-(2-hy droxyethyl)-2-oxo-3,4-dihydroquin oline-1(2*H*)-carboxamide | 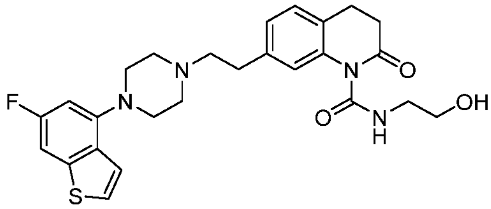 | 497.6[M+H]+ |
| 123 | 7-(2-(4-(6-Fluorobenzo[*b*] thiophen -4-yl)piperazin-1-yl)ethyl)-2-oxo*N*-propyl-3,4-dihydroquinoline-1(2 *H*)-carboxamide | 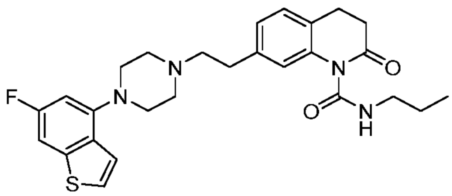 | 495.6[M+H]+ |
| 124 | 7-(2-(4-(6-Fluorobenzo[*b*] thiophen -4-yl)piperazin-1-yl)ethyl)-1-(2-(2-methoxyethoxy)acetyl)-3,4-dihydr oquinolin-2(1*H*)-one | 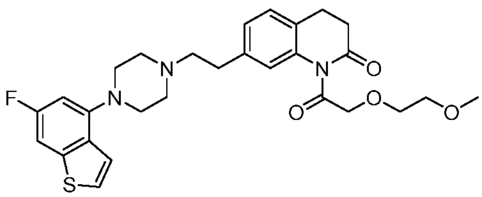 | 526.6[M+H]+ |
| 125 | 7-(2-(4-(6-Fluorobenzo[*b*] thiophen -4-yl)piperazin-1-yl)ethyl)-1-(2-(2-methoxyethoxy)acetyl)quinolin-2( 1*H*)-one | 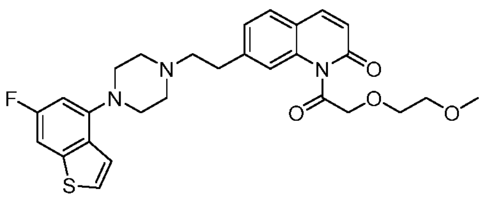 | 524.6[M+H]+ |
| 126 | 7-(2-(4-(6-Fluorobenzo[*b*] thiophen -4-yl)piperazin-1-yl)ethyl)-1-(2-(2-(2-methoxyethoxy)ethoxy)acetyl)-3,4-dihydroquinolin-2(1*H*)-one | 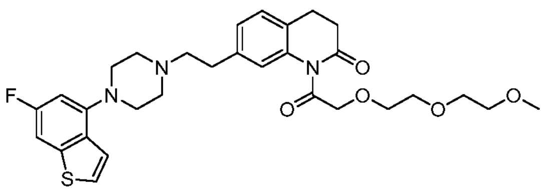 | 570.7[M+H]+ |
| 127 | *N*-Benzyl-7-(2-(4-(6-fluorobenzo[*b* ]thiophen-4-yl)piperazin-1-yl)ethyl )-2-oxo-3,4-dihydroquinoline-1(2*H* )-carboxamide | 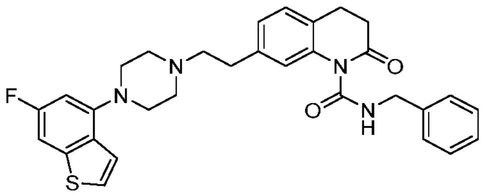 | 543.7[M+H]+ |

(continued)

| Compound No. | Name of the compounds | Structure | ESI-MS (*m/z*) |
|---|---|---|---|
| 128 | 7-(2-(4-(6-Fluorobenzo[*b*] thiophen -4-yl)piperazin-1-yl)ethyl)-1-(morp holine-4-carbonyl)-3,4-dihydroqui nolin-2(1*H*)-one | 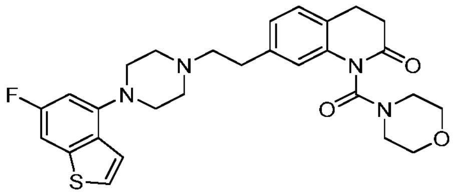 | 523.6[M+H]+ |
| 129 | 1-(Cyclohexanecarbonyl)-7-(2-(4-( 6-fluorobenzo [*b*]thiophen-4-yl)pip erazin-1-yl)ethyl)-3,4-dihydroquin olin-2(1*H*)-one | 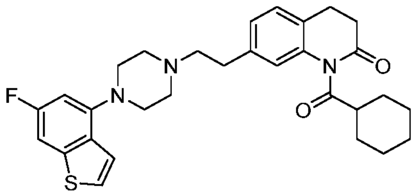 | 520.7[M+H]+ |
| 130 | 7-(2-(4-(6-Fluorobenzo[*b*] thiophen -4-yl)piperazin-1-yl)ethyl)-1-(2-me thoxyacetyl)-3,4-dihydroquinolin-2 (1*H*)-one | 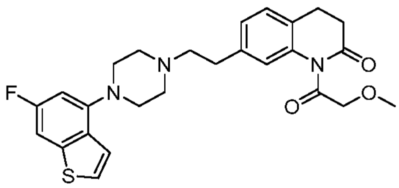 | 482.6[M+H]+ |
| 131 | 2-Methoxyethyl 7-(2-(4-(6-fluorobenzo[*b*]thiophen-4-yl)piperazin-1-yl)ethyl)-2-oxo-3, 4-dihydroquinoline-1(2*H*)-carboxy late | 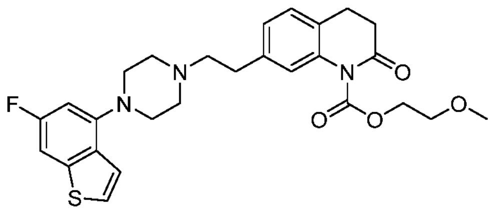 | 512.6[M+H]+ |
| 132 | 7-(2-(4-(6-Fluorobenzo[*b*] thiophen -4-yl)piperazin-1-yl)ethyl)-1-(unde c-10-en-oyl)-3,4-dihydroquino-lin-2( 1*H*)-one | 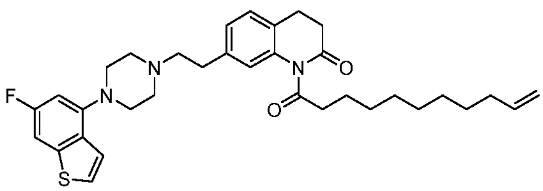 | 576.8[M+H]+ |
| 133 | (Decanoyloxy)methyl 7-(2-(4-(6-fluorobenzo[*b*] thiophen-4-yl)piperazin-1-yl)ethyl)-2-oxo-3, 4-dihydroquinoline-1(2*H*)-carboxy late | 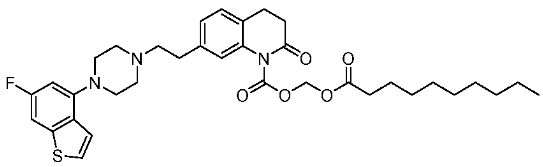 | 638.8[M+H]+ |
| 134 | (7-(2-(4-(6-Fluorobenzo[*b*] thiophe n-4-yl)piperazin-1-yl)ethyl)-2-oxo quinolin-1(2*H*)-yl)methyl dode-canoate | 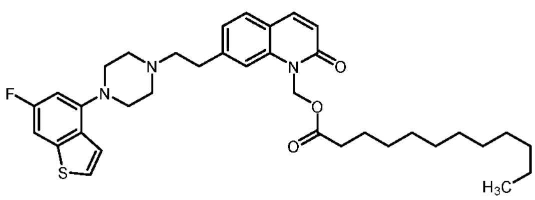 | 620.8[M+H]+ |

**Example**

1) Assay of the Agonistic Activity Against the 5-$HT_{1A}$ Receptor

The agonistic effect of the compounds on the 5-$HT_{1A}$ receptor in the HEK293 cells expressing the human

recombinant 5-$HT_{1A}$ receptor was assayed by using the LANCE™ cAMP 384 kit (PerkinElmer, USA). The agonistic effect of the compounds on the 5-$HT_{1A}$ receptor was assessed by assaying the inhibitory effect of the test compounds on cAMP production in HEK293 cells. cAMP concentration was determined according to the method described in the kit instructions, and the compounds were tested at concentrations of 0.0128-1000 nM. 8-OH-DPAT was used as a positive control. The specific procedures were as follows:

a) the compounds were added to an assay plate (384-well plate) with Echo 550, with the final concentration of DMSO of < 1%, and cells were collected with a stimulation buffer (HBSS + 5 mM HEPES + 0.5 mM IBMX + 0.1% BSA);
b) 10 μL of prepared cell solution was transferred to the assay plate filled with the compounds;
c) the plate was centrifuged at 600 rpm for 3 min and incubated at room temperature for 1 h;
d) 5 μL of 4× Eu-cAMP tracer solution and 5 μL of 4× ULight™-anti-cAMP solution were added, and the plate was centrifuged at 600 rpm for 3 min and incubated at room temperature for 1 h;
c) data were read using EnVision and $EC_{50}$ was calculated by using Graphpad Prism software, with the results shown in Table 1.

2) Assay of the Antagonistic Activity Against the $D_{2L}$ Receptor

The antagonistic effect of the compounds on the $D_{2L}$ receptor in the CHO cells expressing the human recombinant $D_{2L}$ receptor was assayed by using the LANCE® Ultra cAMP kit (PerkinElmer, USA). The antagonistic effect of the compounds on the $D_2$ receptor was assessed by assaying the antagonism of the test compounds to the inhibitory effect of dopamine on cAMP production in CHO cells. cAMP concentration was determined according to the method described in the kit instructions, and the compounds were tested at a starting concentration of 1000 nM in 5-fold dilution. Amisulpride was used as a positive control. The specific procedures were as follows:

a) 1× simulation buffer was prepared according to the kit instructions for later use;
b) the positive compound and the test compounds were serially diluted with DMSO to obtain 10 concentrations, and then the positive compound & DMSO were diluted to 10× with a 1× stimulation buffer;
c) $D_{2L}$-CHO cells were digested with trypsin, the culture medium was removed after centrifugation, and the cells were resuspended in a 1× stimulation buffer, counted, and seeded into a 384-well plate at a density of 3000 cells/well/5 μL;
d) 1 μL of each of the compounds diluted in step b) was added to corresponding experiment wells (10× for the compounds, 10 concentrations, 2 duplicate wells), wherein 1 μL of 10× positive compound (initial concentration) was added to the PC wells, and 1 μL of 10× DMSO buffer (2%) was added to the VC wells, and the plate was incubated at 37 °C for 20 min after centrifugation;
c) 4 μL of 10 μM Forskolin & 25 nM dopamine solution was added to the $D_{2L}$ receptor inhibitor experimental wells; after centrifugation, the plate was incubated at 37 °C for 30 min to induce cAMP production;
f) Eu-cAMP was diluted to a working concentration with a detection buffer, and the dilution was added to corresponding experiment wells at 5 μL/well;
g) ULight™-anti-cAMP was diluted to a working concentration with the detection buffer, and the dilution was added to corresponding experiment wells at 5 μL/well; the plate was incubated at room temperature for 1 h after centrifugation;
h) after incubation, readings at 665 nm and 620 nm were measured. The inhibition rates of the compounds at different concentrations were calculated and $IC_{50}$ was calculated by using Graphpad Prism software, with the results shown in Table 1.

3) Assay of the Antagonistic Activity Against the 5-$HT_{2A}$ Receptor

The antagonistic effect of the compounds on the 5-$HT_{2A}$ receptor in the CHO cells expressing the human recombinant 5-$HT_{2A}$ receptor was assayed by using the IP-One-Gq kit (Cisbio). The assay was performed according to the method described in the kit instructions, and the compounds were tested at a starting concentration of 5000 nM in 5-fold dilution. Risperidone was used as a positive control. The procedures were as follows:

a) 5-$HT_{2A}$-CHO cells were digested with trypsin, the culture medium was removed after centrifugation, the cells were resuspended in F12 + 10% dialyzed FBS culture medium, counted, and seeded into a 384-well plate at a density of 7000 cells/well/25 μL, and the plate was incubated in an incubator for about 18-20 h after centrifugation;
b) 1× simulation buffer was prepared according to the kit instructions for later use;
c) the positive compound and the test compounds were serially diluted with DMSO to obtain 10 concentrations, and then the compounds were diluted to 10× with a 1× stimulation buffer and shaken and mixed uniformly for later use;
d) the cell plate was taken, inverted, and centrifuged, the culture medium was discarded, and then 9.1 μL of 1× stimulation buffer was added to the experimental wells;

c) 1.4 μL of each of 10× compounds diluted in step c) was added to corresponding experiment wells (10× for the compounds, 10 concentrations, 2 duplicate wells), wherein 1.4 μL of the positive compound at a 10× initial concentration was added to the PC wells, and 1.4 μL of 10× DMSO buffer (1%) was added to the VC wells, and the plate was incubated at 37 °C for 10 min after centrifugation;
f) a 160 nM serotonin solution was prepared with a 1× stimulation buffer, 3.5 μL of the solution was added to each of the experimental wells, and the plate was incubated at 37 °C for 60 min after centrifugation;
g) d2-IP1 and anti-IP1-cryptate were diluted to working concentrations with a lysis & detection buffer;
h) after incubation, 3 μL of d2-IP1 was added to each of the experimental wells;
I) then 3 μL of anti-IP1-cryptate was added to each of the experimental wells, and the plate was left to stand at room temperature for 1 h after centrifugation;
j) after incubation, readings at 665 nm and 620 nm were measured.

The inhibition rates of the compounds at different concentrations were calculated and $IC_{50}$ was calculated by using Graphpad Prism software, with the results shown in Table 1. Table 1:

| Test compound | Agonistic activity against 5-$HT_{1A}$ receptor ($EC_{50}$, nM) | Antagonistic activity of $D_{2L}$ receptor ($IC_{50}$, nM) | Antagonistic activity of 5-$HT_{2A}$ receptor ($IC_{50}$, nM) |
|---|---|---|---|
| Compound 1-a | 1.0 | 4.4 | 33 |
| Compound 1 | 0.7 | 3.5 | 32 |
| Amisulpride | / | 1.1 | / |
| 8-OH-DPAT | 5.0 | / | / |
| Risperidone | / | / | 0.76 |

4) Inhibitory Activity Against the 5-HT transporter (SERT)

The inhibitory effect of the compounds on the 5-HT transporter in the HEK-293 cells expressing human SERT was evaluated by using the neurotransmitter transporter uptake assay kit (Molecular Devices). The assay was performed according to the method described in the kit instructions. Citalopram was used as a positive control. The specific procedures were as follows:
a) HEK-hSERT cells were seeded into a 384-well plate at 20,000/well, and then the plate was transferred to an incubator at 37 °C and incubated overnight;
b) the next day, citalopram was transferred to the 384 well plate with a 0.1% BSA solution, with final test concentrations of 2 μM, 0.5 μM, 0.125 μM, 30 nM, 10 nM, 3 nM, 1 nM, 0.3 nM, 0.1 nM, and 0.03 nM; the test compounds were tested at an initial concentration of 10 μM in 3-fold dilution, with 2 replicates for each concentration;
c) the 384-well plate was taken from the incubator, the culture medium was pipetted from the wells, and the test compound solutions were added at 25 μL/well; the plate was incubated at 37 °C for 30 min; 25 μL of fluorescent dye detection solution was added to each well, and the plate was incubated at 37 °C for 30 min;
d) fluorescence values on Flexstation 3 were read and data were analyzed by using Graphpad Prism software, with results shown in Table 2.

Table 2:

| Test compound | Inhibitory activity against 5-HT transporter ($IC_{50}$, nM) |
|---|---|
| Compound 1-a | 15.0 |
| Compound 1 | 13.0 |
| Positive control citalopram | 8.6 |

5) Agonistic Effect on the Dopamine $D_3$ Receptor

The agonistic activity of the compounds on the $D_3$ receptor in CHO cells expressing the human $D_3$ receptor was assessed by determining the effect of the compounds on cAMP modulation by the HTRF detection method.

The experimental scheme was as follows: the cells were suspended in HBSS buffer containing 20 mM Hepes/NaOH (pH 7.4), 70 mM NaCl, 5.33 mM KCl, 1.25 mM $CaCl_2$, 0.5 mM $MgCl_2$, 0.41 mM $MgSO_4$, 0.441 mM $KH_2PO_4$, 0.3 mM $Na_2HPO_4$, 0.1% glucose, 0.1% BSA, and 500 μM IBMX. Then the suspension was distributed in a microplate at a

density of $10^4$ cells/well in the presence of any one of the following components: HBSS (basal control), 300 nM dopamine (stimulation control), or different concentrations of dopamine (for $EC_{50}$ assay) or test compounds. Thereafter, an adenylate cyclase activator NKH 477 was added at a final concentration of 1.5 μM. After incubation at 37 °C for 30 min, the cells were lysed, and a fluorescent acceptor (D2-labeled cAMP) and a fluorescent donor (europium-labeled anti-cAMP antibody) were added. After incubation at room temperature for 60 min, fluorescence transfer was measured at em = 337 nm and em = 620 nm and 665 nm by using a microplate reader (Envision, Perkin Elmer). cAMP concentration was determined by dividing the signal measured at em = 665 nm by the signal measured at em = 620 nm (ratio). Data were analyzed by using Graphpad Prism software, with the results shown in Table 3.

Table 3:

| Test compound | Agonistic activity against the $D_3$ receptor ($EC_{50}$, nM) |
|---|---|
| Compound 1-a | NA |
| Compound 1 | 7.1 |
| Positive control dopamine | 1.0 |
| NA: no effect | |

6) Assay of the hERG potassium channel activity

The hERG potassium channel block experiment was performed using the full-automatic patch-clamp technique ($QPatch^{HTX}$, Sophion, Stockholm, Sweden). The cell line is CHO cells stably expressing the hERG potassium channel. The compounds were tested at the highest test concentration of 20 μM in 3-fold dilution (n = 3). The DMSO content in the final test concentration did not exceed 0.2%. Data were analyzed by using Graphpad Prism software, with the results shown in Table 4.

Table 4:

| Test compound | hERG inhibition ($IC_{50}$, μM) |
|---|---|
| Compound 1-a | 0.92 |
| Compound 1 | 1.81 |
| Positive control cisapride | 0.03 |

As can be seen from the results in Tables 1 to 4 above, compound 1-a and compound 1 had similar $D_2$ receptor antagonistic/5-$HT_{1A}$ receptor agonistic/5-$HT_{2A}$ receptor antagonistic activity, had an inhibitory effect on the 5-HT transporter, and were expected to be able to better improve comorbidities such as anxiety and depression of schizophrenia. Compared with compound 1-a, compound 1 had dopamine $D_3$ receptor agonistic activity, and was expected to have a better therapeutic effect on neuropsychiatric diseases and lower extrapyramidal side effects. Compared with compound 1-a, compound 1 had a lower hERG inhibitory effect, reduced the risk of cardiotoxicity related to hERG, and was expected to have better clinical safety.

7) Human Liver Microsome Assay

Preparation of magnesium-potassium buffer solution: a 100 mM potassium buffer and 5 mM $MgCl_2$ were preheated together to obtain a magnesium-potassium buffer at a pH of 7.41. Preparation of 0.5 mM working solution: a 10 mM compound stock solution was added to 95 μL of acetonitrile. Preparation of 1.5 μM working solution containing microsomes (0.75 mg/mL): 1.5 μL of the 0.5 mM working solution and 18.75 μL of 20 mg/mL human liver microsome solution were added to 479.75 μL of the magnesium-potassium buffer. Preparation of NADPH stock solution (6 mM, 5 mg/mL): NADPH was dissolved in the magnesium-potassium buffer. 30 μL of 1.5 μM working solution containing 0.75 mg/mL human liver microsomes were added to each of the wells of a 96-well plate, and the plate was pre-incubated at 37 °C for 5 min. For a sample at 0 min, 150 μL of acetonitrile solution containing an internal standard was added, and then 15 μL of NADPH stock solution (6 mM) was added to initiate the reaction. For samples at other time points, 15 μL of NADPH stock solution (6 mM) was directly added to initiate the reaction. Final content of components: compound (1 μM), human liver microsomes (0.5 mg/mL), and NADPH (2 mM). 150 μL of cold stop buffer containing an internal standard was added at time points of 5 min, 15 min, 30 min, and 45 min to stop the sample reaction, the plate was centrifuged at 600 rpm for 10 min and then at 6000 rpm for 15 min, and the supernatant was taken for LC-MS/MS analysis.

A standard curve of the ln residual rate of the drug in the incubation system with respect to the incubation time was plotted by using Excel software, linear regression was performed to obtain a slope *k,* and the half-life $T_{1/2}$ (min) and the intrinsic clearance $CL_{int}$ (mL/min/kg) value were calculated according to the following formula:

$$T_{1/2} = -0.693/k$$

$$CL_{int} = \frac{0.693}{in\ vitro\ T_{1/2}} \times \frac{\text{mL incubation volume}}{\text{mg microsome}} \times \frac{\text{mg microsome}}{\text{g liver weight}} \times \frac{\text{g liver weight}}{\text{Kg body weight}}$$

The experimental results are shown in Table 5 below:

Table 5:

| Test compound | Half-life ($t_{1/2}$, min) | Intrinsic clearance (Clint, mL/min/kg) |
|---|---|---|
| Compound 2 | 0.69 | 2531 |
| Compound 3 | 367 | 4.73 |
| Compound 5 | 0.87 | 2002 |
| Compound 6 | 1.0 | 1720 |
| Compound 7 | 30 | 56.81 |
| Compound 8 | >120 | NA |
| NA: not calculable. | | |

The above data showed that compound 3, compound 7, and compound 8 had good metabolic stability in human liver microsomes.

8) Oil Solubility Test

The solubility test was performed by quantitative analysis using an external standard method of high-performance liquid chromatography.

Instruments:

| Name | Model | Supplier |
|---|---|---|
| HPLC | Agilent 1100 | Agilent |
| Electronic balance | XP26DR | Mettler |
| Chromatographic column | LC-SH-493 Waters XTerra MS C18 150×4.6mm, 5μm | Waters |

Chromatographic conditions:

| | |
|---|---|
| Mobile phase A | A 20 mM sodium *n*-octylsufonate aqueous solution (about 4.32 g of sodium *n*-octylsufonate was weighed, dissolved in 1000 mL of water, adjusted to pH 2.0 with phosphoric acid, and filtered) |
| Mobile phase B | Acetonitrile |
| Gradient | <table><tr><td>Time, min</td><td>Solution A, %</td><td>Solution B, %</td></tr><tr><td>0</td><td>90</td><td>10</td></tr><tr><td>20</td><td>20</td><td>80</td></tr><tr><td>25</td><td>20</td><td>80</td></tr></table> |
| Equilibration time | 11 min |
| Flow rate | 1.0 ml/min |
| Detection wavelength | UV 254 nm |
| Column temperature | 30°C |
| Injection volume | 5 µl |
| Diluent | 80% acetonitrile |
| Sample concentration | 0.5 mg/ml |

Solution preparation:

Reference sample solution: 10 mg of reference sample was weighed, placed in a 20 mL volumetric flask, diluted to the scale with 80% acetonitrile, and shaken uniformly.

Test sample solution: an appropriate amount of sample was dissolved in benzyl benzoate to obtain a saturated solution. The mixture was placed in a shaker and shaken at 25 °C and 800 rpm for 2 h. The sample solution was filtered, and 100 µL of filtrate was added to a 10 mL volumetric flask, diluted to the scale with 80% acetonitrile, and shaken uniformly.

The solubility test results are shown in Table 6:

Table 6:

| Test compound | Solubility (mg/mL) |
|---|---|
| Compound 1-a | 22.36 |
| Compound 2 | 74.98 |
| Compound 3 | >200 |
| Compound 4 | 25.79 |
| Compound 5 | 23.44 |
| Compound 6 | 166.22 |

(continued)

| Test compound | Solubility (mg/mL) |
|---|---|
| Compound 7 | >200 |
| Compound 8 | >200 |
| Compound 9 | >200 |
| Compound 10 | >200 |
| Compound 13 | >200 |
| Compound 14 | >200 |
| Compound 15 | >200 |
| Compound 25 | >200 |
| Compound 26 | >200 |
| Compound 27 | >200 |
| Compound 28 | >200 |
| Compound 29 | >200 |
| Compound 30 | >200 |
| Compound 31 | >200 |
| Compound 38 | >200 |
| Compound 43 | >200 |
| Compound 51 | >200 |
| Compound 61 | >200 |
| Compound 62 | >200 |
| Compound 63 | >200 |
| Compound 64 | >200 |
| Compound 65 | >200 |
| Compound 66 | >200 |
| Compound 67 | >200 |
| Compound 68 | >200 |
| Compound 69 | >200 |
| Compound 71 | >200 |
| Compound 72 | >200 |
| Compound 73 | >200 |
| Compound 74 | >200 |
| Compound 75 | >200 |
| Compound 76 | >200 |
| Compound 77 | >200 |
| Compound 108 | >200 |
| Compound 110 | >200 |
| Compound 112 | >200 |
| Compound 113 | >200 |
| Compound 132 | >200 |
| Compound 133 | >200 |

(continued)

| Test compound | Solubility (mg/mL) |
|---|---|
| Compound 134 | >200 |

As can be seen from the above table, the compounds in the examples of the present invention had better solubility in an oily medium benzyl benzoate than compound 1-a, and were more suitable for being prepared into long-acting formulations due to their good lipid solubility.

9) Pharmacokinetic Experiment in Rats

The pharmacokinetic experiment in rats was performed on compounds 2, 3, 5, 6, 7, and 8 (non-fasting SD male rats, administered intramuscularly, at a dose of 43.3 mmol/kg, n = 3). The vehicle was benzyl benzoate, the injection site was the thigh muscle of the hind limb, and about 0.3 mL of blood samples were collected from the jugular vein of rats at blood collection time points of day 0.25, day 2, day 4, day 6, day 8, day 15, day 22, day 29, and day 36. The collected blood was stored under ice-cold conditions, plasma was rapidly isolated by centrifugation and the precipitant was added, and the concentration of compound 1 (active metabolite) was quantitatively determined by LCMS. The graphs of time-active metabolite concentration curves after administration of the compounds are shown in FIGs. 1 and 2.

The experimental results showed that after administration by intramuscular injection, the compounds (except for compound 1) in the examples of the present invention could slowly release the active metabolite (compound 1) in rats, so that the fluctuation of plasma concentration was reduced, and thus the safety and the tolerability of the drug were improved. In particular, there was minimal fluctuation in the plasma concentration of the active metabolite after administration of compound 3 and compound 8.

## Claims

**1.** A N-substituted quinolinone compound represented by general formula (I), or a pharmaceutically acceptable salt, a solvate, stereoisomer, a geometric isomer, an isotopically labeled compound or a prodrug thereof:

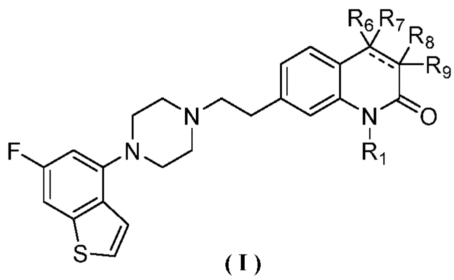

(I)

wherein:

$R_6$, $R_7$, $R_8$, and $R_9$ are each independently hydrogen, deuterium, halogen, methyl, ethyl, hydroxy, amino, or acetylamino;
------ represents a single bond or a double bond; when ------ is a double bond, one of $R_6$ and $R_7$ is absent, and one of $R_8$ and $R_9$ is absent;
$R_1$ is $-Y_1$-O-C(=O)-$R_2$, $-Y_1$-O-C(=O)-O-$R_3$, $-Y_1$-O-$R_4$, or -C(=O)-$R_5$;
each $Y_1$ is independently C1-C6 alkylene;
$R_2$ is C1-C30 alkyl, halogenated C1-C30 alkyl, C2-C30 alkenyl, amino C1-C30 alkyl, amino substituted with C1-C30 alkyl, amino substituted with C2-C30 alkenyl, amino substituted with halogenated C1-C30 alkyl, amino substituted with phenyl C1-C30 alkyl, amino substituted with hydroxy C1-C30 alkyl, amino substituted with C3-C10 cycloalkyl, amino substituted with C3-C10 cycloalkyl C1-C6 alkyl, C3-C10 cycloalkyl, 5- to 10-membered heterocyclyl, C6-C12 aryl, C6-C12 aryl C1-C30 alkyl, C1-C30 alkoxycarbonyl C1-C30 alkyl, C1-C30 alkoxy C1-C30 alkyl, C1-C30 alkoxy C1-C30 alkoxy C1-C30 alkyl,
or C1-C30 alkoxy C1-C30 alkoxy C1-C30 alkoxy C1-C30 alkyl;
$R_3$ is C1-C30 alkyl, halogenated C1-C30 alkyl, phenyl, phenyl C1-C30 alkyl, or C3-C10 cycloalkyl;
$R_4$ is hydrogen, C1-C30 alkyl, halogenated C1-C30 alkyl, phenyl C1-C30 alkyl, or phenyl C1-C30 alkoxy C1-C30

alkyl;
$R_5$ is C1-C30 alkyl, C2-C30 alkenyl, morpholinyl, phenyl, phenyl C1-C30 alkyl, hydroxy C1-C30 alkyl, C3-C10 cycloalkyl, C1-C30 alkoxy, phenyloxy, phenyl C1-C30 alkoxy, C1-C30 alkoxy C1-C30 alkoxy, amino C1-C30 alkyl, amino substituted with C1-C30 alkyl, amino substituted with hydroxy C1-C30 alkyl, amino substituted with phenyl C1-C30 alkyl, C3-C10 cycloalkoxy, C1-C30 alkanoyloxy C1-C6 alkoxy, C1-C30 alkoxy C1-C30 alkyl, C1-C30 alkoxy C1-C30 alkoxy C1-C30 alkyl, or C1-C30 alkoxy C1-C30 alkoxy C1-C30 alkoxy C1-C30 alkyl.

**2.** The N-substituted quinolinone compound, or the pharmaceutically acceptable salt, the solvate, the stereoisomer, the geometric isomer, the isotopically labeled compound or the prodrug thereof according to claim 1, wherein in general formula (I),

$R_1$ is $-Y_1$-O-C(=O)-$R_2$, $-Y_1$-O-C(=O)-O-$R_3$, $-Y_1$-O-$R_4$, or -C(=O)-Rs, preferably $-Y_1$-O-C(=O)-$R_2$;
each $Y_1$ is independently C1-C4 alkylene, preferably $-CH_2-$ or $-CH(CH_3)-$; and/or,
$R_2$ is C1-C30 alkyl, halogenated C1-C30 alkyl, C2-C30 alkenyl, amino C1-C16 alkyl, amino substituted with C1-C16 alkyl, amino substituted with C2-C16 alkenyl, amino substituted with halogenated C1-C16 alkyl, amino substituted with phenyl C1-C16 alkyl, amino substituted with hydroxy C1-C16 alkyl, amino substituted with C3-C10 cycloalkyl, amino substituted with C3-C10 cycloalkyl C1-C6 alkyl, C3-C10 cycloalkyl, 5- to 10-membered heterocyclyl, C6-C12 aryl, C6-C12 aryl C1-C16 alkyl, C1-C16 alkoxycarbonyl C1-C16 alkyl, C1-C16 alkoxy C1-C16 alkyl, C1-C16 alkoxy C1-C16 alkoxy C1-C16 alkyl, or C1-C16 alkoxy C1-C16 alkoxy C1-C16 alkoxy C1-C16 alkyl; preferably, $R_2$ is C1-C30 alkyl, halogenated C1-C30 alkyl, C2-C30 alkenyl, amino C1-C12 alkyl, amino substituted with C1-C12 alkyl, amino substituted with C2-C12 alkenyl, amino substituted with halogenated C1-C12 alkyl, amino substituted with phenyl C1-C12 alkyl, amino substituted with hydroxy C1-C12 alkyl, amino substituted with C3-C7 cycloalkyl, amino substituted with C3-C7 cycloalkyl C1-C4 alkyl, C3-C7 cycloalkyl, 5- to 10-membered heterocyclyl, C6-C12 aryl, C6-C12 aryl C1-C12 alkyl, C1-C12 alkoxycarbonyl C1-C12 alkyl, C1-C12 alkoxy C1-C12 alkyl, C1-C12 alkoxy C1-C12 alkoxy C1-C12 alkyl, or C1-C12 alkoxy C1-C12 alkoxy C1-C12 alkoxy C1-C12 alkyl; preferably, $R_2$ is C1-C30 alkyl (e.g., C1-C30 linear alkyl, isopropyl, tert-butyl, 1-methylpropyl, 3-methylbutyl, 1-methylbutyl, 1,1-dimethylpentyl, 1-methylpentyl, 1-methylhexyl, 1-pentylhexyl, etc.), halogenated C1-C30 alkyl (e.g., halogenated C1-C16 alkyl, halogenated C1-C12 alkyl, or halogenated C1-C6 alkyl, further, e.g., 3,3,3-trifluoropropyl), C2-C30 alkenyl (e.g., C2-C20 alkenyl), amino C1-C6 alkyl (e.g., $NH_2$-methyl), amino substituted with C1-C12 alkyl (e.g., decyl-NH- or heptanyl-NH-), amino substituted with C2-C6 alkenyl (e.g., 2-allyl-NH-), amino substituted with halogenated C1-C6 alkyl (e.g., 3,3,3-trifluoropropyl-NH-), amino substituted with phenyl C1-C6 alkyl (e.g., benzyl-NH-), amino substituted with hydroxy C1-C6 alkyl (e.g., hydroxyethyl-NH-), amino substituted with C3-C7 cycloalkyl (e.g., C3-C7 cycloalkyl-NH-), amino substituted with C3-C7 cycloalkyl C1-C6 alkyl (e.g., C3-C7 cycloalkylmethyl-NH-), C3-C7 cycloalkyl, 5- to 10-membered heterocyclyl (e.g., pyridinyl, piperidinyl, 4-tetrahydropyranyl, morpholinyl, furanyl, thienyl, pyrimidinyl, quinolyl, etc.), phenyl, naphthyl, phenyl C1-C6 alkyl (e.g., benzyl), C1-C6 alkoxycarbonyl C1-C6 alkyl (e.g., *tert*-butoxycarbonylmethyl), C1-C6 alkoxy C1-C6 alkyl (e.g., methoxymethyl), C1-C6 alkoxy C1-C6 alkoxy C1-C6 alkyl (e.g., methoxyethoxymethyl or butoxyethoxymethyl), or C1-C6 alkoxy C1-C6 alkoxy C1-C6 alkoxy C1-C6 alkyl (e.g., methoxyethoxyethoxymethyl); and/or,
$R_3$ is C1-C16 alkyl, halogenated C1-C16 alkyl, phenyl, phenyl C1-C16 alkyl, or C3-C10 cycloalkyl; preferably, $R_3$ is C1-C12 alkyl, halogenated C1-C12 alkyl, phenyl, phenyl C1-C12 alkyl, or C3-C7 cycloalkyl (e.g., cyclohexyl); more preferably, $R_3$ is C1-C6 alkyl, halogenated C1-C6 alkyl (e.g., 2,2,2-trifluoroethyl), phenyl, phenyl C1-C6 alkyl (e.g., benzyl), or C3-7 cycloalkyl (e.g., cyclohexyl); and/or, $R_4$ is hydrogen, C1-C16 alkyl, halogenated C1-C16 alkyl, phenyl C1-C16 alkyl, or phenyl C1-C16 alkoxy C1-C16 alkyl; preferably, $R_4$ is hydrogen, C1-C12 alkyl, halogenated C1-C12 alkyl, phenyl C1-C12 alkyl, or phenyl C1-C12 alkoxy C1-C12 alkyl; more preferably, $R_4$ is hydrogen, C1-C6 alkyl (e.g., isopropyl), halogenated C1-C6 alkyl (e.g., 2,2,2-trifluoroethyl), phenyl C1-C6 alkyl (e.g., benzyl), or phenyl C1-C6 alkoxy C1-C6 alkyl (e.g., benzyloxymethyl); and/or,
$R_5$ is C1-C16 alkyl, C2-C16 alkenyl, morpholinyl, phenyl, phenyl C1-C16 alkyl, hydroxy C1-C16 alkyl, C3-C10 cycloalkyl, C1-C16 alkoxy, phenyloxy, phenyl C1-C16 alkoxy, C1-C16 alkoxy C1-C16 alkoxy, amino C1-C16 alkyl, amino substituted with C1-C16 alkyl, amino substituted with hydroxy C1-C16 alkyl, amino substituted with phenyl C1-C16 alkyl, C3-C10 cycloalkoxy, C1-C16 alkanoyloxy C1-C6 alkoxy, C1-C16 alkoxy C1-C16 alkyl, C1-C16 alkoxy C1-C16 alkoxy C1-C16 alkyl, or C1-C16 alkoxy C1-C16 alkoxy C1-C16 alkoxy C1-C16 alkyl; preferably, $R_5$ is C1-C12 alkyl, C2-C12 alkenyl, morpholinyl, phenyl, phenyl C1-C12 alkyl, hydroxy C1-C12 alkyl, C3-C10 cycloalkyl, C1-C12 alkoxy, phenyloxy, phenyl C1-C12 alkoxy, C1-C12 alkoxy C1-C12 alkoxy, amino C1-C12 alkyl, amino substituted with C1-C12 alkyl, amino substituted with hydroxy C1-C12 alkyl, amino substituted with phenyl C1-C12 alkyl, C3-C10 cycloalkyl, C1-C12 alkanoyloxy C1-C6 alkoxy, C1-C12 alkoxy C1-C12 alkyl, C1-C12 alkoxy C1-C12 alkoxy C1-C12 alkyl, or C1-C12 alkoxy C1-C12 alkoxy C1-C12 alkoxy C1-C12 alkyl; more preferably, $R_5$ is C1-C6 alkyl (e.g., methyl, ethyl, propyl, etc.), C2-C16 alkenyl (e.g., decenyl),

morpholinyl, phenyl, phenyl C1-C6 alkyl, hydroxy C1-C6 alkyl, C3-C10 cycloalkyl (e.g., cyclopentyl or cyclohexyl), C1-C6 alkoxy (e.g., methoxy, ethoxy, propoxy, isopropoxy, etc.), phenoxy, phenyl C1-C6 alkoxy, C1-C6 alkoxy C1-C6 alkoxy (e.g., methoxyethoxy), amino C1-C6 alkyl ($NH_2$-methyl), amino substituted with C1-C6 alkyl (e.g., propylamino), amino substituted with hydroxy C1-C6 alkyl (e.g., hydroxyethyl-NH-), amino substituted with phenyl C1-C6 alkyl (e.g., benzyl-NH-), C1-C16 alkanoyloxy C1-C6 alkoxy (decanoyloxymethyoxy), C1-C6 alkoxy C1-C6 alkyl (e.g., methoxymethyl), C1-C6 alkoxy C1-C6 alkoxy C1-C6 alkyl (e.g., methoxyethoxymethyl or methoxyethoxymethyl), or C1-C6 alkoxy C1-C6 alkoxy C1-C6 alkoxy C1-C6 alkyl (e.g., methoxyethoxyethoxymethyl).

**3.** The N-substituted quinolinone compound, or the pharmaceutically acceptable salt, the solvate, the stereoisomer, the geometric isomer, the isotopically labeled compound or the prodrug thereof according to claim 1, wherein in general formula (I),

$R_6$, $R_7$, $R_8$, and $R_9$ are each independently hydrogen, deuterium, halogen, methyl, ethyl, hydroxy, amino, or acetylamino;
- - - - - - represents a single bond or a double bond; when - - - - - - is a double bond, one of $R_6$ and $R_7$ is absent, and one of $R_8$ and $R_9$ is absent;
$R_1$ is $-Y_1-O-C(=O)-R_2$, $-Y_1-O-C(=O)-O-R_3$, $-Y_1-O-R_4$, or $-C(=O)-R_5$;
$Y_1$ is C1-C6 alkylene;
$R_2$ is C1-C16 alkyl, halogenated C1-C16 alkyl, C2-C16 alkenyl, amino C1-C16 alkyl, amino substituted with C1-C16 alkyl, amino substituted with C2-C16 alkenyl, amino substituted with halogenated C1-C16 alkyl, amino substituted with phenyl C1-C16 alkyl, amino substituted with hydroxy C1-C16 alkyl, amino substituted with C3-C10 cycloalkyl, amino substituted with C3-C10 cycloalkyl C1-C6 alkyl, C3-C10 cycloalkyl, 5- to 10-membered heterocyclyl, C6-C12 aryl, C6-C12 aryl C1-C16 alkyl, C1-C16 alkoxycarbonyl C1-C16 alkyl, C1-C16 alkoxy C1-C16 alkyl, C1-C16 alkoxy C1-C16 alkoxy C1-C16 alkyl, or C1-C16 alkoxy C1-C16 alkoxy C1-C16 alkoxy C1-C16 alkyl;
$R_3$ is C1-C16 alkyl, halogenated C1-C16 alkyl, phenyl, phenyl C1-C16 alkyl, or C3-C10 cycloalkyl;
$R_4$ is hydrogen, C1-C16 alkyl, halogenated C1-C16 alkyl, phenyl C1-C16 alkyl, or phenyl C1-C16 alkoxy C1-C16 alkyl;
$R_5$ is C1-C16 alkyl, C2-C16 alkenyl, morpholinyl, phenyl, phenyl C1-C16 alkyl, hydroxy C1-C16 alkyl, C3-C10 cycloalkyl, C1-C16 alkoxy, phenyl C1-C16 alkoxy, C1-C16 alkoxy C1-C16 alkoxy, amino C1-C16 alkyl, amino substituted with C1-C16 alkyl, amino substituted with hydroxy C1-C16 alkyl, amino substituted with phenyl C1-C16 alkyl, C3-C10 cycloalkoxy, C1-C16 alkoxy C1-C16 alkyl, C1-C16 alkoxy C1-C16 alkoxy C1-C16 alkyl, or C1-C16 alkoxy C1-C16 alkoxy C1-C16 alkoxy C1-C16 alkyl.

**4.** The N-substituted quinolinone compound, or the pharmaceutically acceptable salt, the solvate, the stereoisomer, the geometric isomer, the isotopically labeled compound or the prodrug thereof according to any one of claims 1-3, being represented by general formula (1-1) or (1-2) as follows:

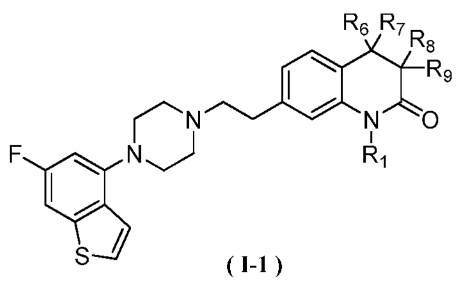

(I-1)

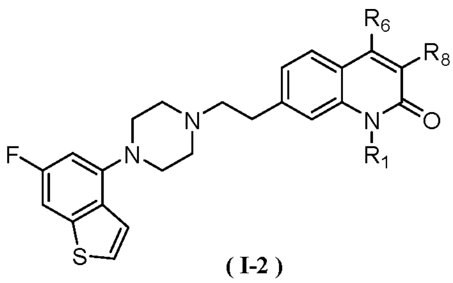

(I-2)

wherein, $R_1$, $R_6$, $R_7$, $R_8$, and $R_9$ have definitions as described in corresponding claims.

**5.** The N-substituted quinolinone compound, or the pharmaceutically acceptable salt, the solvate, the stereoisomer, the geometric isomer, the isotopically labeled compound or the prodrug thereof according to any one of claims 1-4, wherein,

$R_1$ is $-Y_1-O-C(=O)-R_2$ or $-Y_1-O-C(=O)-O-R_3$, preferably $-Y_1-O-C(=O)-R_2$;
$Y_1$ is C1-C4 alkylene, preferably $-CH_2-$ or $-CH(CH_3)-$;
$R_2$ is C1-C12 alkyl, halogenated C1-C12 alkyl, C2-C12 alkenyl, amino C1-C12 alkyl, amino substituted with C1-C12 alkyl, amino substituted with C2-C12 alkenyl, amino substituted with halogenated C1-C12 alkyl, amino substituted with phenyl C1-C12 alkyl, amino substituted with hydroxy C1-C12 alkyl, amino substituted with C3-C6 cycloalkyl, amino substituted with C3-C6 cycloalkyl C1-C4 alkyl, C3-C6 cycloalkyl, pyridinyl, piperidinyl, morpholinyl, furanyl, thienyl, pyrimidinyl, quinolyl, phenyl, phenyl C1-C12 alkyl, C1-C12 alkoxycarbonyl C1-C12 alkyl, C1-C12 alkoxy C1-C12 alkoxy C1-C12 alkyl, or C1-C12 alkoxy C1-C12 alkoxy C1-C12 alkoxy C1-C12 alkyl, preferably C1-C12 alkyl, halogenated C1-C12 alkyl, C3-C6 cycloalkyl, phenyl, phenyl C1-C12 alkyl, C1-C12 alkoxycarbonyl C1-C12 alkyl, C1-C12 alkoxy C1-C12 alkoxy C1-C12 alkyl, or C1-C12 alkoxy C1-C12 alkoxy C1-C12 alkoxy C1-C12 alkyl;

$R_3$ is C1-C12 alkyl, halogenated C1-C12 alkyl, phenyl, phenyl C1-C12 alkyl, or C3-C6 cycloalkyl, preferably C1-C12 alkyl, halogenated C1-C12 alkyl, phenyl, or C3-C6 cycloalkyl.

**6.** The N-substituted quinolinone compound, or the pharmaceutically acceptable salt, the solvate, the stereoisomer, the geometric isomer, the isotopically labeled compound or the prodrug thereof according to any one of claims 1-4, wherein,

$R_1$ is $-Y_1-O-C(=O)-R_2$;

$Y_1$ is C1-C4 alkylene, preferably $-CH_2-$ or $-CH(CH_3)-$;

$R_2$ is C3-C16 alkyl, halogenated C3-C16 alkyl, C3-C16 alkenyl, amino C3-C16 alkyl, amino substituted with C3-C16 alkyl, amino substituted with C3-C16 alkenyl, amino substituted with halogenated C3-C16 alkyl, amino substituted with phenyl C3-C16 alkyl, amino substituted with hydroxy C3-C16 alkyl, amino substituted with C3-C6 cycloalkyl, amino substituted with C3-C6 cycloalkyl C3-C16 alkyl, C3-C6 cycloalkyl, pyridinyl, piperidinyl, morpholinyl, furanyl, thienyl, pyrimidinyl, quinolyl, phenyl, phenyl C3-C16 alkyl, C1-C12 alkoxycarbonyl C3-C16 alkyl, C1-C12 alkoxy C1-C12 alkoxy C3-C16 alkyl, or C1-C12 alkoxy C1-C12 alkoxy C1-C12 alkoxy C3-C16 alkyl, preferably C3-C16 alkyl, halogenated C3-C16 alkyl, C3-C6 cycloalkyl, phenyl, phenyl C3-C16 alkyl, C1-C12 alkoxycarbonyl C3-C16 alkyl, C1-C12 alkoxy C1-C12 alkoxy C3-C16 alkyl, or C1-C12 alkoxy C1-C12 alkoxy C1-C12 alkoxy C3-C16 alkyl.

**7.** The N-substituted quinolinone compound, or the pharmaceutically acceptable salt, the solvate, the stereoisomer, the geometric isomer, the isotopically labeled compound or the prodrug thereof according to any one of claims 1-4, wherein,

$R_1$ is $-Y_1-O-R_4$ or $-C(=O)-R_5$;

$R_4$ is hydrogen, C1-C12 alkyl, halogenated C1-C12 alkyl, phenyl C1-C12 alkyl, or phenyl C1-C12 alkoxy C1-C12 alkyl, preferably hydrogen, C1-C12 alkyl, halogenated C1-C12 alkyl, or phenyl C1-C12 alkyl;

$R_5$ is C1-C12 alkyl, C2-C12 alkenyl, morpholinyl, phenyl, phenyl C1-C12 alkyl, hydroxy C1-C12 alkyl, C3-C6 cycloalkyl, C1-C12 alkoxy, phenyl C1-C12 alkoxy, amino C1-C12 alkyl, amino substituted with C1-C12 alkyl, amino substituted with hydroxy C1-C12 alkyl, amino substituted with phenyl C1-C12 alkyl, C3-C6 cycloalkoxy, C1-C12 alkoxy C1-C12 alkoxy C1-C12 alkyl, or C1-C12 alkoxy C1-C12 alkoxy C1-C12 alkoxy C1-C12 alkyl, preferably C1-C12 alkyl, phenyl, phenyl C1-C12 alkyl, C3-C6 cycloalkyl, C1-C12 alkoxy, phenyl C1-C12 alkoxy, C3-C6 cycloalkoxy, C1-C12 alkoxy C1-C12 alkoxy C1-C12 alkyl, or C1-C12 alkoxy C1-C12 alkoxy C1-C12 alkoxy C1-C12 alkyl.

**8.** The N-substituted quinolinone compound, or the pharmaceutically acceptable salt, the solvate, the stereoisomer, the geometric isomer, the isotopically labeled compound or the prodrug thereof according to any one of claims 1-4, wherein,

$R_6$, $R_7$, $R_8$, and $R_9$ are each independently hydrogen;

$R_1$ is $-Y_1-O-C(=O)-R_2$, $-Y_1-O-C(=O)-O-R_3$, $-Y_1-O-R_4$, or $-C(=O)-R_5$;

$Y_1$ is C1-C4 alkylene, preferably $-CH_2-$ or $-CH(CH_3)-$;

$R_2$ is C1-C16 alkyl or phenyl;

$R_3$ is C1-C12 alkyl;

$R_4$ is hydrogen;

$R_5$ is C1-C6 alkyl or C1-C6 alkoxy.

**9.** The N-substituted quinolinone compound, or the pharmaceutically acceptable salt, the solvate, the stereoisomer, the geometric isomer, the isotopically labeled compound or the prodrug thereof according to any one of claims 1-4, wherein,

$R_6$, $R_7$, $R_8$, and $R_9$ are each independently hydrogen;
$R_1$ is -$Y_1$-O-C(=O)-$R_2$;
$Y_1$ is C1-C4 alkylene, preferably -$CH_2$- or -$CH(CH_3)$-;
$R_2$ is C1-C30 alkyl.

**10.** A N-substituted quinolinone compound, or a pharmaceutically acceptable salt, a solvate, a stereoisomer, a geometric isomer, an isotopically labeled compound or a prodrug thereof, wherein the compound is any one of:

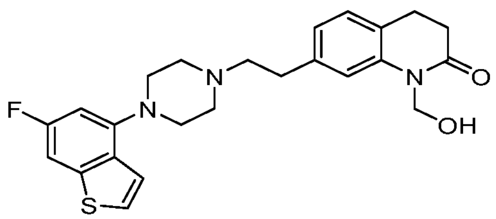 (1), 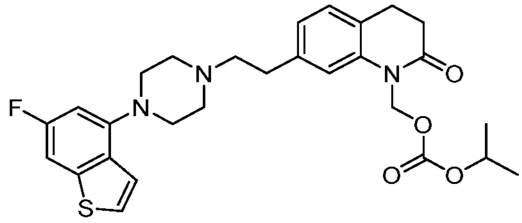 (2),

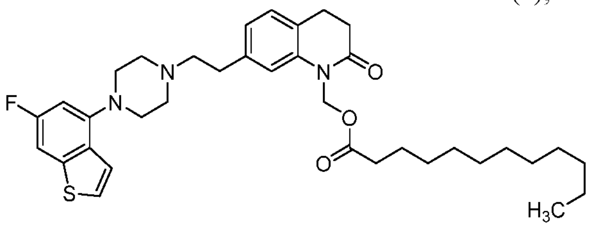 (3), 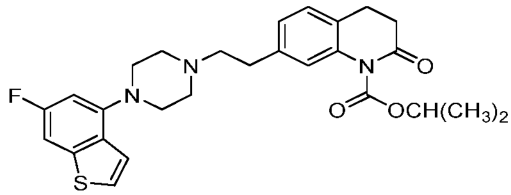 (4),

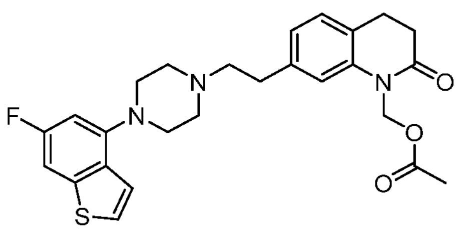 (5), 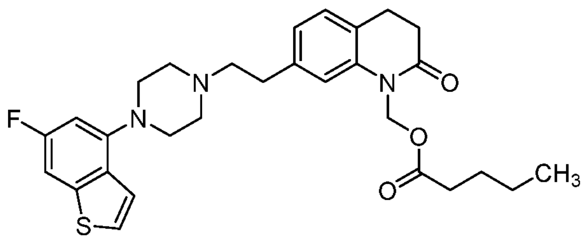 (6),

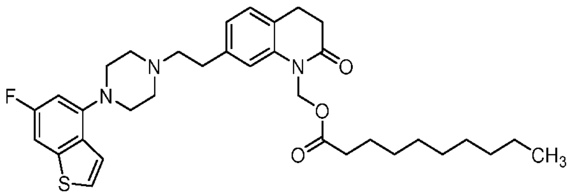 (7), 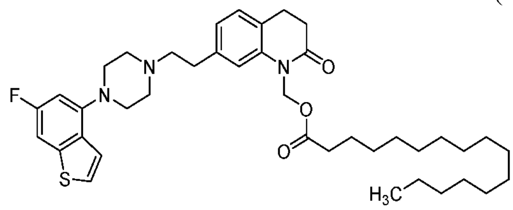 (8),

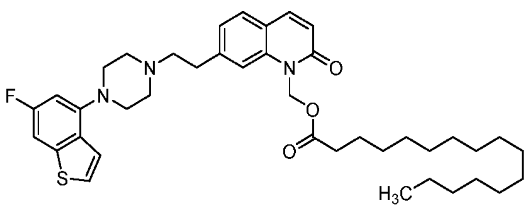 (9), 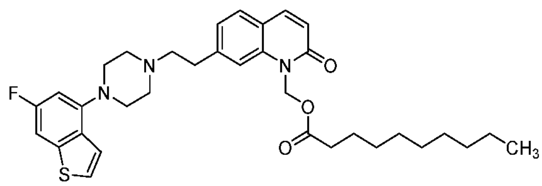 (10),

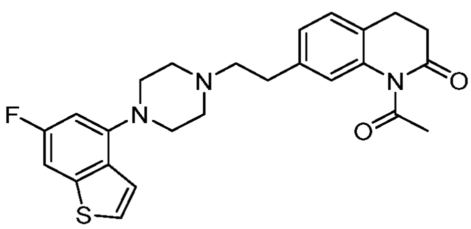 (11), 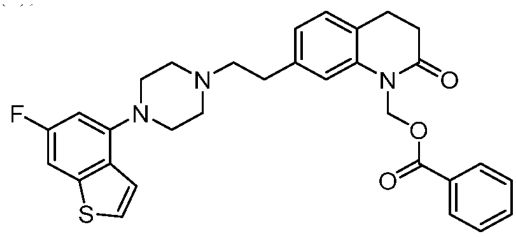 (12),

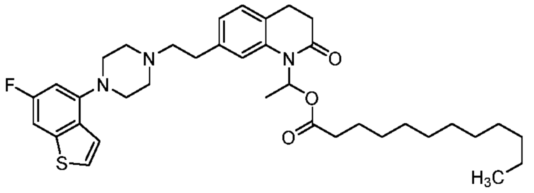 (13), 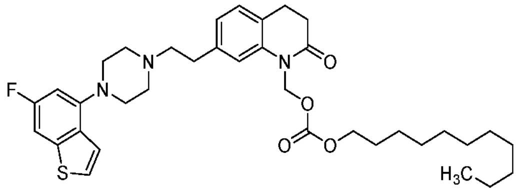 (14),

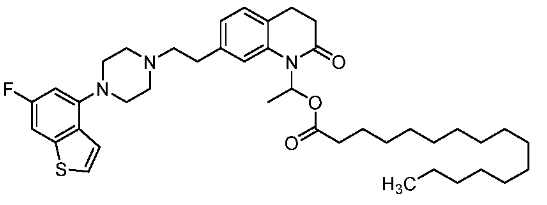 (15), 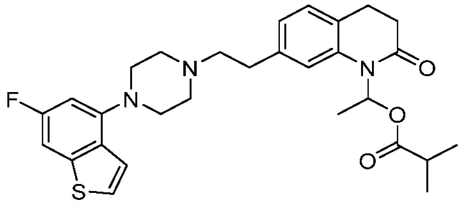 (16),

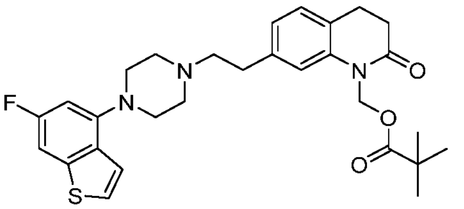 (17), 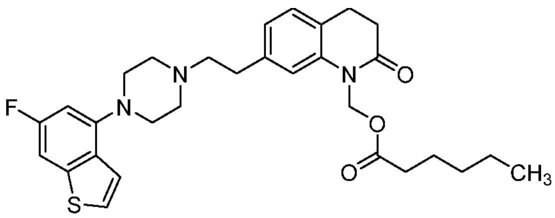 (18),

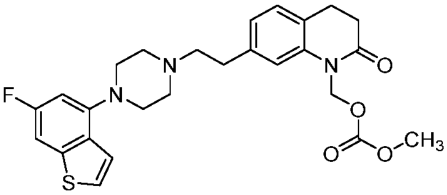 (19), 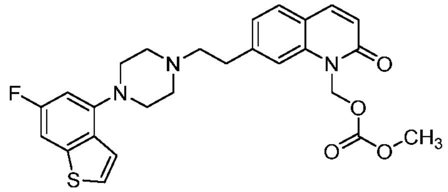 (20),

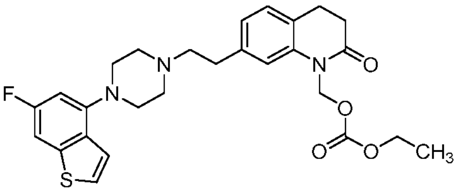 (21), 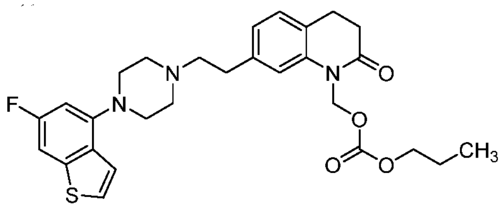 (22),

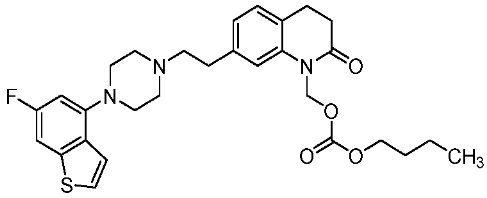 (23), 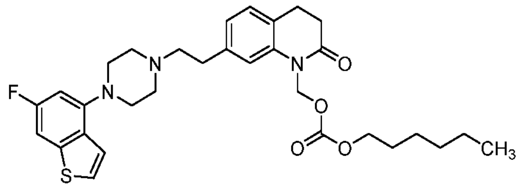 (24),

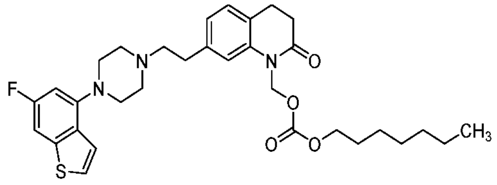 (25), 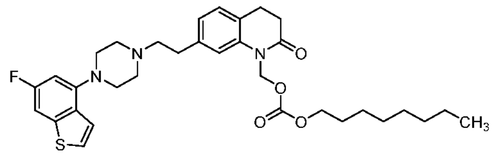 (26),

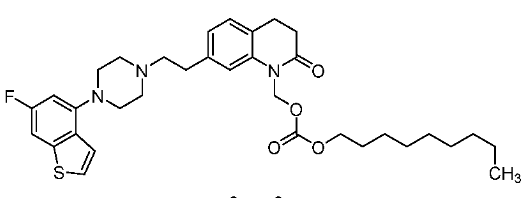 (27),

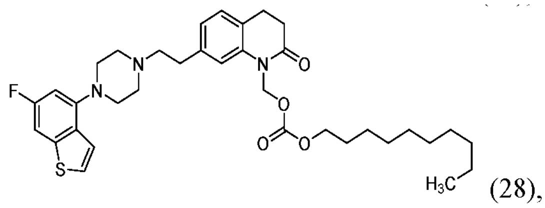 (28),

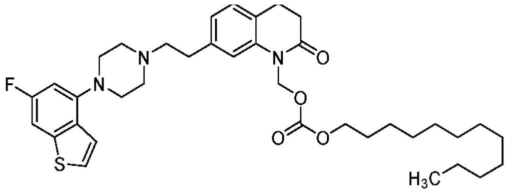 (29),

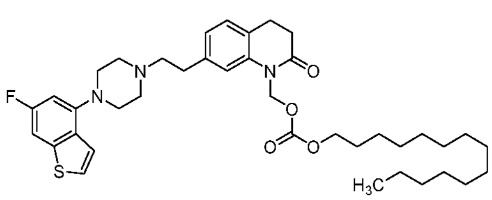 (30),

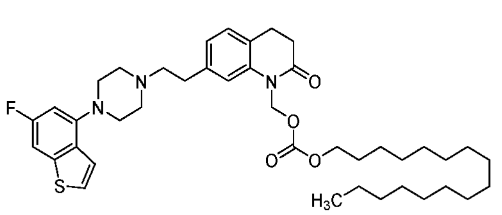 (31),

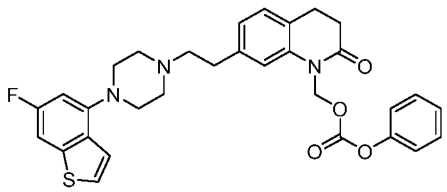 (32),

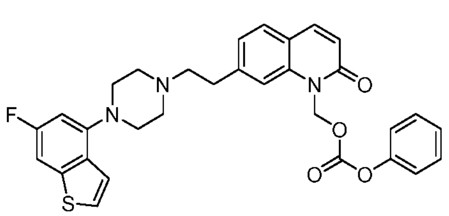 (33),

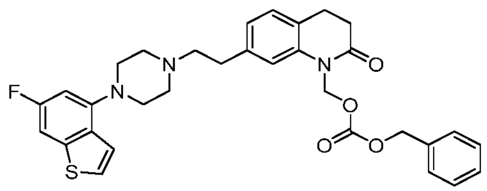 (34),

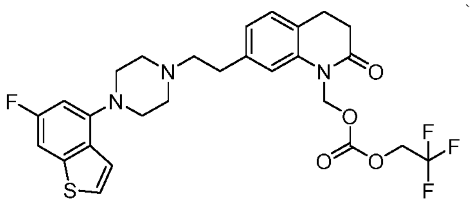 (35),

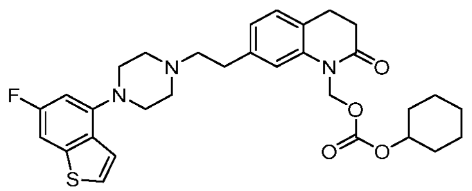 (36),

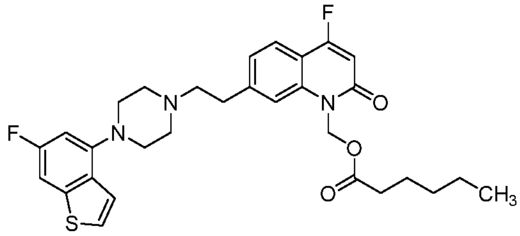 (37),

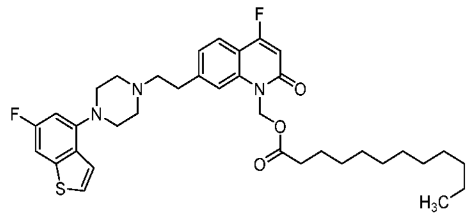 (38),

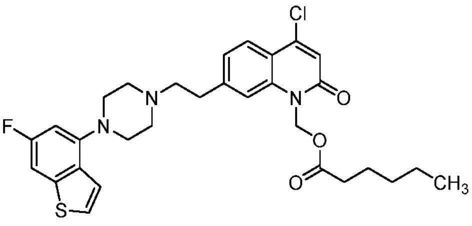 (39), 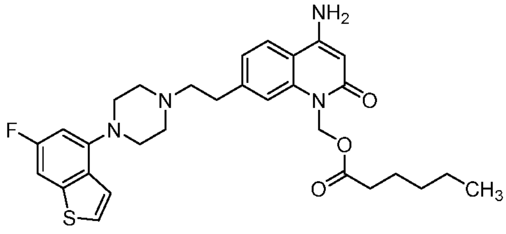 (40),

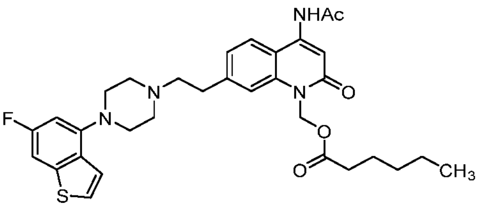 (41), 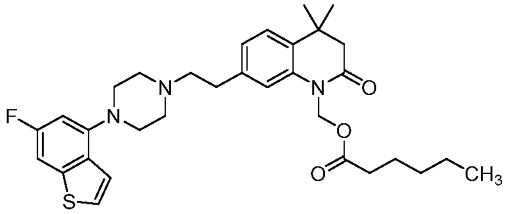 (42),

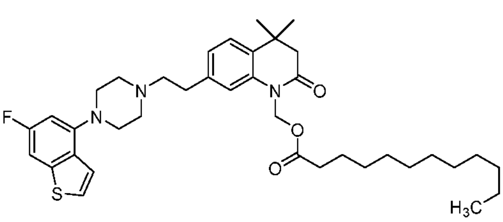 (43), 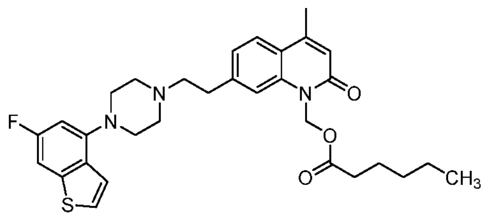 (44),

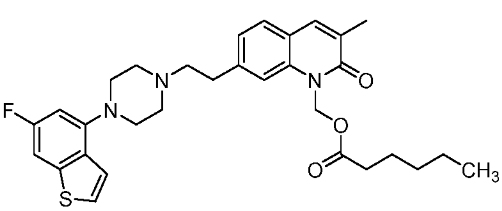 (45), 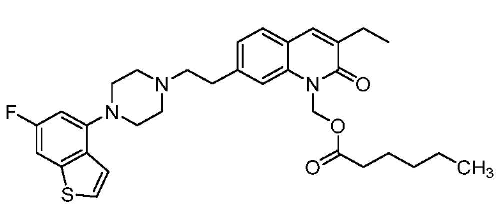 (46)

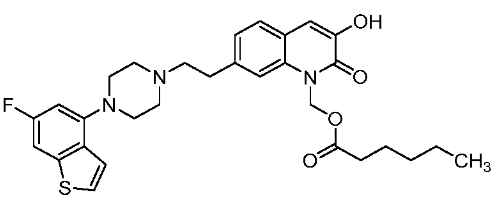 (47), 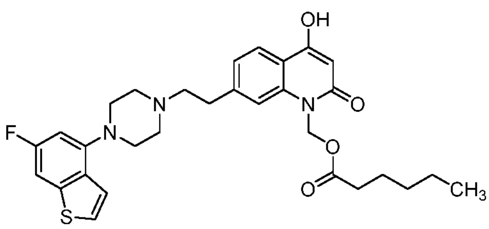 (48),

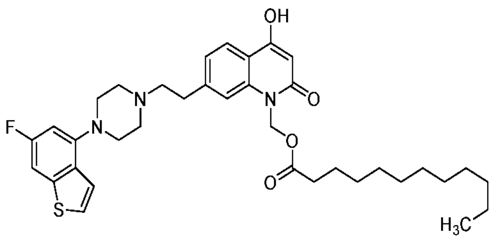 (49), 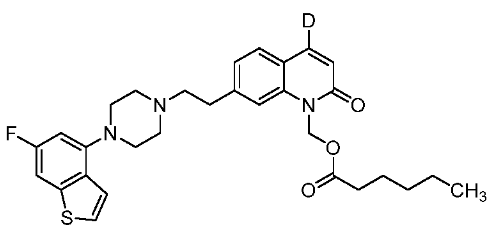 (50),

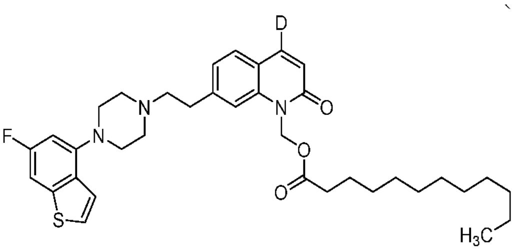 (51), 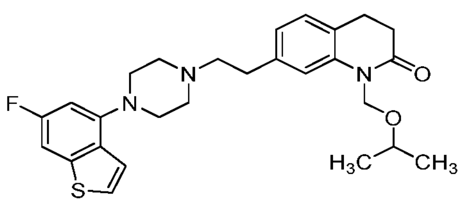 (52),

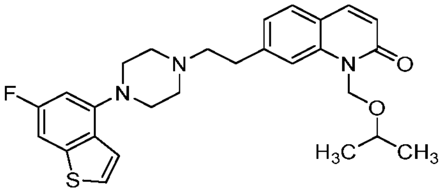 (53),

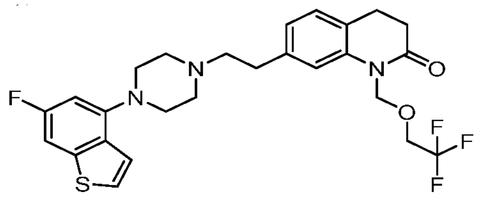 (54),

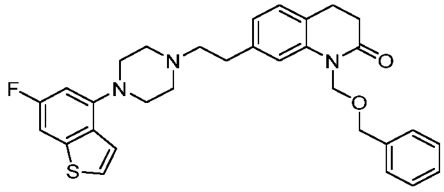 (55),

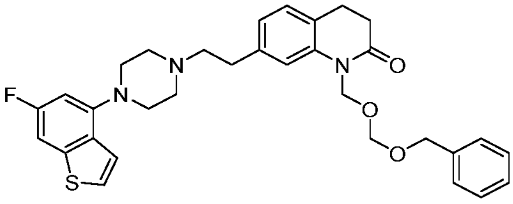 (56),

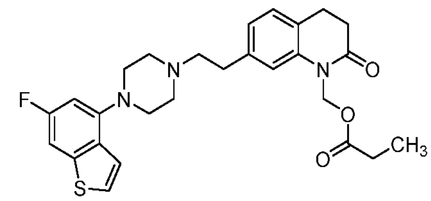 (57),

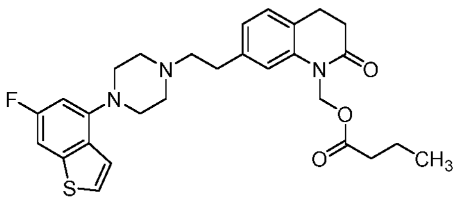 (58),

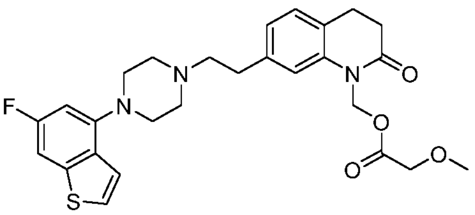 (59),

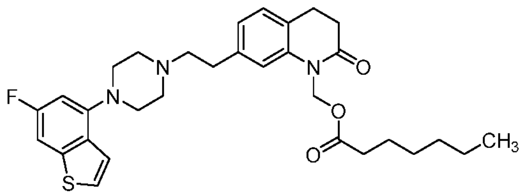 (60),

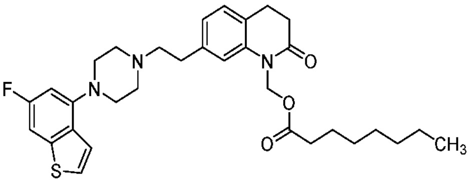 (61),

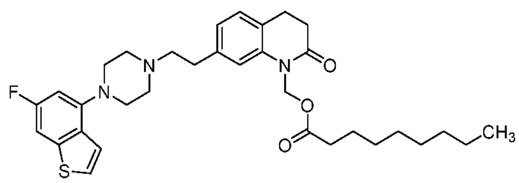 (62),

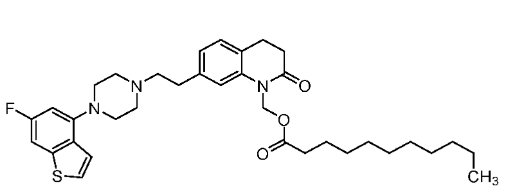 (63),

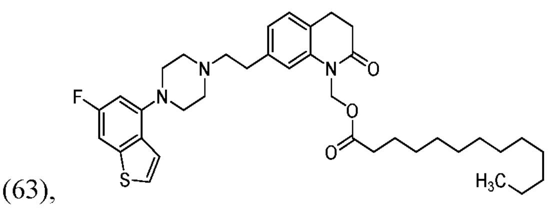 (64),

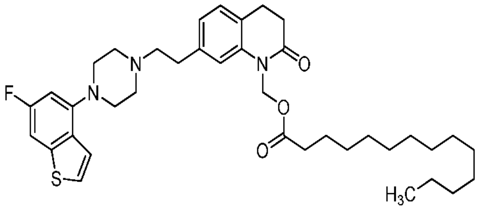 (65),

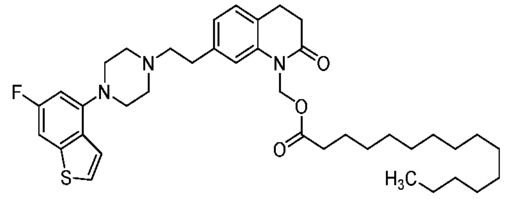 (66),

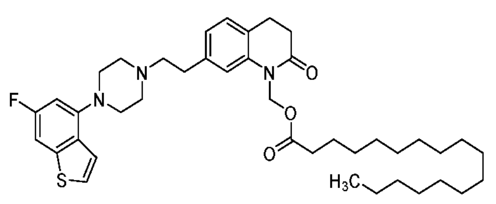 (67),

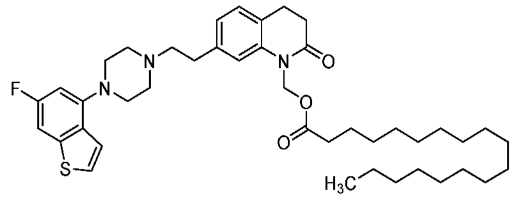 (68),

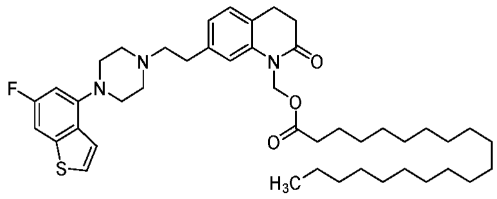 (69),

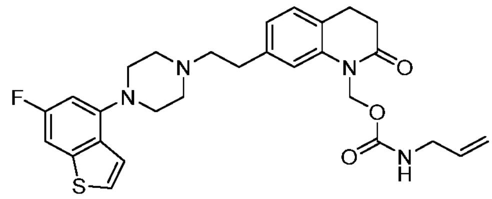 (70),

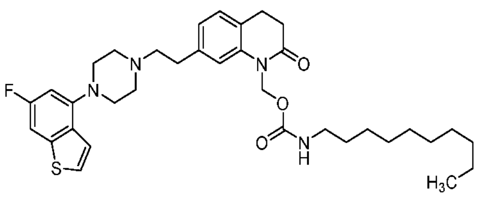 (71),

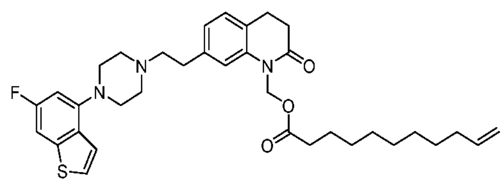 (72),

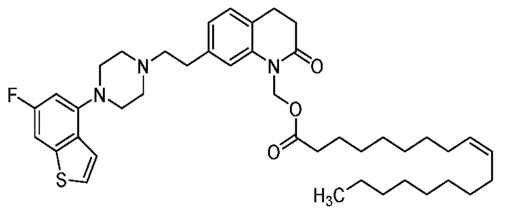 (73),

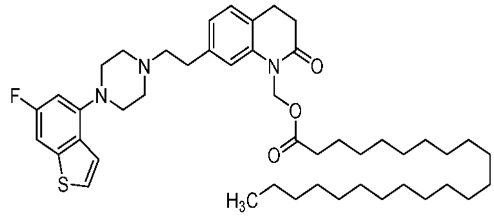 (74),

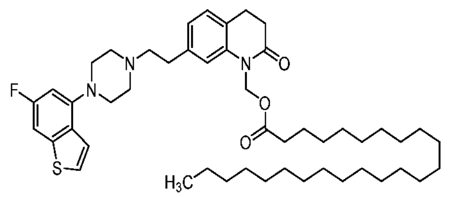 (75),

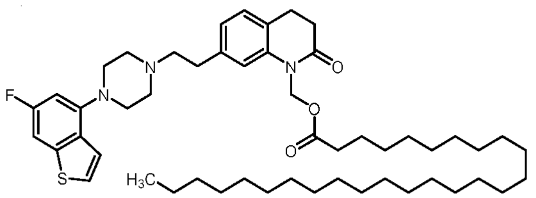 (76),

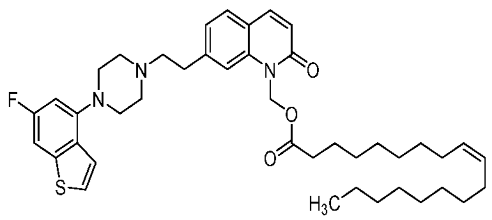 (77),

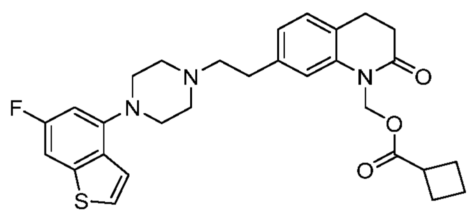 (78),

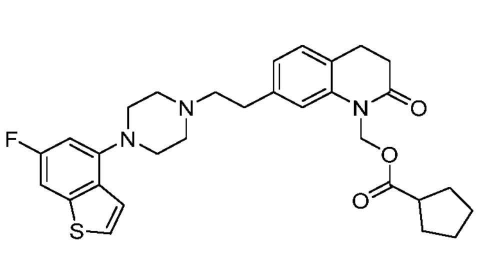 (79),

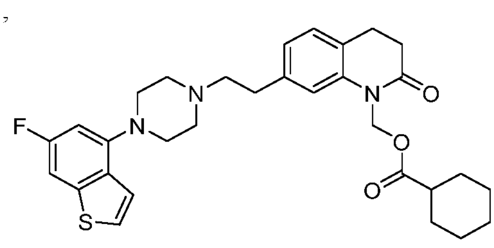 (80),

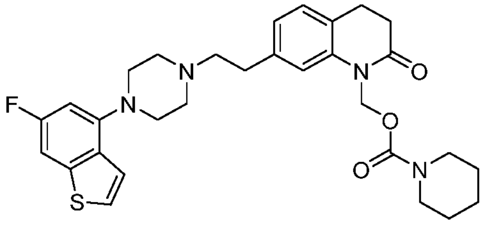 (81), 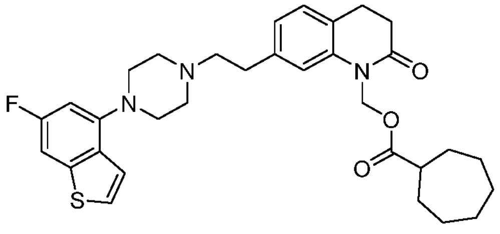 (82),

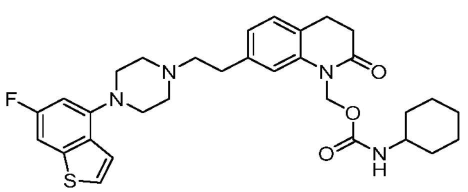 (83), 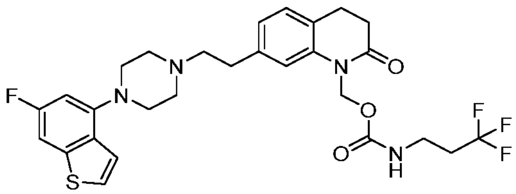 (84),

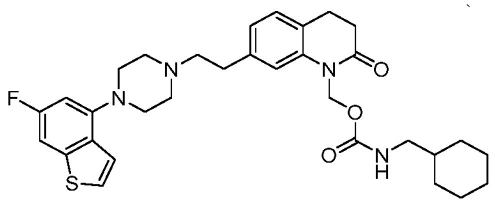 (85), 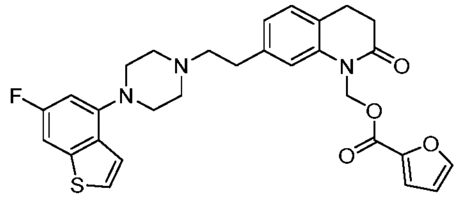 (86),

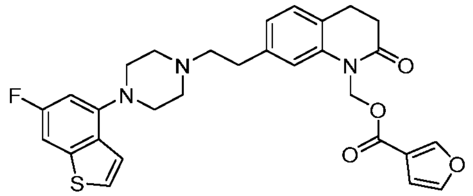 (87), 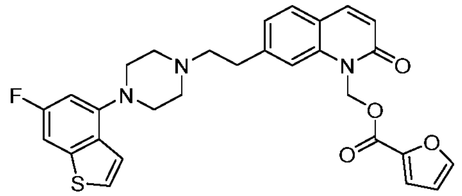 (88),

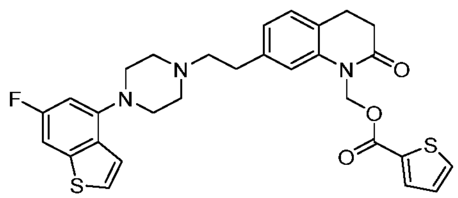 (89), 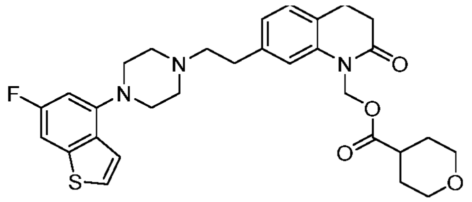 (90),

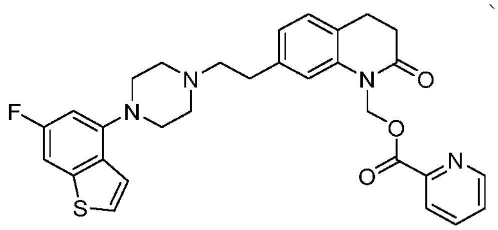 (91), 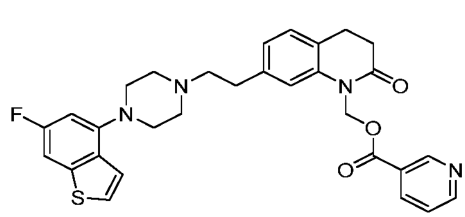 (92),

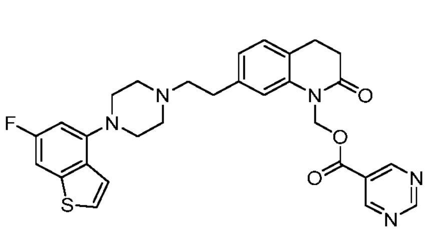 (93), 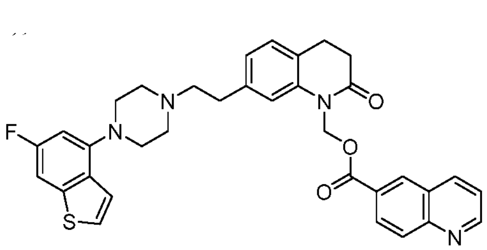 (94),

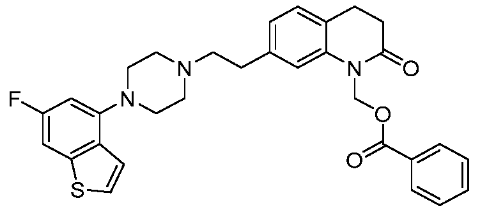 (95), 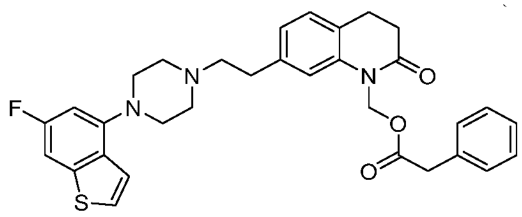 (96),

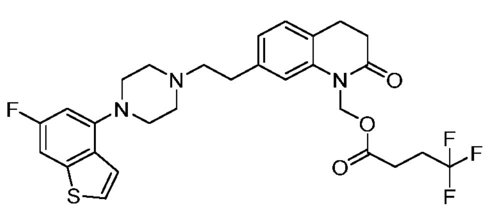 (97), 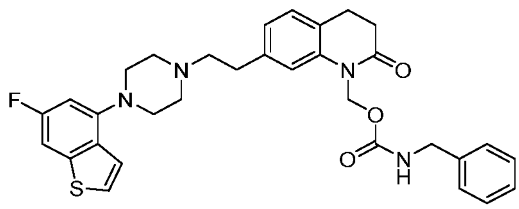 (98),

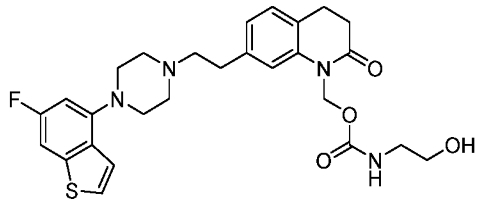 (99), 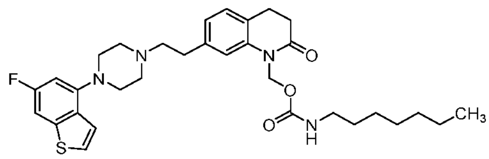 (100),

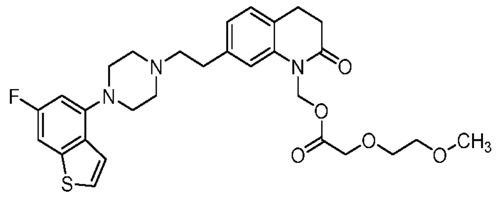 (101), 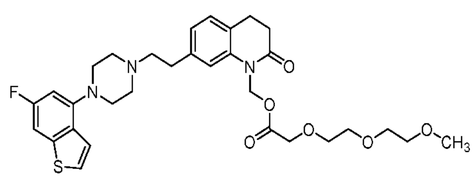 (102),

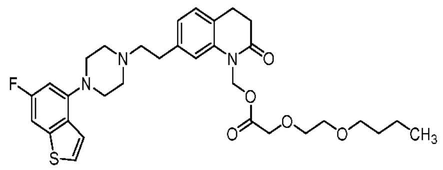 (103), 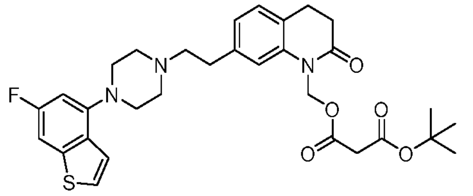 (104),

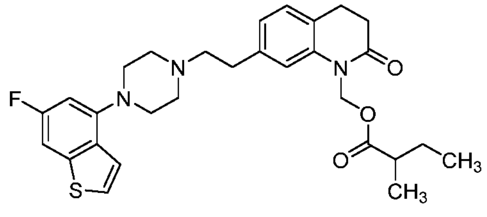 (105), 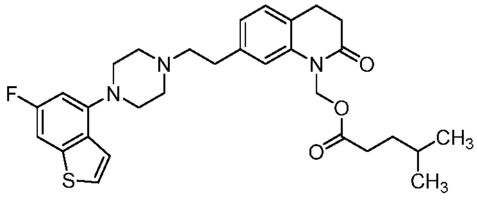 (106),

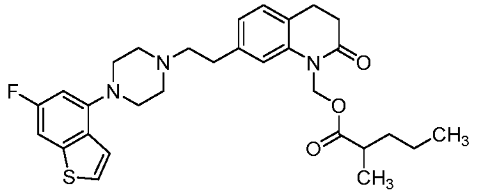 (107), 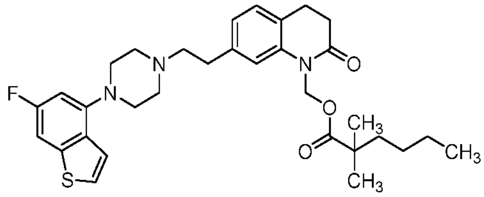 (108),

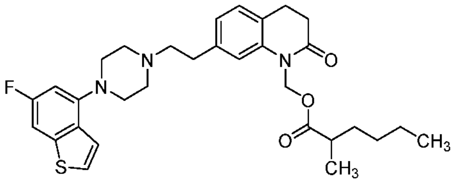 (109), 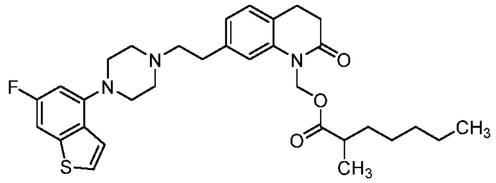 (110),

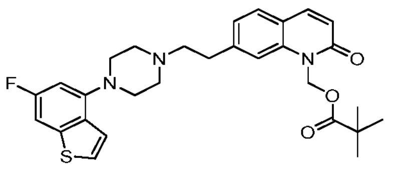 (111), 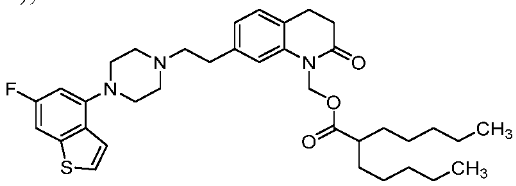 (112),

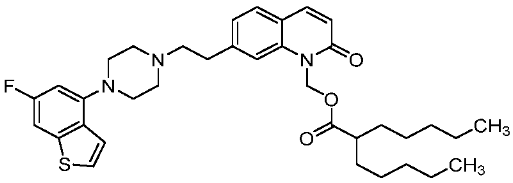 (113), 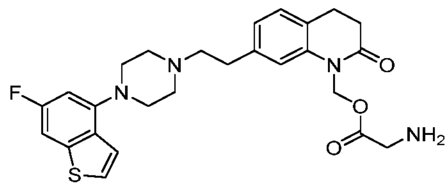 (114),

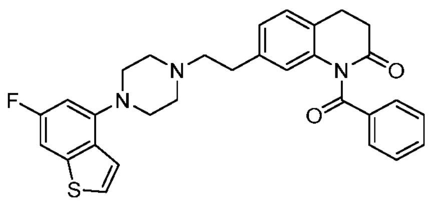 (115), 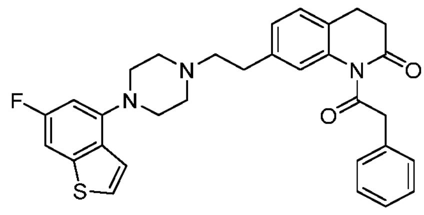 (116),

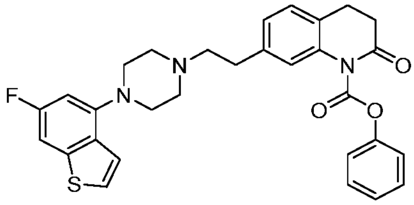 (117), 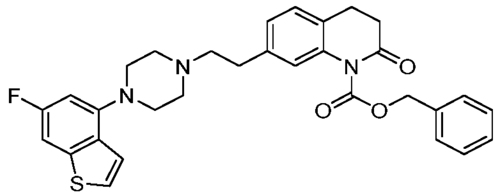 (118),

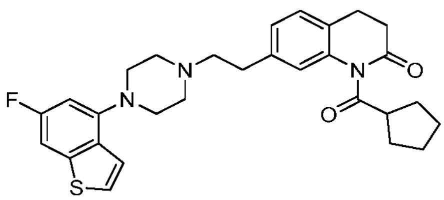 (119), 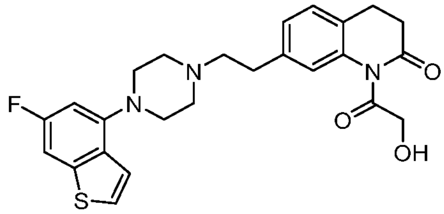 (120),

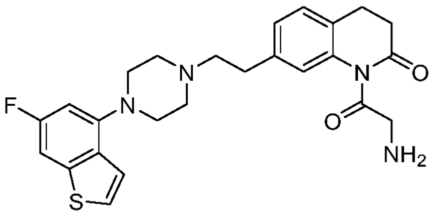 (121), 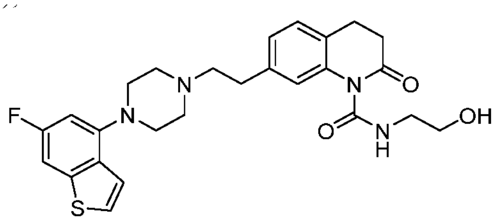 (122),

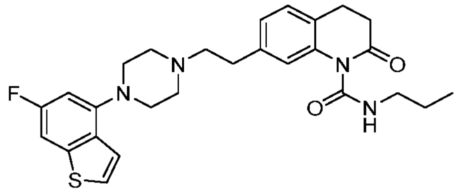
(123),
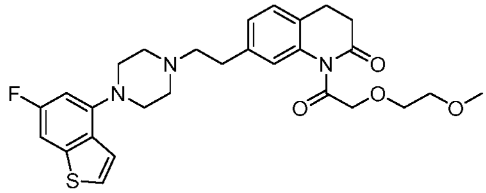
(124),

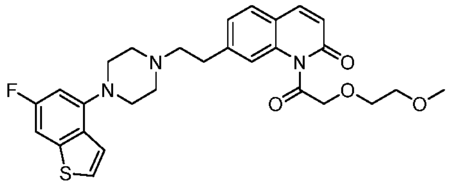
(125),
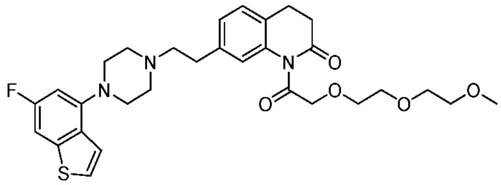
(126),

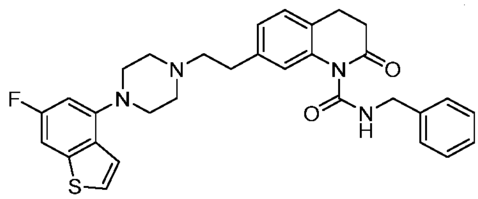
(127),
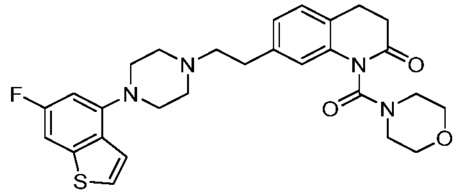
(128),

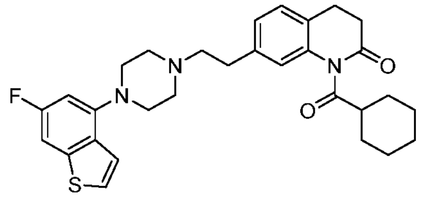
(129),
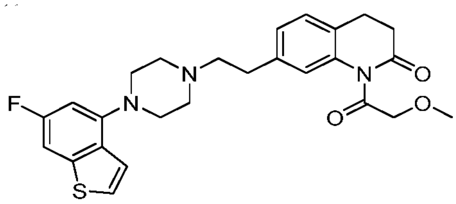
(130),

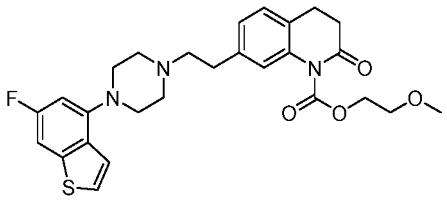
(131),
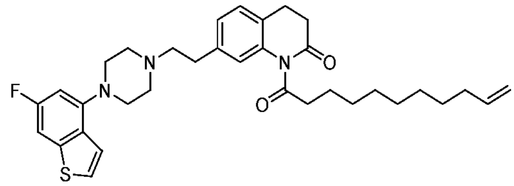
(132),

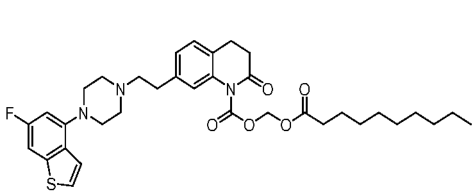
(133), or
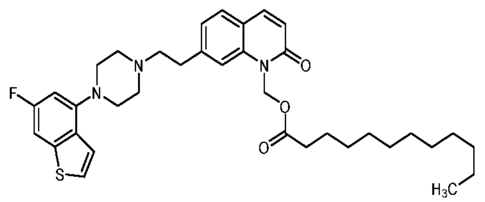
(134).

**11.** A method for preparing the N-substituted quinolinone compound, or the pharmaceutically acceptable salt, the solvate, the stereoisomer, the geometric isomer, the isotopically labeled compound or the prodrug thereof according to any one of claims 1-10, wherein the method is selected from methods 1-4:

method 1: subjecting a compound represented by formula (II) or a salt thereof to a reaction with a compound represented by formula (III) or a salt thereof,

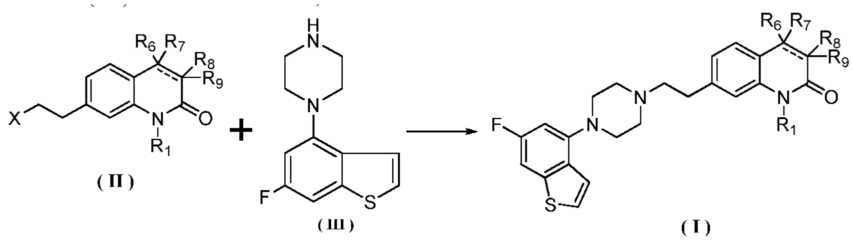

wherein, - - - - - -, $R_1$, $R_6$, $R_7$, $R_8$, and $R_9$ are as defined in any one of claims 1-10;
X is halogen, C1-C6 alkylsulfonyloxy, benzenesulfonyloxy, or naphthalenesulfonyloxy, the C1-C6 alkylsulfonyloxy, benzenesulfonyloxy, or naphthalenesulfonyloxy being optionally substituted with one or more groups selected from halogen, C1-C6 alkyl, C1-C6 alkoxy, nitro, hydroxy, amino, and C1-C6 alkanoyl;
the reaction is performed in the presence or absence of a solvent, preferably in a solvent with or without a base;
the reaction is performed at a temperature of room temperature to 200 °C for a period of 1-120 h;
method 2: subjecting a compound represented by formula (IV) or a salt thereof to a reaction with a compound represented by formula (V) or a salt thereof,

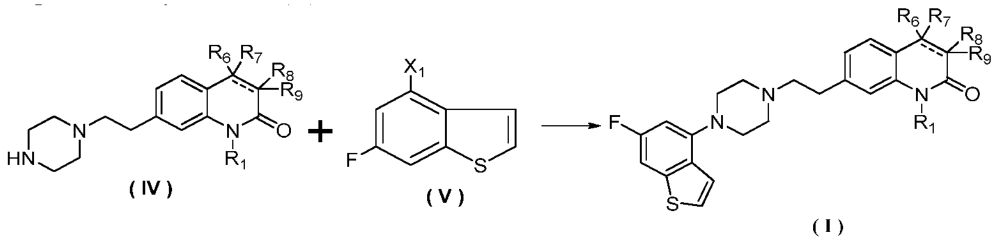

wherein, - - - - - -, $R_1$, $R_6$, $R_7$, $R_8$, and $R_9$ are as defined in any one of claims 1-10;
$X_1$ is halogen or trifluoromethanesulfonyloxy;
preferably, the reaction is performed in the presence of a palladium catalyst and a base;
the reaction is performed at a temperature of room temperature to 200 °C for a period of 1-30 h;
method 3: subjecting a compound represented by formula (I-a) or a salt thereof to a reaction with $R_1X$,

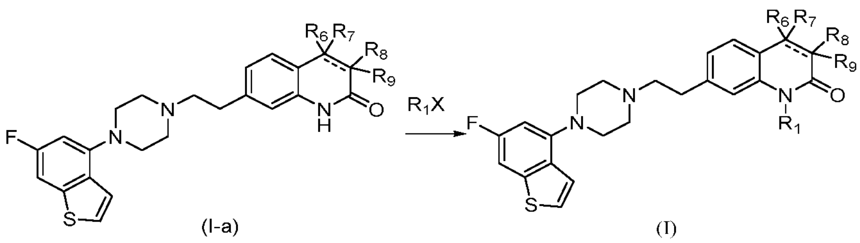


wherein, - - - - - -, $R_1$, $R_6$, $R_7$, $R_8$, and $R_9$ are as defined in any one of claims 1-10;
X is halogen, C1-C6 alkylsulfonyloxy, benzenesulfonyloxy, or naphthalenesulfonyloxy, the C1-C6 alkylsulfonyloxy, benzenesulfonyloxy, or naphthalenesulfonyloxy being optionally substituted with one or more groups selected from halogen, C1-C6 alkyl, C1-C6 alkoxy, nitro, hydroxy, amino, and C1-C6 alkanoyl;
the reaction is performed in the presence or absence of a solvent, preferably in a solvent with or without a base;
the reaction is performed at a temperature of room temperature to 200 °C for a period of 1-120 h;
method 4: subjecting a compound represented by formula (1-b) or a salt thereof to a substitution reaction with a compound represented by X-C(=O)-$R_2$ or X-O-C(=O)-O-$R_3$ or X-$R_4$,

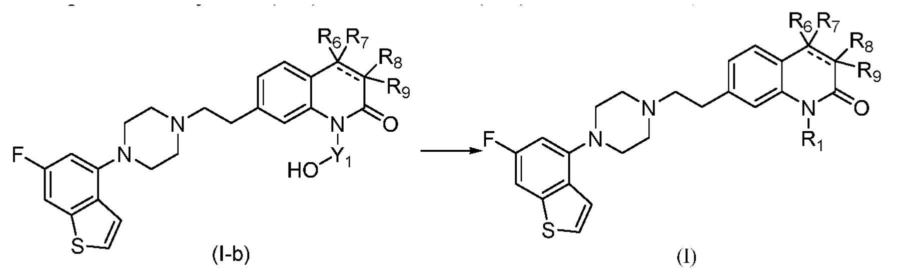

(I-b) (I)

wherein, $R_1$ is $-Y_1-O-C(=O)-R_2$, $-Y_1-O-C(=O)-O-R_3$, or $-Y_1-O-R_4$; ------, $R_6$, $R_7$, $R_8$, $R_9$, $Y_1$, $R_2$, $R_3$, and $R_4$ are as defined in any one of claims 1-10;
X is halogen, preferably bromine, iodine, or chlorine;
the reaction is performed in the presence or absence of a solvent, preferably in a solvent with or without a base;
the reaction is performed at a temperature of room temperature to 200 °C for a period of 1-120 h.

**12.** A pharmaceutical composition comprising the N-substituted quinolinone compound, or the pharmaceutically acceptable salt, the solvate, stereoisomer, the geometric isomer, the isotopically labeled compound or the prodrug thereof according to any one of claims 1-10, and at least one pharmaceutically acceptable carrier.

**13.** A preparation method for the pharmaceutical composition according to claim 12, comprising the following steps: mixing the N-substituted quinolinone compound, or the pharmaceutically acceptable salt, the solvate, stereoisomer, the geometric isomer, the isotopically labeled compound or the prodrug thereof according to any one of claims 1-10 with a pharmaceutically acceptable carrier.

**14.** The N-substituted quinolinone compound, or the pharmaceutically acceptable salt, the solvate, stereoisomer, the geometric isomer, the isotopically labeled compound or the prodrug thereof according to any one of claims 1-10, or the pharmaceutical composition according to claim 12 for use in the prevention and/or treatment of central nervous system diseases, conditions, or disorders, wherein,

preferably, the central nervous system diseases, conditions, or disorders are selected from schizophrenia, affective disorder, mental disorder, mood disorder, bipolar I disorder, bipolar II disorder, depressive disorder, dysthymic disorder, cyclothymic disorder, panic attack, panic disorder, social phobia, obsessive-compulsive disorder, impulsive disorder, post-traumatic stress disorder, anxiety disorder, acute stress disorder, hysteria, anorexia nervosa, sleep disorder, adaptive disorder, cognitive disorder, autism, neuropathic headache, mania, Parkinson's disease, Huntington's chorea, Alzheimer's disease, dementia, memory disorder, hyperactivity disorder, attention deficit/hyperactivity disorder, and tic disorder;
more preferably, the schizophrenia is uncontrollable, intractable, or chronic schizophrenia;
more preferably, the depressive disorder is an intrinsic, severe, or uncontrollable depressive disorder.

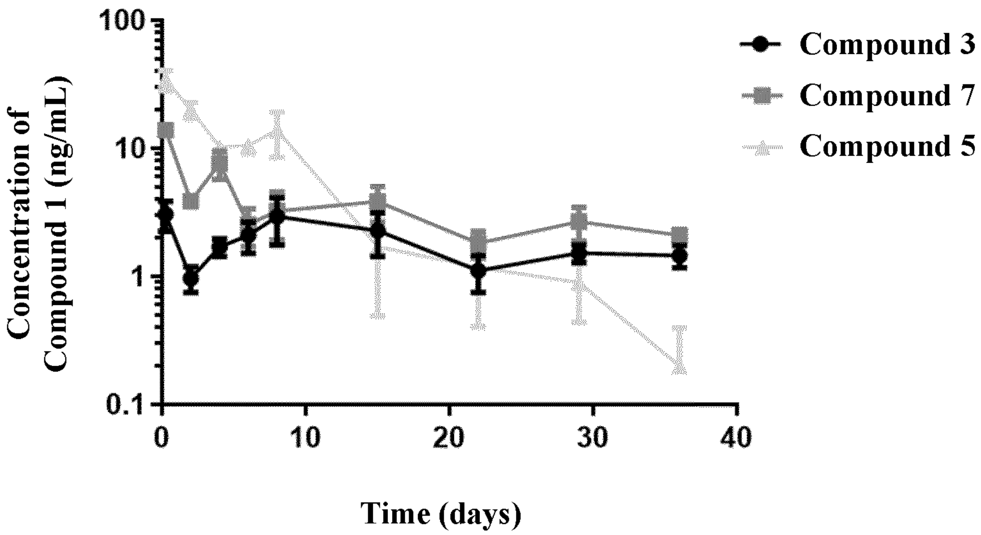
Concentration of Compound 1 (ng/mL)
100
10
1
0.1
0
10
20
30
40
Time (days)
Compound 3
Compound 7
Compound 5

FIG. 1

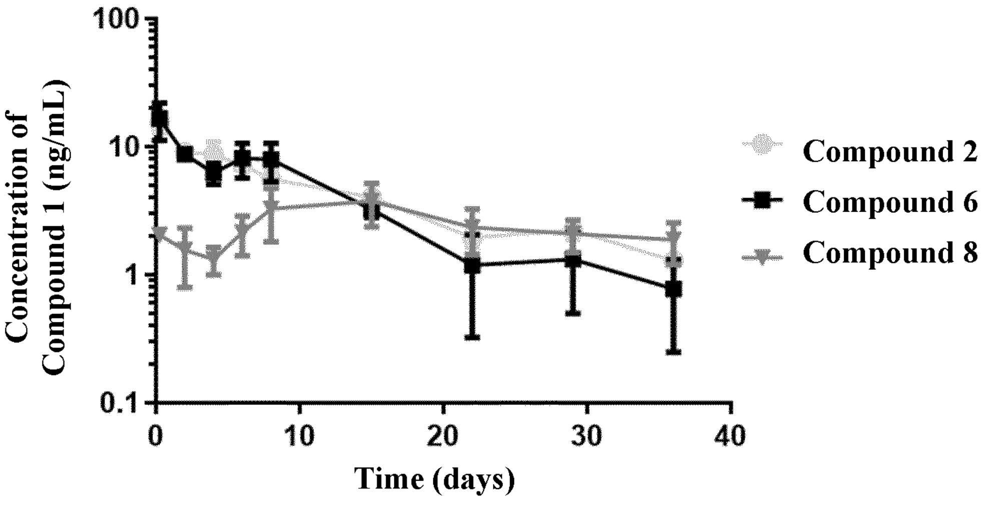
Concentration of Compound 1 (ng/mL)
100
10
1
0.1
0
10
20
30
40
Time (days)
Compound 2
Compound 6
Compound 8

FIG. 2

**INTERNATIONAL SEARCH REPORT**

International application No. **PCT/CN2023/085136**

**A. CLASSIFICATION OF SUBJECT MATTER**

C07D409/12(2006.01)i; A61K31/496(2006.01)i; A61P25/18(2006.01)i; A61P25/14(2006.01)i; A61P25/00(2006.01)i; A61P25/20(2006.01)i; A61P25/16(2006.01)i; A61P25/24(2006.01)i

According to International Patent Classification (IPC) or to both national classification and IPC

**B. FIELDS SEARCHED**

Minimum documentation searched (classification system followed by classification symbols)

IPC: C07D409/12; A61K31/496; A61P 25/-

Documentation searched other than minimum documentation to the extent that such documents are included in the fields searched

Electronic data base consulted during the international search (name of data base and, where practicable, search terms used)

CNTXT, ENTXT, CNABS, VEN, CAPLUS, REGISTRY, MARPAT, CNKI, 万方, WANFANG: 喹啉酮, quinolinone, 苯并噻吩, benzothien, 5-HT, D, antagonist, structure search

**C. DOCUMENTS CONSIDERED TO BE RELEVANT**

| Category* | Citation of document, with indication, where appropriate, of the relevant passages | Relevant to claim No. |
|---|---|---|
| Y | WO 2011163594 A2 (ALKERMES INC. et al.) 29 December 2011 (2011-12-29) see claims 1-15, and description, page 5, line 31-page 6, line 6, page 112, lines 16-31, page 119, lines 8-10, and embodiment 1 | 1-14 |
| Y | WO 2015131856 A1 (SHANGHAI INSTITUTE MATERIA MEDICA et al.) 11 September 2015 (2015-09-11) see claims 1-14 | 1-14 |
| A | CN 1839134 A (WARNER LAMBERT CO.) 27 September 2006 (2006-09-27) see entire document | 1-14 |
| A | JP 2014162781 A (OTSUKA PHARMACEUTICAL CO., LTD.) 08 September 2014 (2014-09-08) see entire document | 1-14 |
| A | CN 105985330 A (SUZHOU WANGSHANWANGSHUI BIOMEDICAL CO., LTD. et al.) 05 October 2016 (2016-10-05) see entire document | 1-14 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 June 2023** | **22 June 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/085136**

| Patent document cited in search report | Publication date (day/month/year) | Patent family member(s) | Publication date (day/month/year) |
|---|---|---|---|
| WO 2011163594 A2 | 29 December 2011 | US 2011319422 A1 | 29 December 2011 |
| | | US 8592427 B2 | 26 November 2013 |
| | | US 2015320875 A1 | 12 November 2015 |
| | | US 9585965 B2 | 07 March 2017 |
| | | AU 2011270701 A1 | 10 January 2013 |
| | | AU 2011270701 B2 | 14 May 2015 |
| | | EP 2585066 A2 | 01 May 2013 |
| | | EP 2585066 A4 | 11 June 2014 |
| | | EP 2585066 B1 | 26 September 2018 |
| | | ES 2691671 T3 | 28 November 2018 |
| | | US 2014094472 A1 | 03 April 2014 |
| | | US 9072788 B2 | 07 July 2015 |
| | | NZ 604423 A | 30 January 2015 |
| | | JP 2016000754 A | 07 January 2016 |
| | | US 2017196848 A1 | 13 July 2017 |
| | | WO 2011163594 A3 | 01 August 2013 |
| | | CA 2802733 A1 | 29 December 2011 |
| | | CA 2802733 C | 21 November 2017 |
| | | JP 2013541494 A | 14 November 2013 |
| | | JP 5848761 B2 | 27 January 2016 |
| WO 2015131856 A1 | 11 September 2015 | AU 2015226578 A1 | 20 October 2016 |
| | | AU 2015226578 B2 | 23 November 2017 |
| | | KR 20160122269 A | 21 October 2016 |
| | | KR 101840249 B1 | 20 March 2018 |
| | | RU 2016137169 A | 21 March 2018 |
| | | RU 2016137169 A3 | 21 September 2018 |
| | | JP 2017508756 A | 30 March 2017 |
| | | JP 6395850 B2 | 26 September 2018 |
| | | CA 2941771 A1 | 11 September 2015 |
| | | CA 2941771 C | 18 February 2020 |
| | | US 2017158680 A1 | 08 June 2017 |
| | | US 10174011 B2 | 08 January 2019 |
| | | EP 3115361 A1 | 11 January 2017 |
| | | EP 3115361 A4 | 26 April 2017 |
| | | EP 3115361 B1 | 23 October 2019 |
| CN 1839134 A | 27 September 2006 | GEP 20084447 B | 10 August 2008 |
| | | PE 20051039 A1 | 12 December 2005 |
| | | NL 1026892 A1 | 28 February 2005 |
| | | NL 1026892 C2 | 21 November 2005 |
| | | US 2006287309 A1 | 21 December 2006 |
| | | GT 200500007 A | 24 October 2005 |
| | | AU 2004266191 A1 | 03 March 2005 |
| | | AU 2004266191 B2 | 26 November 2009 |
| | | UA 82541 C2 | 25 April 2008 |
| | | KR 20060071404 A | 26 June 2006 |
| | | KR 100765668 B1 | 10 October 2007 |
| | | CU 23464 B7 | 17 December 2009 |
| | | RS 20060129 A | 05 June 2008 |
| | | AP 2006003525 A0 | 28 February 2006 |
| | | ZA 200601526 B | 25 April 2007 |

Form PCT/ISA/210 (patent family annex) (July 2022)

**INTERNATIONAL SEARCH REPORT**
**Information on patent family members**

International application No.
**PCT/CN2023/085136**

| Patent document cited in search report | | | Publication date (day/month/year) | Patent family member(s) | | | Publication date (day/month/year) |
|---|---|---|---|---|---|---|---|
| | | | | US | 2005043309 | A1 | 24 February 2005 |
| | | | | US | 7160888 | B2 | 09 January 2007 |
| | | | | HN | 2004000319 | A | 09 June 2010 |
| | | | | EA | 200600294 | A1 | 25 August 2006 |
| | | | | EA | 009645 | B1 | 28 February 2008 |
| | | | | TW | 200524938 | A | 01 August 2005 |
| | | | | TWI | 298327 | B | 01 July 2008 |
| | | | | HK | 1091815 | A1 | 26 January 2007 |
| | | | | WO | 2005019215 | A1 | 03 March 2005 |
| | | | | BRPI | 0413860 | A | 24 October 2006 |
| | | | | IS | 8267 | A | 26 January 2006 |
| | | | | MY | 140693 | A | 15 January 2010 |
| | | | | PA | 8609701 | A1 | 24 May 2005 |
| | | | | IL | 173439 | A0 | 11 June 2006 |
| | | | | AP | 200603525 | D0 | 28 February 2006 |
| | | | | MEP | 45308 | A | 10 February 2011 |
| | | | | CR | 8254 | A | 17 October 2006 |
| | | | | ECSP | 066391 | A | 30 August 2006 |
| | | | | MXPA | 06002070 | A | 19 May 2006 |
| | | | | EP | 1660497 | A1 | 31 May 2006 |
| | | | | MA | 28003 | A1 | 03 July 2006 |
| | | | | AR | 045386 | A1 | 26 October 2005 |
| | | | | CA | 2538915 | A1 | 03 March 2005 |
| | | | | CA | 2538915 | C | 15 June 2010 |
| | | | | JP | 2007503386 | A | 22 February 2007 |
| | | | | JP | 4034811 | B2 | 16 January 2008 |
| | | | | NZ | 545078 | A | 25 September 2009 |
| | | | | TNSN | 06063 | A1 | 03 October 2007 |
| | | | | OA | 13238 | A | 13 December 2006 |
| | | | | NO | 20060324 | L | 18 May 2006 |
| | | | | US | 2006287310 | A1 | 21 December 2006 |
| | | | | CR | 9834 | A | 21 April 2008 |
| | | | | KR | 20070053364 | A | 23 May 2007 |
| | | | | KR | 100801794 | B1 | 05 February 2008 |
| JP | 2014162781 | A | 08 September 2014 | None | | | |
| CN | 105985330 | A | 05 October 2016 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202210333341
- WO 2015131856 A